(19) Europäisches Patentamt
European Patent Office
Office européen des brevets

(11) EP 2 826 371 B1

(12) **EUROPEAN PATENT SPECIFICATION**

(45) Date of publication and mention of the grant of the patent:
**02.01.2019 Bulletin 2019/01**

(51) Int Cl.:
***A01N 59/16*** *(2006.01)* ***A61K 31/33*** *(2006.01)*

(21) Application number: **14173546.4**

(22) Date of filing: **23.11.2008**

# (54) Methods and compositions for inhibiting integrins using tellurium-containing compounds

Verfahren und Zusammensetzungen zum hemmen von integrinen unter Verwendung von telluriumhaltigen Verbindungen

Procédés et compositions pour inhiber les intégrines à l'aide de composés contenant du tellure

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **23.11.2007 US 996548 P**

(43) Date of publication of application:
**21.01.2015 Bulletin 2015/04**

(62) Document number(s) of the earlier application(s) in accordance with Art. 76 EPC:
**08852795.7 / 2 222 167**

(73) Proprietor: **Feramda Ltd.**
**4438029 Kfar-Saba (IL)**

(72) Inventors:
- **Albeck, Michael**
  **52223 Ramat-Gan (IL)**
- **Sredni, Benjamin**
  **44380 Kfar-Saba (IL)**

(74) Representative: **Pearl Cohen Zedek Latzer Baratz UK LLP**
**16 Upper Woburn Place**
**London WC1H 0BS (GB)**

(56) References cited:
**WO-A2-2005/069735 WO-A2-2006/030440**
**WO-A2-2008/032227**

- **SREDNI ET AL: "47.33 Antiviral effect of the immuno modulator AS101 on West Nile Virus by Alpha V Beta intefrin Inhibition", J IMMUNOLOGY , vol. 178 April 2007 (2007-04), XP002732584, Retrieved from the Internet: URL:http://www.jimmunol.org/cgi/content/meeting_abstract/178/MeetingAbstracts/S73 [retrieved on 2014-11-18]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 826 371 B1

## Description

FIELD AND BACKGROUND OF THE INVENTION

The present invention relates to novel therapeutic methods and pharmaceutical compositions for treating conditions associated with inhibition of integrins.

Integrins are family of heterodimeric proteins, comprising an α subunit and a β subunit, located in the plasma membrane of cells. To date, 18 α and 8 β subunits have been identified, as well as over 20 combinations of two subunits. Integrins bind the cells to the extracellular matrix (ECM) and various receptors and mediate the transfer of signals between the cells and the ECM, thereby participating in signals for cell proliferation, differentiation and survival. Integrin binding to an extracellular ligand may regulate intracellular processes ("outside-in signaling"), and intracellular signals may regulate the binding of integrin to the extracellular ligands ("inside-out signaling"). By regulating the connection between cells and their surroundings, integrins play a vital role in coordinating the cells of multicellular organisms.

Angiogenesis, the growth of new blood vessels, is an important process in normal growth and wound healing. However, angiogenesis also plays a key role in the development of cancerous tumors, both by providing the oxygen, nutrients and waste removal necessary for tumor growth, and by providing a conduit by which invasive cells can enter the blood stream, leading to metastasis. There is evidence that angiogenesis is essential for the growth and persistence of solid tumors and their metastases [Folkman et al., Nature 339:58, 1989; Kim et al., Nature 362:841, 1993; Hori et al., Cancer Res., 51:6180, 1991; Zetter, Annu. Rev. Med. 49:407, 1998]. Angiogenesis inhibitors are therefore being developed and tested in order to prevent the development of premalignant growths, and in order to stop the growth of malignant tumors and induce their regression [Bergers et al., Science 284:808, 1999].

Because angiogenesis entails a change in the cellular structure of tissue, integrins play an important role. Integrins on endothelial cells, particularly integrins that bind collagen and fibronectin such as αVβ3 integrin and α5β1 integrin, have been found to mediate pro-angiogenesis signaling [Davis and Senger, Circ. Res. 97:1093, 2005]. Anticancer treatment by antagonism of αVβ3 integrin is being investigated [McNeel et al. Clin. Cancer Res. 11:7851, 2005]. Angiogenesis also contributes to certain inflammatory conditions such as rheumatoid arthritis, and anti-αVβ3 treatments for arthritis are also being tested [Lainer and Brahn, Expert Opin. Investig. Drugs 14:1, 2005].

In addition to promoting angiogenesis, integrins may help promote tumor growth because of their pro-survival properties. Under normal conditions, integrins regulate cell survival by promoting survival of cells bound to the ECM, and promoting apoptosis in cells not bound to the ECM [Stupack, Cell Death and Differen. 12:1021, 2005]. This function of integrins leads to orderly development of cells in tissue. However, many tumor cells express high levels of integrins such as α6β1 and α6β4 integrins that allow them to survive the low-oxygen low-nutrient conditions inside tumors [Chung and Mercurio, Molecules and Cells 17:203, 2004]. These integrins also aid migration of the tumor cells, thereby increasing the invasiveness of the tumor. Thus integrins can aid tumor growth both by promoting angiogenesis and by inhibiting apoptosis.

Integrins are also important regulators of immune responses. The exit of leukocytes from the blood stream that occurs during tissue inflammation is mediated by αLβ2, α2β2, α4β1 and α4β7 integrins that bind molecules on the endothelial wall. Adhesion of the leukocytes to the endothelium allows the leukocytes to exit the blood vessel, while simultaneously inducing structural changes in the leukocyte that cause the leukocyte to be motile. Integrins also mediate the migration of leukocytes through inflamed tissue, and in and out of the lymph nodes by binding the extracellular matrix (ECM) [Hogg et al., J. Cell Sci. 16:4695, 2003]. Antagonists of α4 integrins are being used in order to treat certain inflammatory conditions [Van Assche and Rutgeerts, Am J Physiol Gastrointest Liver Physiol 288:G169, 2005; Rudick et al., N. Eng. J. Med. 354:911, 2006]. The importance of β2 integrins is evidenced by the fact that genetic deficiencies in β2 expression lead to the immunological disorder, leukocyte adhesion deficiency type 1, characterized by chronic infections.

The integrin LFA-1 (αLβ2), in addition to binding leukocytes to endothelium, also binds lymphocytes to antigen-presenting cells, leading to lymphocyte activation. Inhibition of T-cell activation by blocking LFA-1, may be used to control immune responses [Nishibori et al., J. Pharmacol Sci 92:7, 2003].

αEβ7 integrin was found to mediate allograft rejections, apparently by binding T-cells to the graft cells and thus initiating an inflammatory response [Kilshaw and Higgins, J. Exp. Med. 196:873, 2002].

A5β1 and αVβ3 integrins mediate the pro-inflammatory effects of shear stress in vascular endothelial cells. These effects are an important stage in the development of atherosclerosis [Orr et al., Mol. Biol. Cell 17:4686, 2006].

The platelet integrin, GpIIb/IIIa (αIIbβ3 integrin), plays an important part in thrombosis. GpIIb/IIIa causes platelets to aggregate by binding fibrinogen and also connects platelets to the injured tissue by binding von Willebrand factor-collagen complexes. A lack of GpIIb/IIIa results in a bleeding disorder, and inhibitors of GpIIb/IIIa are used as anti-platelet agents. Platelet integrins also promote the generation of thrombin, and mediate some of the effects of thrombin on platelets [Stouffer and Smyth, Arterio. Thrombosis Vasc. Biol. 23:1971, 2003]. Fibrinogen also induces immune responses by binding αMβ2 integrin on leukocytes, resulting in localized inflammatory responses [Flick et al., Exp. Boil. Med.

229:1105, 2004].

Recently, the $\alpha V\beta 3$ integrin was found to be a thyroxine receptor, mediating angiogenesis independently of previously known thyroid hormone receptors [Bergh et al., Endocrinology 146:2864, 2005].

The $\alpha V\beta 3$ integrin is also essential for bone resorption, and antagonists of this integrin can block osteoporosis [Engleman et al., J. Clin. Investig. 99:2284, 1997]. In contrast, $\beta 1$ and $\beta 2$ integrins promote bone formation [Iwaniec et al., J. Appl. Physiol. 98:690, 2005; Miura et al, Proc. Nat. Acad. Sci. 102:14022, 2005].

$\beta 1$ integrins promote cardiac hypertrophy, and inhibition of $\beta 1$ integrins can prevent hypertrophy [Ross et al., Circulation Res. 82:1160, 1998].

Activation of TGF-$\beta$ by $\alpha V\beta 6$ integrin is a necessary stage in the development of pulmonary fibrosis, and pulmonary edema in acute lung injury [Jenkins et al., J. Clin. Invest. 116:1606, 2006; Sheppard, Chest 121:21S, 2002]. $\alpha V\beta 6$ integrin, as well as $\alpha V\beta 1$ integrin, is involved in the TGF-$\beta$-induced renal fibrosis leading to chronic renal failure [Ma et al., Am. J. Pathol. 163:1261, 2003; Ruiz-Torres et al., J. Nephrol. 18:334, 2005].

Integrins play an important part in the reproduction process. $\alpha V\beta 3$, $\alpha 4\beta 1$, and $\alpha 5\beta 1$ integrin are believed to mediate embryo implantation in the uterus [Staun-Ram and Shalev, Reprod. Biol. Endocrinol. 3:56, 2005]. Integrins may also mediate sperm-oocyte fusion [Tatone and Carbone, Reprod. Biol. Endocrinol. 4:48, 2006].

Integrins additionally serve as a target for many viruses and bacteria, which bind integrins in order to enter cells. Adenovirus, echovirus, hantavirus, foot and mouth disease virus, and *E. coli, Y. pseudotuberculosis, Listeria* and *Pseudomonas* bacteria are examples of viruses and bacteria that target integrins.

Various tellurium compounds have been described in the art as having immunomodulating properties. A particularly effective family of tellurium-containing compounds is taught, for example, in U.S. Patents Nos. 4,752,614; 4,761,490; 4,764,461 and 4,929,739, and another effective family is taught, for example, in WO 2006/030437. The immunomodulating properties of this family of tellurium-containing compounds are described, for example, in U.S. Patents Nos. 4,962,207, 5,093,135, 5,102,908 and 5,213,899.

One of the most promising compounds described in these patents is ammonium trichloro(dioxyethylene-O,O')tellurate, which is also referred to herein and in the art as AS101.

AS 101 is characterized by low toxicity. Toxicity tests have shown that LD50 values in rats following intravenous and intramuscular administration of AS 101 are 500-1000 fold higher than the immunologically effective dose.

Another promising tellurium-containing compound is $[TeO_4(COCH)_2]_2$, which is also referred to herein and in the art as SAS.

## SUMMARY OF THE INVENTION

In view of the findings that a myriad of medical conditions are associated with integrins, there is a widely recognized need for and it would be highly advantageous to have, novel agents that are capable of inhibiting integrins and hence can be beneficially utilized in the treatment of such conditions.

The prior art fails to teach the involvement of tellurium-containing compounds such as AS101 and SAS in inhibition of integrins and hence fails to teach the use of tellurium-containing compounds in the treatment of various integrin-related diseases and disorders.

Thus, disclosed herein are compositions and methods utilizing tellurium-containing compounds for inhibiting integrins and hence for treating a medical condition being angiogenesis associated with integrins.

According to an aspect of some embodiments of the invention there is provided the use of at least one tellurium-containing compound in the preparation of a medicament for the treatment of an eye disease or disorder characterized by ocular neovascularization;
wherein said at least one tellurium-containing compound is selected from the group consisting of tellurium dioxide ($TeO_2$), a complex of $TeO_2$, a compound having general Formula I:
the method comprising administering to a subject in need thereof a therapeutically effective amount of at least one tellurium-containing compound having at least one tellurium dioxo moiety.

According to an aspect of some embodiments of the invention there is provided a use of at least one tellurium-containing compound having at least one tellurium dioxo moiety in the preparation of a medicament for treating of a condition being angiogenesis in which inhibition of an integrin is beneficial.

According to an aspect of some embodiments of the invention there is provided a pharmaceutical composition comprising, as an active ingredient, at least one tellurium-containing compound having at least one tellurium dioxo moiety and a pharmaceutically acceptable carrier, the composition being packaged in a packaging material and identified in print, in or on the packaging material, for use in the treatment of a condition being angiogenesis in which inhibition of an integrin is beneficial.

According to some embodiments of the invention, the integrin is associated with angiogenesis.

According to some embodiments of the invention, the integrin binds fibronectin.

According to some embodiments of the invention, the integrin comprises a subunit selected from the group

consisting of αV, β1, β2, β3 and β6 integrin subunits.

According to some embodiments of the invention, the integrin is αVβ3 integrin.

According to some embodiments of the invention, the integrin is VLA-4.

According to some embodiments of the invention, the condition is myocardial angiogenesis.

According to some embodiments of the invention, the medicament is for use in combination with the anti-neoplastic agent.

According to some embodiments of the invention, the pharmaceutical composition is further identified for use in combination with the anti-neoplastic agent.

According to some embodiments of the invention, the tellurium-containing compound and the anti-neoplastic agent are administered concomitantly.

According to some embodiments of the invention, the tellurium-containing compound and the anti-neoplastic agent are administered sequentially.

According to some embodiments of the invention, the medicament further comprises the anti-neoplastic agent.

According to some embodiments of the invention, the at least one tellurium-containing compound is selected from the group consisting of tellurium dioxide ($TeO_2$), a complex of $TeO_2$, a compound having general Formula I:

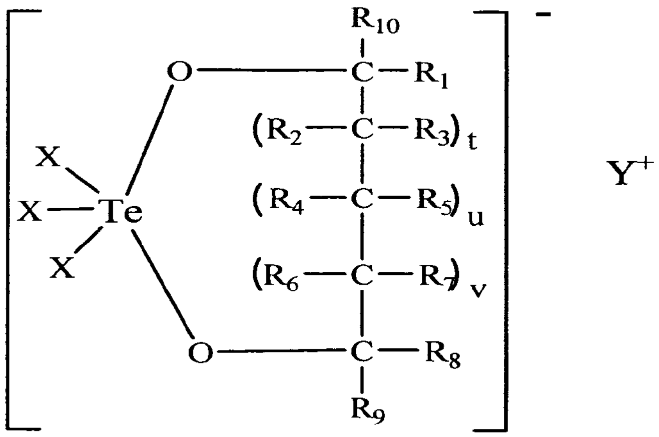

Formula I

a compound having general Formula II:

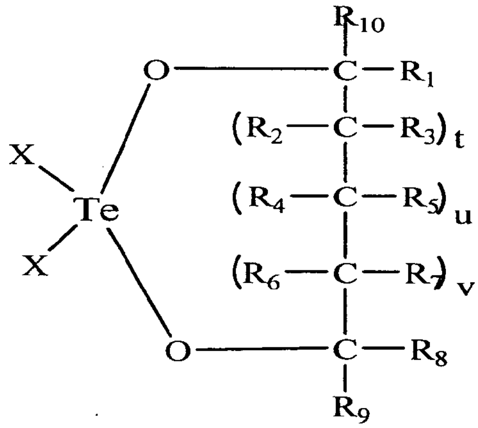

Formula II

a compound having general Formula III:

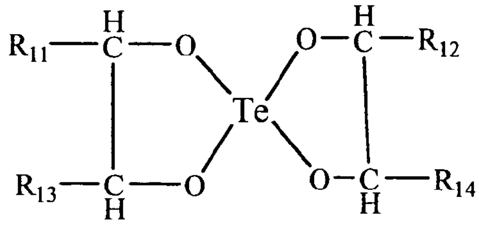

Formula III

and a compound having general Formula IV:

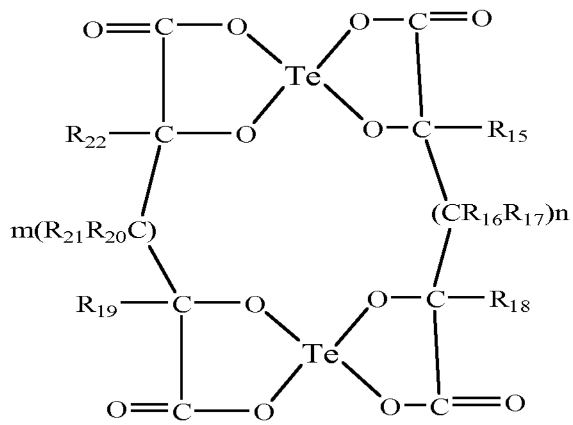

Formula IV

wherein:

each of t, u and v is independently 0 or 1;
each of m and n is independently 0, 1, 2 or 3;
Y is selected from the group consisting of ammonium, phosphonium, potassium, sodium and lithium;
X is a halogen atom; and
each of $R_1$-$R_{22}$ is independently selected from the group consisting of hydrogen, hydroxyalkyl, hydroxy, thiohydroxy, alkyl, alkenyl, alkynyl, alkoxy, thioalkoxy, halogen, haloalkyl, carboxy, carbonyl, alkylcarbonylalkyl, carboxyalkyl, acyl, amido, cyano, N-monoalkylamidoalkyl, N,N-dialkylamidoalkyl, cyanoalkyl, alkoxyalkyl, carbamyl, cycloalkyl, heteroalicyclic, sulfonyl, sulfinyl, sulfate, amine, aryl, heteroaryl, phosphate, phosphonate and sulfoneamido.

According to some embodiments of the invention, the tellurium-containing compound has the general Formula I.
According to some embodiments of the invention, t, u and v are each 0.
According to some embodiments of the invention, each of $R_1$, $R_8$, $R_9$ and $R_{10}$ is hydrogen.
According to some embodiments of the invention, X is chloro.
According to some embodiments of the invention, Y is ammonium.
According to some embodiments of the invention, the compound has the general Formula IV.
According to some embodiments of the invention, each of m and n is 0.
According to some embodiments of the invention, each of $R_{15}$, $R_{18}$, $R_{19}$ and $R_{22}$ is hydrogen.
According to some embodiments of the invention, the administering is effected by a route selected from the group consisting of inhalation, oral, buccal, rectal, transmucosal, transdermal, intradermal, transnasal, intestinal and/or parenteral routes; intramuscular, subcutaneous and/or intramedullary injection routes; intrathecal, direct intraventricular, intravenous, intraperitoneal, intranasal, and/or intraocular injection routes; and/or direct injection into a tissue region.
According to some embodiments of the invention, the therapeutically effective amount ranges from about 0.01 mg/kg/day to about 20 mg/kg/day.
Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In

addition, the materials, methods, and examples are illustrative only and not intended to be limiting.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

The term "comprising" means that other steps and ingredients that do not affect the final result can be added. This term encompasses the terms "consisting of' and "consisting essentially of'.

The phrase "consisting essentially of' means that the composition or method may include additional ingredients and/or steps, but only if the additional ingredients and/or steps do not materially alter the basic and novel characteristics of the claimed composition or method.

As used herein, the term "pharmaceutically acceptable" means approved by a regulatory agency of the Federal or a state government or listed in the U.S. Pharmacopeia or other generally recognized pharmacopeia for use in animals, and more particularly in humans. Herein, the phrases "physiologically suitable carrier" and "pharmaceutically acceptable carrier" are interchangeably used and refer to an approved carrier or a diluent that does not cause significant irritation to an organism and does not abrogate the biological activity and properties of the administered conjugate.

As used herein, the singular form "a" "an" and "the" include plural references unless the context clearly dictates otherwise. For example, the term "a compound" or "at least one compound" may include a plurality of compounds, including mixtures thereof.

Throughout this disclosure, various aspects of this invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

## BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:

FIG's. 1A-B present photographs (FIG 1A) and a bar graph (FIG 1B) demonstrating the inhibitory effect of AS101 and SAS on angiogenesis in the chick chorioallantoic membrane (CAM) assay;

FIG. 2 presents photographs showing the inhibition of angiogenesis by AS101 and SAS in a mouse plug assay, as well as a numerical representation of the results and p values;

FIG's. 3A-B present a bar graph (FIG 3A) and photographs (FIG 3B) demonstrating the inhibitory effect of AS101 (middle photograph) and SAS (lower photograph) on tumor growth in a CAM assay relative to the control (upper photograph);

FIG. 4 presents photographs showing the inhibitory effect of AS101 and SAS on tube formation in both SVEC4-10 and HUVEC endothelial cells;

FIG's. 5A-C present the effect of AS 101 and SAS on the sprouting of blood vessels from aortic arches as photographs (FIG 5A) and bar graphs representing the length (FIG 5B) and number (FIG 5C) of the sprouting vessels;

FIG's. 6A-B present photographs (FIG 6A) and a bar graph (FIG 6B) demonstrating the inhibitory effect of AS101 and SAS on the sprouting of blood vessels from aortic arches treated with ECGS;

FIG. 7 is a bar graph demonstrating the inhibition by AS101 and SAS of SVEC4-10 cell migration in a Boyden

chamber assay;

FIG's. 8A-B present photographs (FIG 8A) and a bar graph (FIG 8B) demonstrating the inhibition by AS101 and SAS of SVEC4-10 cell migration in a "scratch" wound closure assay;

FIG. 9 is a flow chart demonstrating an exemplary method of preparing a cell attachment assay as described in the Examples section hereinbelow;

FIG. 10 is a bar graph demonstrating the inhibition by AS101 and SAS of cell attachment to fibronectin using SVEC4-10, RAW, BV-2 and LAN-1 cell lines;

FIG's. 11A-B are bar graphs demonstrating the inhibition by AS101 and SAS of cell attachment to C16 peptide (FIG 11A) and AG73 peptide (FIG 11B);

FIG's. 12A-B are bar graphs demonstrating the inhibition by AS101 and SAS of $\alpha V\beta 3$ integrin binding of fibronectin (FIG 12A) and C16 peptide (FIG 12B);

FIG's. 13A-D present photographs of Western Blot analyses (FIG 13A) and bar graphs (FIG's 13B-D) demonstrating the inhibition by AS101 and SAS of ECGS-induced FAK Y397 phosphorylation in B16F10 (FIG's 13A and 13B), LAN-1 (FIG's 13A and 13C) and SVEC4-10 (FIG's 13A and 13D) cell lines;

FIG. 14 presents photographs showing the cytopathic effect caused by West Nile Virus as well as the inhibition of the WNV-induced cytopathic effect by AS101;

FIG. 15 is a bar graph demonstrating the effect of AS101 on the decrease in Vero cell viability 72 hours after WNV infection;

FIG's. 16A-B present a bar graph (FIG 16A) and photograph (FIG 16B) demonstrating the inhibition by AS101 of WNV-induced plaque formation in Vero cells;

FIG. 17 is a bar graph demonstrating the inhibition by 5 $\mu$g/ml AS101 of WNV yields 12, 24 and 36 hours after infection of Vero cells;

FIG's. 18A-B present a photograph (FIG 18A) and a bar graph (FIG 18B), demonstrating the inhibition of WNV envelope protein expression 8 hours after infection of Vero cells;

FIG. 19 is a photograph of a Western blot for WNV envelope (WNV-E) protein demonstrating the inhibition by AS 101 of WNV attachment to Vero cells 5 and 15 minutes after infection;

FIG. 20 is a bar graph demonstrating the AS 101-induced increase of WNV plaque forming units (PFU) in culture supernatant 1 hour after WNV infection;

FIG. 21 is a bar graph demonstrating the inhibition by AS101 of attachment of Vero cells to anti-$\alpha_V\beta_3$ integrin antibody;

FIG. 22 is a bar graph demonstrating the inhibition by AS101 of binding between $\alpha_V\beta_3$ integrin binding to WNV;

FIG. 23 is a bar graph demonstrating the inhibition by AS101 of attachment of SVEC cells to surfaces coated with antibodies against $\alpha_1$, $\alpha_4$, $\alpha_5$, $\beta_1$, $\beta_2$ and $\beta_6$ integrin subunits and against $\alpha_V\beta_3$ integrin;

FIG's. 24A-D are graphs demonstrating the increased resistance of HL-60 cells (FIG's A and D) and U937 cells (FIG's B and C) to cytosine arabinoside (ARA-C) (FIG's A and B) and daunorubicin (DRB) (FIG's C and D) when grown on fibronectin (FN) as compared to when grown on BSA or VCAM-1;

FIG's. 25A-C are bar graphs demonstrating the effect of AS101 on cell viability of HL-60 cells exposed to daunorubicin (DRB) in fibronectin-coated plates (FIG 25A), BSA-coated plates (FIG 25B) and VCAM-1-coated plates (FIG 25C);

FIG's. 26A-C are bar graphs demonstrating the effect of AS101 on cell viability of HL-60 cells exposed to cytosine arabinoside (ARAC) in fibronectin-coated plates (FIG 26A), BSA-coated plates (FIG 26B) and VCAM-1-coated plates (FIG 26C);

FIG's. 27A-C are bar graphs demonstrating the effect of AS101 on cell viability of U937 cells exposed to cytosine arabinoside (ARAC) in fibronectin-coated plates (FIG 27A), BSA-coated plates (FIG 27B) and VCAM-1-coated plates (FIG 27C);

FIG. 28 presents histograms demonstrating the effect of AS101 on the percentage of U937 cells in sub-GI phase when exposed to cytosine arabinoside (ARAC) in fibronectin-coated plates or BSA-coated plates, as determined by fluorescence-based flow cytometry;

FIG's. 29A-B present scatter plots demonstrating the effect of AS101 on the proportion of annexin V-positive U937 cells in cells exposed to cytosine arabinoside (Ara-c) in fibronectin-coated plates (FIG 29A) or BSA-coated plates (FIG 29B), as determined by fluorescence-based flow cytometry;

FIG's. 30A-B present histograms demonstrating the effect of AS101 on the proportion of U937 cells exhibiting high caspase-3 activity when exposed to cytosine arabinoside (ARA-C) in fibronectin-coated plates (FIG 30A) or BSA-coated plates (FIG 30B), as determined by fluorescence-based flow cytometry;

FIG. 31 is a photograph of a Western blot demonstrating that AS101 decreases phosphorylation of Akt in U937 cells grown on fibronectin (FN) but not in cells grown on BSA;

FIG. 32 is a photograph of a Western blot showing Bcl2 levels in U937 cells grown in BSA-coated plates (Columns 1-4) or fibronectin coated plates (Columns 5-8); cells were treated with AS101 (Columns 2 and 6), ARA-C (Columns 3 and 7) or AS101 and ARA-C (Columns 4 and 8) or served as controls (Columns 1 and 5);

FIG's. 33A-B present bar graphs demonstrating the effects of AS101 and anti-VLA-4 antibodies on cell viability of

U937 cells exposed to ARA-C in fibronectin-coated plates (FIG 33A) and BSA-coated plates (FIG 33B);
FIG's. 34A-C present bar graphs demonstrating the effects of AS 101 and anti-VLA-5 antibodies on cell viability of U937 cells exposed to ARA-C in fibronectin-coated plates (FIG 34A), VCAM-1-coated plates (FIG 34B) and BSA-coated plates (FIG 34C);
FIG's. 35A-B present histograms showing the levels of VLA-4 and VLA-5 in two acute myelogenous leukemia (AML) patients (FIG 35A) and bar graphs demonstrating the effect of AS 101 on viability of cells from each patient when exposed to ARA-C in BSA-coated plates or fibronectin-coated plates (FIG 35B);
FIG's. 36A-B present bar graphs demonstrating the effect of AS101 on viability of cells from AML patients with high VLA-4 levels (FIG 36A) or low VLA-4 levels (FIG 36B) when exposed to ARA-C in BSA-coated plates or fibronectin-coated plates;
FIG's. 37A-B present bar graphs demonstrating the effect of DTNB (5, 5'-dithiobis-(2-nitrobenzoic acid)) on viability of leukemic cells when exposed to ARA-C in fibronectin-coated plates (FIG 37A) or BSA-coated plates (FIG 37B);
FIG's. 38A-B present bar graphs demonstrating the effect of AS101 and DTNB (5, 5'-dithiobis-(2-nitrobenzoic acid)) on viability of U937 cells when exposed to ARA-C in fibronectin-coated plates (FIG 38A) or BSA-coated plates (FIG 38B);
FIG's. 39A-B present bar graphs demonstrating the effect of AS101 and DTNB (5, 5'-dithiobis-(2-nitrobenzoic acid)) on viability of cells from AML patients with high VLA-4 levels (FIG 39A) or low VLA-4 levels (FIG 39B) when exposed to ARA-C in fibronectin-coated plates; and
FIG. 40 presents photographs of Western blots demonstrating the presence of free thiols on VLA-4 $a_4$ chains (Row B) and not on VLA-4 P! chains (Row D) of U937 cells grown on fibronectin (FN), and the elimination of free thiols by AS 101 (FN+AS).

DESCRIPTION OF THE PREFERRED EMBODIMENTS

The present application discloses therapeutic methods and pharmaceutical compositions utilizing tellurium-containing compounds for inhibiting integrins and for treating angiogenesis as integrin-related disease and disorder.

The present application provides at least one tellurium-containing compound in the preparation of a medicament for the treatment of an eye disease or disorder characterized by ocular neovascularization, wherein said at least one tellurium-containing compound is tellurium dioxide ($TeO_2$), a complex of $TeO_2$, or a compound of general Formula I, II, II or IV as disclosed herein.

The present application also provides at least one tellurium-containing compound for use in treating an eye disease or disorder characterized by ocular neovascularization, wherein the at least one tellurium-containing compound is tellurium dioxide ($TeO_2$), a complex of $TeO_2$, or a compound of general Formula I, II, II or IV as disclosed herein.

The principles and operation of the compositions and methods according to some embodiments of the present invention may be better understood with reference to the drawings and accompanying descriptions.

Before explaining at least one embodiment of the invention in detail, it is to be understood that the invention is not limited in its application to the details set forth in the following description or exemplified by the Examples. The invention is capable of other embodiments or of being practiced or carried out in various ways. Also, it is to be understood that the phraseology and terminology employed herein is for the purpose of description and should not be regarded as limiting.

As shown in the Examples section hereinbelow, while reducing the present invention to practice it was shown, using a chick chorioallantoic membrane assay, that exemplary tellurium-containing compounds inhibit angiogenesis (FIG 1). Exemplary tellurium-containing compounds were also shown to inhibit *in vivo* angiogenesis in a mouse plug assay (FIG 2). Moreover, it was shown that exemplary tellurium-containing compounds reduce the growth of tumors grafted onto chick chorioallantoic membranes (FIG 3).

Exemplary tellurium-containing compounds were also shown to inhibit angiogenesis in *in vitro* assays. It was shown that exemplary tellurium-containing compounds inhibit tube formation in endothelial cell lines (FIG 4). It was further shown that treatment with exemplary tellurium-containing compounds result in shorter lengths of blood vessels sprouting from aortic arches *ex vivo* (FIG 5) and from aortic arches wherein vessel sprouting has been induced by ECGS (endothelial cell growth supplement) (FIG 6). Furthermore, endothelial cell migration, a step in angiogenesis, was shown to be inhibited

in endothelial cell lines by exemplary tellurium-containing compounds using both a Boyden chamber assay (FIG 7) and a scratch wound assay (FIG 8).

As mentioned in the Background section hereinabove, angiogenesis is regulated in part by the interactions between the ECM (extracellular matrix) and cellular integrins that attach thereto. The effect of tellurium-containing compounds on the attachment of cells to proteins and peptides of the ECM was therefore investigated, using a plate coated with the ECM peptide, according to the method shown in FIG 9. It was shown that exemplary tellurium-containing compounds inhibit the attachment of a variety of cell types to the ECM protein, fibronectin (FIG 10). It was also shown

that exemplary tellurium-containing compounds moderately decrease the attachment of an endothelial cell line to C16 and AG73, peptides derived from laminin, an ECM protein (FIG 11). Moreover, the attachment of $\alpha V\beta 3$ integrin, an integrin known to promote angiogenesis, to fibronectin and C16, was shown to be inhibited by tellurium-containing compounds (FIG 12).

As mentioned hereinabove, attachment of integrins to the ECM induces cell signaling. FAK is a downstream mediator of integrin signaling, and undergoes autophosphorylation at tyrosine-397 in response to the binding of integrin. It was shown that FAK autophosphorylation is inhibited by tellurium-containing compounds in a variety of cell types (FIG 13).

Tellurium-containing compounds were also shown to inhibit cell damage and death caused by a virus (FIG's 14-16). Viral yields (FIG 17) and protein expression (FIG 18) were shown to be inhibited in infected cells. Entry of the virus into cells was blocked by tellurium-containing compounds, resulting in less virus inside the cells (FIG 19) and more virus remaining outside cells (FIG 20).

As $\alpha V\beta 3$ integrin may serve as a receptor for viruses, the ability of tellurium-containing compounds to inhibit $\alpha V\beta 3$ integrin binding was tested. Tellurium-containing compounds were shown to inhibit binding of $\alpha V\beta 3$ integrin to anti-$\alpha V\beta 3$ antibodies (FIG 21) and to viruses (FIG 22).

Using specific antibodies against various integrin subunits, it was shown that tellurium-containing compounds inhibit binding of a wide range of integrin types by antibodies specific to the integrin (FIG 23).

The protein fibronectin, which binds to integrins, was shown to induce resistance to chemotherapeutic agents in cancer cells (FIG 24). Tellurium-containing compounds were shown to sensitize cancer cells grown on fibronectin to cell death by chemotherapeutic agents, thereby overcoming the fibronectin-induced resistance to chemotherapy (FIG's 25-27). Cancer cells sensitized by tellurium-containing compounds were shown to have an increased tendency to be in a sub-G1 phase (FIG 28), be annexin V-positive (FIG 29) and exhibit high caspase-3 activity (FIG 30), all being indications of cell damage or death. In addition, tellurium-containing compounds lowered levels of the survival factors phosphorylated Akt (FIG 31) and Bcl2 (FIG 32) in cells grown on fibronectin.

Antibodies against the integrin VLA-4 were shown to sensitize cancer cells in a manner similar to that of tellurium-containing compounds, and tellurium-containing compounds did not have an additional effect in the presence of these antibodies (FIG 33). Antibodies against VLA-5 did not sensitize cancer cells (FIG 34). It was thus shown that expression of VLA-4 mediates the sensitization of cancer cells by tellurium-containing compounds, and that VLA-4 levels in cancer cells serve as an indication that tellurium-containing compounds would sensitize the cancer cells to chemotherapeutic agents (FIG's 35-36).

While further studying the sensitization effect of otherwise resistant cancer cells by tellurium-containing compounds, the effect of thiol blocking compounds was studied. Thiol blocking compounds were shown to sensitize cancer cells grown on fibronectin in a manner similar to that of tellurium-containing compounds (FIG 37), and tellurium-containing compounds did not have an additional effect in the presence of these antibodies (FIG 38-39). As tellurium-containing compounds are known to react with thiols, it is suggested that the sensitization induced by tellurium-containing compounds is mediated by thiol groups present on the VLA-4 integrin. Free thiol groups were indeed shown to be present on the $\alpha$ chain of VLA-4 integrin (FIG 40).

In conclusion, it has been shown, in both *in vitro* and *in vivo* studies, that tellurium-containing compounds exhibit anti-angiogenic properties, anti-viral properties, and chemosensitization properties.

It has been demonstrated that the mode of action of tellurium-containing compounds is by inhibition of integrins. Thus, exemplary tellurium-containing compounds have been shown to inhibit integrin-mediated processes such as angiogenesis and cell attachment to ECM, viruses and fibronectin, and in general, to inhibit integrin-mediated cell signaling via FAK and actin stress fiber formation.

The data presented here suggest that tellurium-containing compounds may contribute a significant role in blocking many integrin-mediated pathophysiological conditions, via inhibition of integrin binding and subsequent cell signaling.

The growing recognition of the role of integrins in various human pathologies indicates the need for identifying their targeting therapeutic potential by finding effective means for controlling their activation. Alternative and new approaches are desirable in order to supersede existing strategies of integrin inactivation. Importantly, the tools uncovered by the present inventors for modulation of integrin activity are particularly suitable for treatment protocols, as they are substantially non-toxic.

As used herein, the phrase "tellurium-containing compound" encompasses any compound that includes one or more tellurium atoms.

Herein throughout, the phrases "tellurium dioxo moiety" and "tellurium dioxide moiety" are used interchangeably, and describe an -O-Te-O-, in which the tellurium center can be further substituted, or a O=Te=O.

The tellurium-containing compound may be an inorganic compound or an organic compound.

Inorganic tellurium-containing compounds include, for example, tellurium dioxide ($TeO_2$) *per se.*

Organic tellurium-containing compounds may be in the form of an organic complex such as, for example, a

Te02 complex with citric acid or ethylene glycol, which may form $TeO_2$ as an end product in aqueous solutions. A representative example of the latter is the complex $TeO_2$'$HOCH_2CH_2OH$'NH4Cl. Otherwise, the tellurium-containing compounds described herein include one or more tellurium atoms and one or more organic moieties that are attached thereto, for example, ammonium salts, or any other salts, of halogenated tellurium-containing compounds having a bidentate cyclic moiety attached to the tellurium atom.

Exemplary compounds in this category can be collectively represented by the general Formula I:

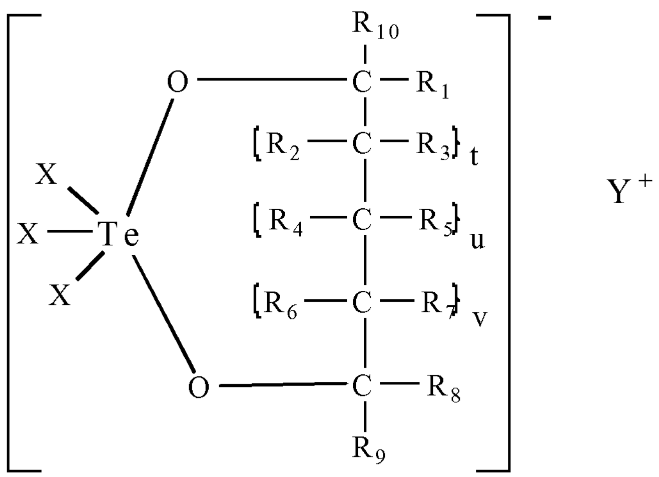

Formula I

In the general Formula I above, each of t, u and v is independently 0 or 1, such that the compound may include a five-membered ring, a six-membered ring, or a seven-membered ring. Preferably, each of t, u and v is 0, such that the compound includes a five-membered ring.

X is a halogen atom, as described hereinabove, and is preferably chloro.

Y can be ammonium, phosphonium, potassium, sodium and lithium, and is preferably ammonium.

Each of $R_1$-$R_{10}$ is independently selected from the group consisting of hydrogen, hydroxyalkyl, hydroxy, thiohydroxy, alkyl, alkenyl, alkynyl, alkoxy, thioalkoxy, halogen, haloalkyl, carboxy, carbonyl, alkylcarbonylalkyl, alkoxy, carboxyalkyl, acyl, amido, cyano, N-monoalkylamidoalkyl, N,N-dialkylamidoalkyl, cyanoalkyl, alkoxyalkyl, carbamyl, cycloalkyl, heteroalicyclic, sulfonyl, sulfinyl, sulfate, amine, aryl, heteroaryl, phosphate, phosphonate and sulfoneamido.

As used herein, the term "alkyl" refers to a saturated aliphatic hydrocarbon including straight chain and branched chain groups. Preferably, the alkyl group has 1 to 20 carbon atoms. More preferably, the alkyl is a medium size alkyl having 1 to 10 carbon atoms. Most preferably, unless otherwise indicated, the alkyl is a lower alkyl having 1 to 5 carbon atoms. The alkyl group may be substituted or unsubstituted. When substituted, the substituent group can be as described herein for $R_1$.

As used herein, the term "hydroxyalkyl" refers to an alkyl, as this term is defined herein, substituted by a hydroxy group, as defined herein, and includes, for example, hydroxymethyl, hydroxyethyl, hydroxypropyl and hydroxy-n-butyl.

As used herein, the term "halogen", which is also referred to herein interchangeably as "a halogen atom" or "halo", includes chloro (Cl), bromo (Br), iodo (I) and fluoro (F).

The term "haloalkyl" refers to an alkyl, as this term is defined herein, substituted by a halogen, as defined herein, and includes, for example, chloromethyl, 2-iodoethyl, 4-bromo-n-butyl, iodoethyl, 4-bromo-n-pentyl and the like.

The term "alkanoyloxy" refers to a carbonyl group, as define herein and includes, for example, acetyl, propionyl, butanoyl and the like.

The term "carboxyalkyl" refers to an alkyl, as this term is defined herein, substituted by a carboxy group, as defined herein, and includes, for example, carboxymethyl, carboxyethyl, ethylenecarboxy and the like.

The term "alkylcarbonylalkyl" refers to an alkyl, as this term is defined herein, substituted by a carbonyl group, as defined herein, and includes, for example, methanoylmethyl, ethanoylethyl and the like.

The term "amidoalkyl" refers to an alkyl, as this term is defined herein, substituted by an amide group, as defined herein, and includes, for example, $-CH_2CONH_2$; $-CH_2CH_2CONH_2$; $-CH_2CH_2CH_2CONH_2$ and the like.

The term "cyanoalkyl" refers to an alkyl, as this term is defined herein, substituted by an cyano group, as defined herein, and includes, for example, $-CH_2CN$; $-CH_2CH_2CN$;$-CH_2CH_2CH_2CN$ and the like.

The term "N-monoalkylamidoalkyl" refers to an alkyl, as this term is defined herein, substituted by an amide group, as defined herein, in which one of R' and R" is an alkyl, and includes, for example, $-CH_2CH_2CONHCH_3$, and -CH-

$_2CONHCH_2CH_3$.

The term N,N-dialkylamidoalkyl refers to an alkyl, as this term is defined herein, substituted by an amide group, as defined herein, in which both R' and R" are alkyl, and includes, for example, $-CH_2CON(CH_3)_2$; $CH_2CH_2CON(CH_2-CH_3)_2$ and the like.

A "cycloalkyl" group refers to an all-carbon monocyclic or fused ring (i.e., rings which share an adjacent pair of carbon atoms) group wherein one of more of the rings does not have a completely conjugated pi-electron system. Examples, without limitation, of cycloalkyl groups are cyclopropane, cyclobutane, cyclopentane, cyclopentene, cyclohexane, cyclohexadiene, cycloheptane, cycloheptatriene, and adamantane. A cycloalkyl group may be substituted or unsubstituted. When substituted, the substituent group can be as described herein for R1.

An "alkenyl" group refers to an alkyl group which consists of at least two carbon atoms and at least one carbon-carbon double bond.

An "alkynyl" group refers to an alkyl group which consists of at least two carbon atoms and at least one carbon-carbon triple bond.

An "aryl" group refers to an all-carbon monocyclic or fused-ring polycyclic (i.e., rings which share adjacent pairs of carbon atoms) groups having a completely conjugated pi-electron system. Examples, without limitation, of aryl groups are phenyl, naphthalenyl and anthracenyl. The aryl group may be substituted or unsubstituted. When substituted, the substituent group can be as described herein for R1.

A "heteroaryl" group refers to a monocyclic or fused ring (i.e., rings which share an adjacent pair of atoms) group having in the ring(s) one or more atoms, such as, for example, nitrogen, oxygen and sulfur and, in addition, having a completely conjugated pi-electron system. Examples, without limitation, of heteroaryl groups include pyrrole, furan, thiophene, imidazole, oxazole, thiazole, pyrazole, pyridine, pyrimidine, quinoline, isoquinoline and purine. The heteroaryl group may be substituted or unsubstituted. When substituted, the substituent group can be as described herein for R1.

A "heteroalicyclic" group refers to a monocyclic or fused ring group having in the ring(s) one or more atoms such as nitrogen, oxygen and sulfur. The rings may also have one or more double bonds. However, the rings do not have a completely conjugated pi-electron system. The heteroalicyclic may be substituted or unsubstituted. When substituted, the substituent group can be as described herein for R1.

A "hydroxy" group refers to an -OH group.

An "alkoxy" group refers to both an -O-alkyl and an -O-cycloalkyl group, as defined herein.

An "aryloxy" group refers to both an -O-aryl and an -O-heteroaryl group, as defined herein.

A "thiohydroxy" group refers to a -SH group.

A "thioalkoxy" group refers to both an -S-alkyl group, and an -S-cycloalkyl group, as defined herein.

A "thioaryloxy" group refers to both an -S-aryl and an -S-heteroaryl group, as defined herein.

A "carbonyl" group refers to a -C(=O)-R' group, where R' is hydrogen, alkyl, alkenyl, cycloalkyl, aryl, heteroaryl (bonded through a ring carbon) or heteroalicyclic (bonded through a ring carbon) as defined herein.

A "thiocarbonyl" group refers to a -C(=S)-R' group, where R' is as defined herein.

A "carboxy" group refers to a -C(=O)-O-R' or a -O-C(=O)-R' group, where R' is as defined herein.

A "sulfinyl" group refers to an -S(=O)-R' group, where R' is as defined herein.

A "sulfonyl" group refers to an $-S(=O)_2-R'$ group, where R' is as defined herein.

A "sulfate" group refers to a $-O-S(=O)_2-OR'$ group, where R' is as defined herein.

A "sulfoneamido" group refers to a $-S(=O)_2-NR'R''$ group or a $R'S(=O)_2-NR''$, with R' is as defined herein and R" is as defined for R'.

A "carbamyl" or "carbamate" group refers to an -OC(=O)-NR'R" group or a R"OC(=O)-NR'- group, where R' and R" are as defined herein.

A "thiocarbamyl" or "thiocarbamate" group refers to an -OC(=S)-NR'R" group or an R"OC(=S)NR'- group, where R' and R" are as defined herein.

An "amino" group refers to an -NR'R" group where R' and R" are as defined herein.

An "amido" group refers to a -C(=O)-NR'R" group or a R'C(=O)-NR" group, where R' and R" are as defined herein.

A "nitro" group refers to an $-NO_2$ group.

A "cyano" group refers to a -C≡N group.

The term "phosphonyl" describes a -O-P(=O)(OR')(OR") group, with R' and R" as defined hereinabove.

The term "phosphinyl" describes a -PR'R" group, with R' and R" as defined hereinabove.

As cited hereinabove, the compounds in this category are salts of organic tellurium-containing compounds. The salts can be, for example, ammonium salts, phosphonium salts and alkaline salts such as potassium salts, sodium salts, lithium salts and the like.

Hence, Y in Formula I above can be a phosphonium group, as defined herein, an ammonium group, as defined herein, potassium ($K^+$), sodium ($Na^+$) or lithium ($Li^+$).

As used herein, the term "phosphonium" describes a $-P^+R'R''R'''$ group, with R' and R" as defined herein and

R''' is as defined for R'. The term "phosphonium", as used herein, further refers to a $-P^+R_6$ group, wherein each of the six R substituents is independently as defined herein for R, R" and R'''.

The term "ammonium" describes a $-N^+R'R"R'''$ group, with R', R" and R''' as defined herein.

Preferred compounds in this category include compounds having the general Formula I described above, in which Y is ammonium or phosphonium, t, u and v are each 0, and each of $R_1$, $R_8$, $R_9$ and $R_{10}$ is independently hydrogen or alkyl. These compounds can be represented by the following structure:

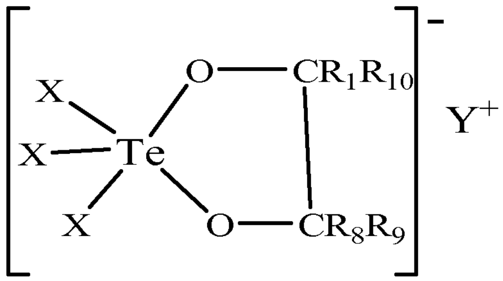

wherein each of $R_1$, $R_8$, $R_9$ and $R_{10}$ is independently hydrogen or alkyl, whereas a preferred alkyl is methyl, and X is halogen, preferably chloro.

The presently most preferred compound for use in the context of the present embodiments has the following structure:

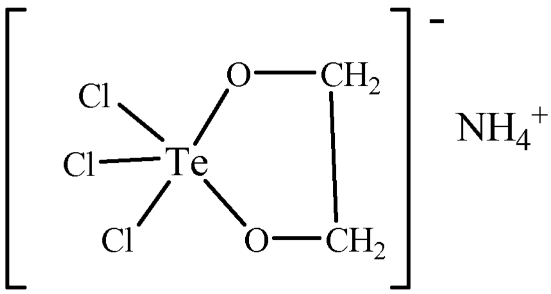

This compound is ammonium trichloro(dioxyethylene-O,O')tellurate, which is also referred to herein and in the art as AS101.

Additional representative examples of organic tellurium-containing compound that are suitable for use in the context of the present invention include halogenated tellurium having a bidentate cyclic moiety attached to the tellurium atom. The bidentate cyclic moiety is preferably a dioxo ligand having two oxygen atoms attached to the tellurium atom.

Exemplary compounds in this category can be represented by the general Formula II:

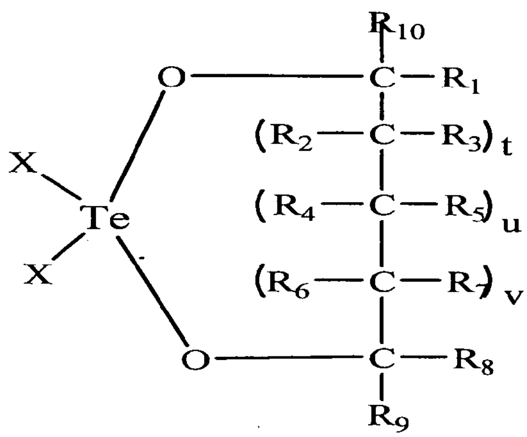

Formula II

wherein t, u, v, X and $R_1$-$R_{10}$ are as defined hereinabove.

Preferred compounds are those in which t, u, and v are each 0, and X is chloro, such as, but not limited to, the compound having the following structure:

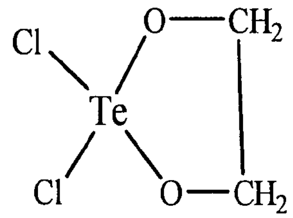

The above compound is also known in the art and referred to herein as AS 103.

The organic tellurium-containing compounds having Formulae I and II can be readily prepared by reacting tetrahalotelluride such as $TeCl_4$ with a dihydroxy compound,
as is described in detail in U.S. Patents Nos. 4,752,614, 4,761,490, 4,764,461 and 4,929,739.

Additional representative examples of organic tellurium-containing compounds that are suitable for use in the context of the present embodiments include compounds in which two bidentatic cyclic moieties are attached to the tellurium atom. Preferably, each of the cyclic moieties is a dioxo moiety.

Exemplary compounds are those having the following structure:

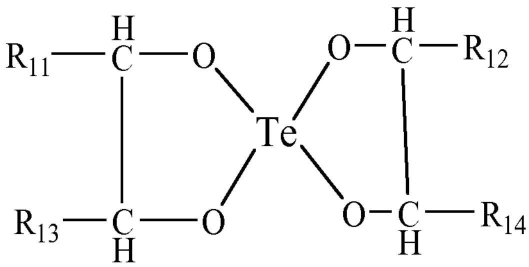

wherein each of $R_{11}$-$R_{14}$ is independently selected from the group consisting of hydrogen, hydroxyalkyl, hydroxy, thiohydroxy, alkyl, alkenyl, alkynyl, alkoxy, thioalkoxy, halogen, haloalkyl, carboxy, carbonyl, alkylcarbonylalkyl, alkoxy, carboxyalkyl, acyl, amido, cyano, N-monoalkylamidoalkyl, N,N-dialkylamidoalkyl, cyanoalkyl, alkoxyalkyl, carbamyl, cycloalkyl, heteroalicyclic, sulfonyl, sulfinyl, sulfate, amine, aryl, heteroaryl, phosphate, phosphonate and sulfoneamido, as these terms are defined herein.

The most preferred compound in this category is a compound in which each of $R_{11}$-$R_{14}$ is hydrogen. This compound is also known in the art and referred to herein as AS102.

Additional representative examples of organic tellurium-containing compounds that are suitable for use in the context of the present embodiments include the recently disclosed ditellurium compounds having general Formula IV:

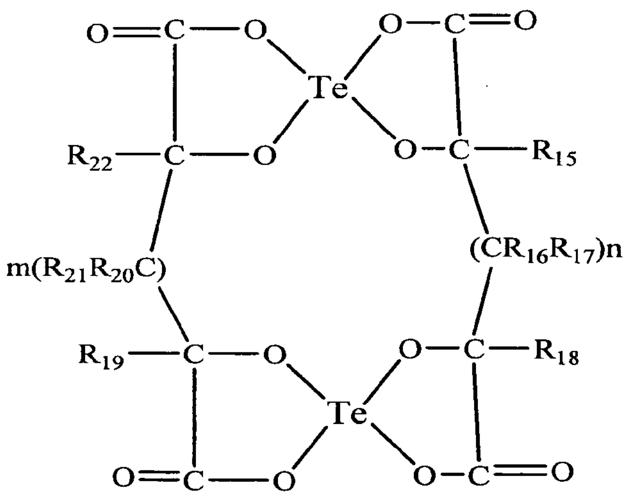

Formula IV

wherein each of $R_{15}$-$R_{22}$ is independently selected from the group consisting of hydrogen, hydroxyalkyl, hydroxy, thiohydroxy, alkyl, alkenyl, alkynyl, alkoxy, thioalkoxy, halogen, haloalkyl, carboxy, carbonyl, alkylcarbonylalkyl, alkoxy, carboxyalkyl, acyl, amido, cyano, N-monoalkylamidoalkyl, N,N-dialkylamidoalkyl, cyanoalkyl, alkoxyalkyl, carbamyl, cycloalkyl, heteroalicyclic, sulfonyl, sulfinyl, sulfate, amine, aryl, heteroaryl, phosphate, phosphonate and sulfoneamido, as these terms are defined herein; and
m and n are each an integer from 0 to 3.

Exemplary compounds in this category are those in which m and n are each 0.

The presently most preferred compound in this family is a compound in which $R_{15}$, $R_{18}$, $R_{19}$ and $R_{22}$ are all hydrogen, referred to hereinafter as SAS, and which has the following structure:

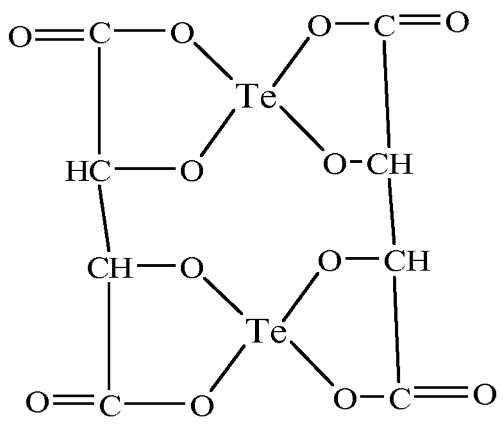

According to a most preferred embodiment of the present invention, the tellurium-containing compound is either AS101 or SAS.

The compounds described above can be administered or otherwise utilized in the various aspects of the present invention, either as is or as a pharmaceutically acceptable salt thereof.

The phrase "pharmaceutically acceptable salt" refers to a charged species of the parent compound and its counter ion, which is typically used to modify the solubility characteristics of the parent compound and/or to reduce any significant irritation to an organism by the parent compound, while not abrogating the biological activity and properties of the administered compound.

The compounds described are advantageously utilized in the treatment of a subject suffering from a condition being angiogenesis in which inhibition of an integrin is beneficial, so as to treat the condition via integrin inhibition.

As described hereinabove, integrins play a role in various biological pathways and processes. For example, integrins such as αVβ3 integrin and α5β1 integrin participate in angiogenesis, integrins such as α4β1 (also known and referred to herein as VLA-4), α6β1 and α6β4 promote survival and migration of tumor cells, integrins such as integrins and β2 integrins (e.g. αLβ2, α2β2, αMβ2, α4β1 (VLA-4) and α4β7 integrin), as well as αVβ3, αEβ7 and α5β1 integrin, mediate inflammation, αEβ7 integrin mediates allograft rejection, αIIbβ3 integrin participates in thrombosis, αVβ3 integrin acts as a thyroxine receptor and mediates bone resorption, integrins such as β1 and β2 integrins mediate bone formation, β1 integrins mediate the formation of cardiac hypertrophy, integrins such as αVβ6 integrin mediate the formation of pulmonary fibrosis, integrins such as αVβ6 and αVβ1 integrin mediate the formation of renal fibrosis, and integrins such as αVβ3 α4β1, and α5β1 integrin mediate embryo implantation in the uterus. Integrins serve as targets that allow many viruses and bacteria to enter cells. For example, adenoviruses utilize αV and β1 integrin to enter cells, herpesviruses utilize β1 and β3 (e.g., αVβ3) integrins, hantaviruses utilize β3 integrins, reoviruses utilize β3 and β1 integrins, echoviruses utilize α2 integrins, HIV utilizes αV integrins *[*Stewart and Nemerow, Trends in Microbiol. 15:500, 2007*;* Ballana et al., Blood, Oct. 7, 2008 *(electronically published ahead of print);* Bentz and Yurochko, Proc. Natl. Acad. Sci. USA 105:5531, 2008*]*.

Hence, inhibition of integrins can inhibit many biological pathways.

As used herein, the phrase "a condition in which inhibition of an integrin is beneficial" refers to any disease, disorder, symptom or medical condition that a subject wishes to prevent, alleviate or ameliorate, and which is at least in part characterized by, or mediated by, any of the abovementioned biological pathways and processes, such that inhibition of the biological pathway or process by inhibition of the integrin involved in this pathway or process would be expected to prevent, alleviate or ameliorate the condition.

In an embodiment of the present invention, the abovementioned use or composition relate to the inhibition of an integrin that is associated with angiogenesis. Preferably, the angiogenesis-associated integrin is αVβ3 integrin.

In another embodiment of the invention, the abovementioned use or composition relate to the inhibition of an integrin that binds fibronectin. In one embodiment, the fibronectin-binding integrin is VLA-4. In some embodiments, fibronectin-binding integrin is αVβ3 integrin.

In an embodiment of the invention, the integrin comprises a subunit selected from the group consisting of aV, β1, β2, β3 and β6 integrin subunits. In some embodiments, the integrin comprises β1, β2 or β6 subunits.

In some embodiments of the invention, the conditions treatable by the tellurium-containing compounds described herein include angiogenesis conditions in which inhibition of αVβ3 integrin is beneficial.

In some embodiments, the conditions treatable by the tellurium-containing compounds described herein include angiogenesis conditions in which inhibition of VLA-4 integrin is beneficial.

Exemplary ocular disorders that are treated by the tellurium-containing compounds include, but are not limited to, eye diseases characterized by ocular neovascularization including, but not limited to, diabetic retinopathy, retinopathy of prematurity, neovascular glaucoma, retinoblastoma, retrolental fibroplasia, rubeosis, uveitis, macular degeneration, diseases associated with choroidal neovascularization or iris neovascularization, and corneal graft neovascularization. Ocular disorders characterized by inflammation or tumors are also included.

As used herein, the phrase "anti-neoplastic agent" refers to any agent used in the medical arts to kill neoplastic cells (as in the case of cytotoxic agents) and/or to inhibit growth and/or proliferation of neoplastic cells. Non-limiting examples of antineoplastic agents usable in the context of the present invention include cytosine arabinoside, daunorubicin, doxorubicin, idarubicin, amrubicin, pirarubicin, epirubicin, mitoxantrone, etoposide, teniposide, vinblastine, vincristine, mitomycin C, 5-FU, paclitaxel, docetaxel, actinomycin D, colchicine, topotecan, irinotecan, gemcitabine cyclosporin, verapamil, valspodor, probenecid, MK571, GF120918, LY335979, biricodar, terfenadine, quinidine, pervilleine A and XR9576. In some embodiments, the anti-neoplastic agent is cytosine arabinoside or daunorubicin.

Exemplary neoplastic conditions (e.g., malignant and metastatic conditions) and exemplary anti-neoplastic agents are described in additional detail hereinbelow.

In some embodiments, the resistance is characterized by the presence of a fibronectin-binding integrin (e.g., VLA-4). Thus, as demonstrated in the Examples hereinbelow, resistance is in some cases induced by the presence of fibronectin, and is inhibited by inhibition of an integrin (e.g., VLA-4) which binds fibronectin.

Optionally, the tellurium-containing compound is administered along with an anti-neoplastic agent, such as, for example, an anti-neoplastic agent to which a resistance has been identified.

Resistance of many neoplasms (e.g., tumors and cancers) to established treatment regimens constitutes a major problem in therapy. Novel strategies to target cancer cell resistance are essential for improving patient outcome.

Resistance to anti-neoplastic agents may be acquired, for example, as a result of previous treatments. Thus, chemotherapy is often less effective when administered to a patient for a second time, as relapse of a neoplastic condition following the first chemotherapy treatment will often be in a form resistant to one or more chemotherapeutic agents.

Alternatively, resistance to a neoplastic agent may be inherent. For example, the resistance may be a property of the genetic makeup of the subject with the neoplastic condition and/or a property of the cell type of the neoplastic cells.

There exists substantial evidence that the tumor microenvironment may contribute to the failure of standard chemotherapy to eradicate the entire cancer cell population and may facilitate the emergence of acquired drug resistance to anti-neoplastic agents used in chemotherapy [Meads et al., Clin. Cancer Res. 14:2519, 2008*]*.

For example, bone marrow residual disease (MRD) causes relapse after chemotherapy in patients with acute myelogenous leukemia (AML) due to acquired drug resistance. It has been found that this resistance is induced by the attachment of the integrin receptor VLA-4 on leukemic cells to its ligand fibronectin (FN) on bone marrow stromal cells *[*Matsunaga et al., Nature Med. 9:1158, 2003*]*.

As demonstrated in the Examples section hereinbelow, fibronectin-induced resistance is inhibited by tellurium-containing compounds, via inhibition of VLA-4 integrin. Thus, tellurium-containing compounds may obviate the resistance of the neoplastic cells and convert resistant cless to non-resistant cells. As further exemplified therein, the suitability of a patient for treatment by the tellurium-containing compound can be readily determined by determining the expression of VLA-4 in the neoplastic cells of the patient. The ability to determine suitable patients thereby increases the effectivity of treatment with tellurium-containing compounds, as patients least likely to respond positively to the treatment are excluded.

Determination of a high level of expression of the integrin may be performed by any suitable method known in the art. The determination may be performed, for example, when testing cells from the subject for the presence of a neoplastic condition cells. Alternatively, determining the level of expression of the integrin may be performed after a neoplastic condition has already been diagnosed. The level of expression of the integrin may be determined according to any method known in the art (e.g., ELISA, fluorescence-based flow cytometry), and one of skill in the art will be familiar with a variety of suitable methods.

In some embodiments, expression of VLA-4 is determined by fluorescence-based flow cytometry, using anti-VLA-4 antibodies labeled with a fluorescent label (e.g., fluorescein isocyanate), such that high fluorescence is indicative for high level of expression of the integrin; low fluorescence is indicative for a low level of expression of the integrin and

no fluorescence is indicative for the absence of the integrin. Flow cytometry is a standard method used in the art, and one of skill in the art will be readily capable of determining a high expression of VLA-4 by this method, using standard procedures.

In some embodiments, the phrase "high level of expression" refers to a level of expression in neoplastic cells that is at least 20 % higher than in normal cells (i.e., cells which are not neoplastic) of the same type, under the same conditions, as the neoplastic cells, optionally at least 40 % higher, optionally at least 60 % higher, optionally at least 80 % higher, optionally at least 100 % higher, optionally at least 200 % higher, and optionally at least 500 % higher than in normal cells.

By determining a level of expression of the integrin, as described herein, a suitable treatment for a subject with an anti-neoplastic condition can be more optimally determined.

According to some embodiments, the integrin associated with resistance to the anti-neoplastic agent is an integrin that binds fibronectin. In some embodiments, the integrin is VLA-4.

The tellurium-containing compound and the anti-neoplastic agent, utilized in embodiments of the methods and uses described herein, may be administered concomitantly. Alternatively, the tellurium-containing compound may be administered before or after the anti-neoplastic agent (i.e., sequentially).

In any of the uses described herein, administration of the tellurium- containing compound and optionally of additional active agents (e.g., anti-neoplastic agents) can be performed via various routes of administrations.

Suitable routes of administration may, for example, include the inhalation, oral, buccal, rectal, transmucosal, transdermal, intradermal, transnasal, intestinal and/or parenteral routes; the intramuscular, subcutaneous and/or intramedullary injection routes; the intrathecal, direct intraventricular, intravenous, intraperitoneal, intranasal, and/or intraocular injection routes; and/or the route of direct injection into a tissue region of a subject.

As used herein, the term "therapeutically effective amount" or "pharmaceutically effective amount" denotes that dose of an active ingredient or a composition comprising the active ingredient that will provide the therapeutic effect for which the active ingredient is indicated. More specifically, a therapeutically effective amount means an amount of active ingredients effective to prevent, alleviate or ameliorate symptoms of disease or prolong the survival of the subject being treated.

Determination of a therapeutically effective amount is well within the capability of those skilled in the art.

For any preparation used in the methods of the invention, the therapeutically effective amount or dose can be estimated initially from in vitro assays. For example, a dose can be formulated in animal models and such information can be used to more accurately determine useful doses in humans.

Toxicity and therapeutic efficacy of the active ingredients described herein can be determined by standard pharmaceutical procedures *in vitro,* in cell cultures or experimental animals. The data obtained from these *in vitro* and cell culture assays and animal studies can be used in formulating a range of dosage for use in human. The dosage may vary depending upon the dosage form employed and the route of administration utilized. The exact formulation, route of administration and dosage can be chosen by the individual physician in view of the patient’s condition. [See e.g., Fingl, et al., (1975) "The Pharmacological Basis of Therapeutics", Ch. 1 p.1].

Depending on the severity and responsiveness of the condition to be treated, dosing can be of a single or a plurality of administrations, with course of treatment lasting from several days to several weeks or until cure is effected or diminution of the disease state is achieved.

When administering systemically, a therapeutically effective amount of the tellurium-containing compounds described herein may range, for example, from about 0.01 mg/m$^2$/day to about 20 mg/m$^2$/day and thus can be for example, 0.01 mg/m$^2$/day, 0.02 mg/m$^2$/day, 0.03 mg/m$^2$/day, 0.04 mg/m$^2$/day, 0.05 mg/m$^2$/day, 0.1 mg/m$^2$/day, 0.5 mg/m$^2$/day, 1 mg/m$^2$/day, 2 mg/m$^2$/day, 3 mg/m$^2$/day, 4 mg/m$^2$/day, 5 mg/m$^2$/day, and up to 10 mg/m$^2$/day. Preferably, for systemic administration, a therapeutically effective amount of a compound of formula I, II, III or IV ranges from about 0.01 mg/m$^2$/day to about 10 mg/m$^2$/day. Higher therapeutically effective amounts, such as, for example, up to 20 mg/m$^2$/day can also be employed.

In one embodiment, when administered intraperitoneally, the therapeutically effective amount is 0.01 mg/m$^2$/day and higher and thus can be, for example,0.01 mg/m$^2$/day, 0.05 mg/m$^2$/day, 0.1 mg/m$^2$/day, 0.2 mg/m$^2$/day, 0.5 mg/m$^2$/day, 0.6 mg/m$^2$/day, 0.7 mg/m$^2$/day, 0.8 mg/m$^2$/day, 0.9 mg/m$^2$/day, 1 mg/m$^2$/day, 2 mg/m$^2$/day, 3 mg/m$^2$/day, 4 mg/m$^2$/day, 5 mg/m$^2$/day, and up to 20.0 mg/m$^2$/day. When administered orally in humans, a daily dose typically ranges between 0.1 mg and 200 mg, more preferably between 1 mg and 100 mg, depending on the age and weight of the subject. The total daily dose may be administered as a single dosage, or may be divided into a number of separate doses.

In any of the methods and uses described herein, the tellurium-containing compounds can be utilized in combination with an additional active agent, preferably being advantageous for treating the indicated condition.

For example, any of the methods described herein can further comprise, in addition to administering the tellurium-containing compounds described above, co-administration of an additional active agent. The co-administration can be effected prior to, concomitant with or subsequent to the administration of the tellurium-containing compound. The additional active agent is used for providing an additive beneficial effect in terms of the ailment being treated, conditions

associated with the ailment being treated or other parameters such as psychological effects and prophylactic effects.

As discussed hereinabove, in some embodiments of the methods and uses described herein, the tellurium-containing compound is utilized in combination with an anti-neoplastic agent, as detailed hereinabove. In these embodiments, further active agents can also be utilized for providing an additive beneficial effect in terms of the ailment being treated, conditions associated with the ailment being treated or other parameters such as psychological effects and prophylactic effects.

Exemplary additional active agents according to this embodiment of present invention include, without limitation, one or more, or any combination of, an anti-viral agent, a steroidal anti-inflammatory agent, a non-steroidal anti-inflammatory agent, an antibiotic agent, an anti-diabetic agent, an antihyperglycemic agent, an antimicrobial agent, an anti-obesity agent, an anesthetic agent, an analgesic, an anti-thrombogenic agent, an anti-proliferative agent, a suitable anti-oxidant, an antidepressant, an anti-histamine, a vitamin, and a hormone.

Representative examples of non-steroidal anti-inflammatory agents that are usable in this context of the present invention include, without limitation, oxicams, such as piroxicam, isoxicam, tenoxicam, sudoxicam, and CP-14,304; salicylates, such as aspirin, disalcid, benorylate, trilisate, safapryn, solprin, diflunisal, and fendosal; acetic acid derivatives, such as diclofenac, fenclofenac, indomethacin, sulindac, tolmetin, isoxepac, furofenac, tiopinac, zidometacin, acematacin, fentiazac, zomepirac, clindanac, oxepinac, felbinac, and ketorolac; fenamates, such as mefenamic, meclofenamic, flufenamic, niflumic, and tolfenamic acids; propionic acid derivatives, such as ibuprofen, naproxen, benoxaprofen, flurbiprofen, ketoprofen, fenoprofen, fenbufen, indopropfen, pirprofen, carprofen, oxaprozin, pranoprofen, miroprofen, tioxaprofen, suprofen, alminoprofen, and tiaprofenic; pyrazoles, such as phenylbutazone, oxyphenbutazone, feprazone, azapropazone, and trimethazone. Mixtures of these non-steroidal anti-inflammatory agents may also be employed, as well as the pharmaceutically acceptable salts and esters of these agents.

Representative examples of steroidal anti-inflammatory drugs include, without limitation, corticosteroids such as hydrocortisone, hydroxyltriamcinolone, alpha-methyl dexamethasone, dexamethasone-phosphate, beclomethasone dipropionates, clobetasol valerate, desonide, desoxymethasone, desoxycorticosterone acetate, dexamethasone, dichlorisone, diflorasone diacetate, diflucortolone valerate, fluadrenolone, fluclorolone acetonide, fludrocortisone, flumethasone pivalate, fluosinolone acetonide, fluocinonide, flucortine butylesters, fluocortolone, fluprednidene (fluprednylidene) acetate, flurandrenolone, halcinonide, hydrocortisone acetate, hydrocortisone butyrate, methylprednisolone, triamcinolone acetonide, cortisone, cortodoxone, flucetonide, fludrocortisone, difluorosone diacetate, fluradrenolone, fludrocortisone, diflurosone diacetate, fluradrenolone acetonide, medrysone, amcinafel, amcinafide, betamethasone and the balance of its esters, chloroprednisone, chlorprednisone acetate, clocortelone, clescinolone, dichlorisone, diflurprednate, flucloronide, flunisolide, fluoromethalone, fluperolone, fluprednisolone, hydrocortisone valerate, hydrocortisone cyclopentylpropionate, hydrocortamate, meprednisone, paramethasone, prednisolone, prednisone, beclomethasone dipropionate, triamcinolone, and mixtures thereof.

Non-limiting examples of anesthetic drugs that are suitable for use in the context of the present invention include pharmaceutically acceptable salts of lidocaine, bupivacaine, chlorprocaine, dibucaine, etidocaine, mepivacaine, tetracaine, dyclonine, hexylcaine, procaine, cocaine, ketamine, pramoxine and phenol.

Non-limiting examples of anti-oxidants that are usable in the context of the present invention include ascorbic acid (vitamin C) and its salts, ascorbyl esters of fatty acids, ascorbic acid derivatives (e.g., magnesium ascorbyl phosphate, sodium ascorbyl phosphate, ascorbyl sorbate), tocopherol (vitamin E), tocopherol sorbate, tocopherol acetate, other esters of tocopherol, butylated hydroxy benzoic acids and their salts, 6-hydroxy-2,5,7,8-tetramethylchroman-2-carboxylic acid (commercially available under the trade name Trolox$^{R}$), gallic acid and its alkyl esters, especially propyl gallate, uric acid and its salts and alkyl esters, sorbic acid and its salts, lipoic acid, amines (e.g., N,N-diethylhydroxylamine, amino-guanidine), sulfhydryl compounds (e.g., glutathione), dihydroxy fumaric acid and its salts, lycine pidolate, arginine pilolate, nordihydroguaiaretic acid, bioflavonoids, curcumin, lysine, methionine, proline, superoxide dismutase, silymarin, tea extracts, grape skin/seed extracts, melanin, and rosemary extracts.

Non-limiting examples of antidepressants usable in the context of the present invention include norepinephrine-reuptake inhibitors ("NRIs"), selective-serotonin-reuptake inhibitors (SSRIs), monoamine-oxidase inhibitors (MAOIs), serotonin-and-noradrenaline-reuptake inhibitors ("SNFIs), corticotropin-releasing factor (CRF) antagonists, $\alpha$-adrenoreceptor antagonists, NK1-receptor antagonists, 5-$HT_{1A}$-receptor agonist, antagonists, and partial agonists and atypical antidepressants, as well as norepinephrine-reuptake inhibitors such as, but are not limited to amitriptyline, desmethylamitriptyline, clomipramine, doxepin, imipramine, imipramine-oxide, trimipramine; adinazolam, amiltriptylinoxide, amoxapine, desipramine, maprotiline, nortriptyline, protriptyline, amineptine, butriptyline, demexiptiline, dibenzepin, dimetacrine, dothiepin, fluacizine, iprindole, lofepramine, melitracen, metapramine, norclolipramine, noxiptilin, opipramol, perlapine, pizotyline, propizepine, quinupramine, reboxetine, tianeptine, and serotonin-reuptake inhibitors such as, but are not limited to, binedaline, m-chloropiperzine, citalopram, duloxetine, etoperidone, femoxetine, fluoxetine, fluvoxamine, indalpine, indeloxazine, milnacipran, nefazodone, oxaflazone, paroxetine, prolintane, ritanserin, sertraline, tandospirone, venlafaxine and zimeldine.

Non-limiting examples of vitamins usable in the context of the present invention include vitamin A and its analogs

and derivatives: retinol, retinal, retinyl palmitate, retinoic acid, tretinoin, iso-tretinoin (known collectively as retinoids), vitamin E (tocopherol and its derivatives), vitamin C (L-ascorbic acid and its esters and other derivatives), vitamin $B_3$ (niacinamide and its derivatives), alpha hydroxy acids (such as glycolic acid, lactic acid, tartaric acid, malic acid, citric acid, etc.) and beta hydroxy acids (such as salicylic acid and the like).

Non-limiting examples of antihistamines usable in the context of the present invention include chlorpheniramine, brompheniramine, dexchlorpheniramine, tripolidine, clemastine, diphenhydramine, promethazine, piperazines, piperidines, astemizole, loratadine and terfenadine.

Suitable hormones for use in the context of the present invention include, for example, androgenic compounds and progestin compounds.

Representative examples of androgenic compounds include, without limitation, methyltestosterone, androsterone, androsterone acetate, androsterone propionate, androsterone benzoate, androsteronediol, androsteronediol-3-acetate, androsteronediol-17-acetate, androsteronediol 3-17-diacetate, androsteronediol-17-benzoate, androsteronedione, androstenedione, androstenediol, dehydroepiandrosterone, sodium dehydroepiandrosterone sulfate, dromostanolone, dromostanolone propionate, ethylestrenol, fluoxymesterone, nandrolone phenpropionate, nandrolone decanoate, nandrolone furylpropionate, nandrolone cyclohexane-propionate, nandrolone benzoate, nandrolone cyclohexanecarboxylate, androsteronediol-3-acetate-1-7-benzoate, oxandrolone, oxymetholone, stanozolol, testosterone, testosterone decanoate, 4-dihydrotestosterone, 5$\alpha$-dihydrotestosterone, testolactone, 17$\alpha$-methyl-19-nortestosterone and pharmaceutically acceptable esters and salts thereof, and combinations of any of the foregoing.

Representative examples of progestin compounds include, without limitation, desogestrel, dydrogesterone, ethynodiol diacetate, medroxyprogesterone, levonorgestrel, medroxyprogesterone acetate, hydroxyprogesterone caproate, norethindrone, norethindrone acetate, norethynodrel, allylestrenol, 19-nortestosterone, lynoestrenol, quingestanol acetate, medrogestone, norgestrienone, dimethisterone, ethisterone, cyproterone acetate, chlormadinone acetate, megestrol acetate, norgestimate, norgestrel, desogrestrel, trimegestone, gestodene, nomegestrol acetate, progesterone, 5$\alpha$-pregnan-3$\beta$,20$\alpha$-diol sulfate, 5$\alpha$-pregnan-3$\beta$,20$\beta$-diol sulfate, 5$\alpha$-pregnan-3$\beta$-ol-20-one, 16,5$\alpha$-pregnen-3$\beta$-ol-20-one, 4-pregnen-20$\beta$-ol-3-one-20-sulfate, acetoxypregnenolone, anagestone acetate, cyproterone, dihydrogesterone, flurogestone acetate, gestadene, hydroxyprogesterone acetate, hydroxymethylprogesterone, hydroxymethyl progesterone acetate, 3-ketodesogestrel, megestrol, melengestrol acetate, norethisterone and mixtures thereof.

In any of the different embodiments described herein, the tellurium-containing compounds described herein can be utilized either *per se,* or as part of a pharmaceutical composition where it is mixed with a pharmaceutically acceptable carrier.

As used herein a "pharmaceutical composition" refers to a preparation of one or more of the active ingredients described herein with other chemical components such as physiologically suitable carriers and excipients. The purpose of a pharmaceutical composition is to facilitate administration of a compound to the subject treated.

Hereinafter, the phrases "physiologically acceptable carrier" and "pharmaceutically acceptable carrier" which may be interchangeably used refer to a carrier or a diluent that does not cause significant irritation to the subject and does not abrogate the biological activity and properties of the administered compound. As used herein, the term "carrier" refers to a diluent, adjuvant, excipient, or vehicle with which the therapeutic is administered.

Herein the term "excipient" refers to an inert substance added to a pharmaceutical composition to further facilitate administration of an active ingredient. Examples, without limitation, of excipients include calcium carbonate, calcium phosphate, various sugars and types of starch, cellulose derivatives, gelatin, vegetable oils and polyethylene glycols.

Pharmaceutically acceptable carriers or diluents may be, for example, binders, (e.g., syrup, gum Arabic, gelatin, sorbitol, tragacanth, polyvinylpyrrolidone, etc), excipients (e.g., lactose, sucrose, corn starch, sorbitol), lubricants (e.g., magnesium stearate, talc, polyethylene glycol, silica, etc.), disintegrants (e.g. microcrystalline cellulose, potato starch, etc.), wetting agents (e.g. sodium lauryl sulfate, etc.), and the like. These pharmaceutical preparations may be in the form of a solid preparation such as tablets, capsules, powders, etc., or in the form of a liquid preparation such as solution, suspension, emulsion, etc., when administered orally. When administered parenterally, the pharmaceutical preparations may be in the form of a suppository, an injection or an intravenous drip, a physiological salt solution, and so on.

Therapeutic application of the tellurium-containing compounds can be contemplated to be accomplished by any suitable therapeutic method and technique presently or prospectively known to those skilled in the art. The tellurium-containing compound may be administered in a variety of forms. These include orally, parenterally, rectally, nasally, topically or via inhalation. The parenteral route of administration may be intravenous, subcutaneous, intramuscular, etc.

Dosages can be titrated to the individual patient. The dose of a tellurium-containing compound varies depending on the exact formulation, route of administration, ages, weights and condition of individual patients, or the severity of the disease.

Depending on the severity and responsiveness of the condition to be treated, dosing can be of a single or a plurality of administrations, with course of treatment lasting from several days to several weeks or until cure is effected or diminution of the disease state is achieved.

Pharmaceutical compositions of the present invention may be manufactured by processes well known in the art, e.g., by means of conventional mixing, dissolving, granulating, dragee-making, levigating, emulsifying, encapsulating, entrapping or lyophilizing processes.

Pharmaceutical compositions for use in accordance with the present invention may be formulated in conventional manner using one or more physiologically acceptable carriers comprising excipients and auxiliaries, which facilitate processing of the active ingredients into preparations which, can be used pharmaceutically. Proper formulation is dependent upon the route of administration chosen.

For injection, the active ingredients may be formulated in aqueous solutions, preferably in physiologically compatible buffers such as Hank's solution, Ringer's solution, or physiological salt buffer.

For oral administration, the compounds can be formulated readily by combining the active compounds with pharmaceutically acceptable carriers well known in the art. Such carriers enable the compounds of the invention to be formulated as tablets, pills, dragees, capsules, liquids, gels, syrups, slurries, suspensions, and the like, for oral ingestion by a patient. Pharmacological preparations for oral use can be made using a solid excipient, optionally grinding the resulting mixture, and processing the mixture of granules, after adding suitable auxiliaries if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose; and/or physiologically acceptable polymers such as polyvinylpyrrolidone (PVP). If desired, disintegrating agents may be added, such as crosslinked polyvinyl pyrrolidone, agar, or alginic acid or a salt thereof such as sodium alginate.

Dragee cores are provided with suitable coatings. For this purpose, concentrated sugar solutions may be used which may optionally contain gum arabic, talc, polyvinyl pyrrolidone, carbopol gel, polyethylene glycol, titanium dioxide, lacquer solutions and suitable organic solvents or solvent mixtures. Dyestuffs or pigments may be added to the tablets or dragee coatings for identification or to characterize different combinations of active compound doses.

Pharmaceutical compositions, which can be used orally, include push-fit capsules made of gelatin as well as soft, sealed capsules made of gelatin and a plasticizer, such as glycerol or sorbitol. The push-fit capsules may contain the active ingredients in admixture with filler such as lactose, binders such as starches, lubricants such as talc or magnesium stearate and, optionally, stabilizers. In soft capsules, the active ingredients may be dissolved or suspended in suitable liquids, such as fatty oils, liquid paraffin, or liquid polyethylene glycols. In addition, stabilizers may be added. All formulations for oral administration should be in dosages suitable for the chosen route of administration.

For buccal administration, the compositions may take the form of tablets or lozenges formulated in conventional manner.

For administration by nasal inhalation, the active ingredients for use according to the present invention are conveniently delivered in the form of an aerosol spray presentation from a pressurized pack or a nebulizer with the use of a suitable propellant, e.g., dichlorodifluoromethane, trichlorofluoromethane, dichloro-tetrafluoroethane or carbon dioxide. In the case of a pressurized aerosol, the dosage unit may be determined by providing a valve to deliver a metered amount. Capsules and cartridges of, e.g., gelatin for use in a dispenser may be formulated containing a powder mix of the compound and a suitable powder base such as lactose or starch.

The preparations described herein may be formulated for parenteral administration, e.g., by bolus injection or continuous infusion. Formulations for injection may be presented in unit dosage form, e.g., in ampoules or in multidose containers with optionally, an added preservative. The compositions may be suspensions, solutions or emulsions in oily or aqueous vehicles, and may contain formulatory agents such as suspending, stabilizing and/or dispersing agents.

Pharmaceutical compositions for parenteral administration include aqueous solutions of the active preparation in water-soluble form. Additionally, suspensions of the active ingredients may be prepared as appropriate oily or water based injection suspensions. Suitable lipophilic solvents or vehicles include fatty oils such as sesame oil, or synthetic fatty acids esters such as ethyl oleate, triglycerides or liposomes. Aqueous injection suspensions may contain substances, which increase the viscosity of the suspension, such as sodium carboxymethyl cellulose, sorbitol or dextran. Optionally, the suspension may also contain suitable stabilizers or agents which increase the solubility of the active ingredients to allow for the preparation of highly concentrated solutions.

Alternatively, the active ingredient may be in powder form for constitution with a suitable vehicle, e.g., sterile, pyrogen-free water based solution, before use.

The preparation of the present invention may also be formulated in rectal compositions such as suppositories or retention enemas, using, e.g., conventional suppository bases such as cocoa butter or other glycerides.

The amount of a composition to be administered will, of course, be dependent on the subject being treated, the severity of the affliction, the manner of administration, the judgment of the prescribing physician, etc.

Compositions of the present invention may, if desired, be presented in a pack or dispenser device, such as an FDA approved kit, which may contain one or more unit dosage forms containing the active ingredient. The pack may, for example, comprise glass, plastic foil, such as a blister pack. The pack or dispenser device may be accompanied by instructions for administration. The pack or dispenser may also be accommodated by a notice associated with the

container in a form prescribed by a governmental agency regulating the manufacture, use or sale of pharmaceuticals, which notice is reflective of approval by the agency of the form of the compositions or human or veterinary administration. Such notice, for example, may be of labeling approved by the U.S. Food and Drug Administration for prescription drugs or of an approved product insert.

In one embodiment, the pharmaceutical composition described herein is packaged in a packaging material and identified in print, in or on said packaging material, for use in the treatment of a condition as described herein.

Optionally, the pharmaceutical composition is further identified for use in combination with the anti-neoplastic agent.

In one embodiment, a concentration of tellurium-containing compound in the carrier ranges from about 0.01 weight percent to about 50 weight percents, more preferably from about 0.1 weight percent to about 25 weight percents, of the total weight of the composition.

Optionally, the pharmaceutical composition may further comprise at least one additional active agent, as described herein.

The pharmaceutical composition may optionally further comprise at least one ingredient such as a humectant, a deodorant agent, an antiperspirant, a sun screening agent, a sunless tanning agent, a pH adjusting agent, a chelating agent, a preservative, an emulsifier, an occlusive agent, an emollient, a thickener, a solubilizing agent, a penetration enhancer, an anti-irritant, a colorant, a propellant and a surfactant.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

## EXAMPLES

Reference is now made to the following examples, which together with the above descriptions, illustrate the invention in a non limiting fashion.

### *MATERIALS AND METHODS*

#### *Chemicals:*

AS101 (ammonium trichloro(dioxyethylene-O,O')tellurate) was prepared as described, for example, in Synthesis, Aug. 1989, page 635.

A stock solution of AS101 was prepared by diluting AS101 in PBS (phosphate buffered saline) to a concentration of 170 μg/ml, which was diluted in sample medium to obtain the final concentrations given hereinbelow.

SAS ($[TeO_4(COCH)_2]_2$) was prepared as described in Albeck et al., ChemMedChem, Vol. 2, pp. 1601-1606.

A stock solution of SAS was prepared by diluting SAS in PBS (phosphate buffered saline) to a concentration of 300 μg/ml, which was diluted in sample medium to obtain the final concentrations given hereinbelow.

Anti-β-actin antibody was obtained from Santa Cruz;
Anti-integrin antibody (mouse) was obtained from Chemicon International;
Anti-α-tubulin antibody was obtained from Santa Cruz;
Anti-WNV-E antibody was obtained from Chemicon;
Bradford reagent was obtained from Bio-Rad;
BMCC (1-biotinamido-4-(4'-[maleimidoethylcyclohexane]-carboxamido)butane) was obtained from Pierce (Rockford, IL);
BSA (bovine serum albumin) was obtained from Sigma-Aldrich;
Eagle's minimum essential medium (EMEM) was obtained from Beit Haemek (Kibbutz Beit Haemek, Israel);
Endothelial cell growth supplement (ECGS) was obtained from Sigma-Aldrich;
Fetal calf serum (FCS) was obtained from Beit Haemek;
Fibronectin was obtained from Beit Haemek;
FITC-labeled anti-mouse antibody was obtained from Santa Cruz;
Hoechst dye was obtained from Sigma-Aldrich;
Horseradish peroxidase-conjugated anti-mouse and anti-rabbit antibodies were obtained from Jackson Immunoresearch Laboratories Inc.;
αVβ3 integrin was obtained from Chemicon;
L-glutamine was obtained from Gibco;
LPS (lipopolysaccharisde) was obtained from Sigma-Aldrich;
Lysis buffer was prepared as described previously [Kalechman et al, Int. J. Cancer, 86:281, 2000];
Streptomycin sulfate was obtained from Gibco;

TRITC-phalloidin was obtained from Sigma-Aldrich;
Trypsin-EDTA was obtained from Beit Haemek;
Trypan blue was obtained from Beit Haemek;
C16 and AG73, the laminin-1 peptides, were a gift from Dr. Nomizu's laboratory.

***Cell lines:***

BV-2 cells, a murine microglia cell line, C6 cells, a rattus glioma cell line, and LAN-1 cells, a murine neuroblastoma cell line, were maintained in RPMI (Roswell Park Memorial Institute) medium supplemented with 10 % fetal calf serum (FCS), 1 % L-glutamine and 1 % antibiotic, as culture media.

SVEC4-10, a murine endothelial cell line, C2C12 , a murine fibroblast cell line, and B16F10, a murine melanoma cell line, were maintained in DMEM (Dulbecco Modified Eagle's Medium) supplemented with 10 % fetal calf serum, 1 % L-glutamine and 1 % antibiotic, as culture media (all cell lines are adherent).

Vero cells and BHK-21 cells (American Type Culture Collection (ATCC), Manassas, VA) were maintained in Eagle's minimum essential medium (EMEM) containing 10 % inactivated FCS, 2 mM L-glutamine, 100 U/ml penicillin G sodium, and 100 μg/ml streptomycin sulfate.

BGM cells (ATCC) were grown in Medium 199 (M199) supplemented as described above for Vero and BHK-21 cells. Viral infection studies were performed in 2 % FCS medium because of virus growth requirements.

***Viral strain:***

Infectious WNV strain M-552/02 is a mosquito isolate from 2002 obtained from the virus collection held at the Central Virology Laboratory, Sheba Medical Center, Tel Hashomer, Israel. This virus was genetically similar to the New York 1999 (NY99) strain. To prepare a virus stock, confluent monolayers of Vero cells were infected with WNV at a multiplicity of infection (MOI) of approximately 5 PFU/cell. At 96 hours post-infection, when significant cytopathic effect (CPE) was observed, the supernatants were harvested, centrifuged to remove cell debris, aliquoted, snap-frozen, and stored at -80 °C. The titer of the virus preparation was determined on Vero cells by plaque assay of 10-fold serial dilutions. Plaques were counted after 3-5 days of incubation at 37 °C, 5 % $CO_2$. The stock WNV titer was 5 x $10^8$ PFU/ml.

***Experimental procedures:***

All cells were maintained in the indicated culture media in Falcon plastic 10 cm tissue culture plates at 37 °C and 5 % $CO_2$, and were split twice weekly. Before the splitting procedure, cells were washed with fresh culture media (DMEM or RPMI), peeled with 0.025 % Trypsin-EDTA (TE), collected to a tube with an additional amount of the culture media, and centrifuged to remove TE. Before each biological test, cells were reconstituted with the culture media supplemented with 10 % serum and were separated by plating 0.3 x $10^6$ - 0.5 x $10^6$ cells per well in 6-well tissue culture plates or 1-2 x $10^6$ cells per 6 mm tissue culture plate. For measurement of NO secretion from BV-2 or SVEC4-10 cells, supernatants from cells treated by different treatments for 48 hours or 24 hours were collected and analyzed by Griess assay. For elucidating the mechanism of action in treated cells, cells were incubated for different incubation times (5 minutes -48 hours) with AS101, SAS, or LPS, for signal transduction analysis. Morphological changes that occur in BV-2 cells were observed in a phase contrast microscope and recorded with a Nikon-F2 camera, enlargement x100 or x200. The images were arranged for comparative analysis with Adobe Photoshop 7.0.

***Cell counts:***

An aliquot of cell suspension was diluted with 0.1 ml of a stain solution, as detailed hereinunder, and cells were then counted in a Neubauer hemocytomenter. Viability was assessed by the trypan blue exclusion method: dead cells absorb the blue stain whereas the cell membrane of viable cells is impermeable to the stain and these therefore appear to be coreless.

The number of cells obtained was calculated using the following formula:

$$N = n \times Y \times V/10^{-4}$$

wherein:

N = total number of cells;
n = number of cells counted in chamber;

Y = dilution factor of stain;
V = volume of resuspended cells in DMEM; and
$10^{-4}$ = volume of counting chamber/ml.

***Preparation of cell extracts:***

Treated cells were collected in a tube, washed twice with cold PBS (phosphate-buffered saline), transferred to an Eppendorf tube and homogenized with a lysis buffer for 30 minutes on ice. The homogenate was centrifuged at 4 °C for 40 minutes at 14,000 rpm to precipitate lipids and DNA. Extracts were transferred to a dry sterile Eppendorf tube and kept at 20 °C until use.

***Bradford assay:***

Quantitative determination of protein concentration was performed using the Bradford assay (Bradford MM 1976). Cell lysate (5 μl) was added to 995 μl of Bradford reagent and mixed gently, and absorbance was read at 595 nm. Readings were compared with a standard curve produced by using a BSA protein concentration range of 1 to 20 μg/ml.

***Western blot analysis of FAK phosphorylation:***

Cell lysates (20 μg) were diluted with an equal volume of 2 x SDS (sodium dodecyl sulfate) sample buffer and separated according to size by SDS-PAGE (SDS-polyacrylamide gel electrophoresis). Proteins were transferred to a polyvinylidene difluoride membrane via electroelution by a transfer system (Bio-Rad), blocked for 1 hour by blocking buffer (5 % skim milk in PBS with 0.05 % Tween 20) and incubated for 2 hours in room temperature or for overnight in 4 °C with primary antibodies diluted in blocking buffer. Membranes were washed several times in PBS with 0.05 % Tween (PBS-T) and incubated for 1 hour with horseradish peroxidase-conjugated anti-mouse or anti-rabbit antibodies (diluted 1:10,000). Proteins were detected by ECL Western Blotting Detection Reagents according to the instructions of the manufacturer (PIERCE).

***Western blot analysis of West Nile Virus (WNV) protein synthesis:***

Vero cells grown in 6-well plates ($2x10^5$ cells/well) were harvested and total cell extracts were prepared by suspension in 50 μl of ice-cold lysis buffer containing 50 mM Tris (pH 8.0), 150 mM NaCl, 1% NP-40, 0.1% SDS, 5 mM EDTA, 0.2 mM PMSF, 50 mM NaF, 200 mM sodium vanadate, 5 mg/ml aprotinin and 5 mg/ml leupeptin. Cell lysates were boiled for 5 minutes and electrophoresed on 8 % SDS-polyacrylamide gel, and were then transferred to a nitrocellulose membrane which was then incubated consecutively with a monoclonal antibody against the WNV envelope (WNV-E) protein and an anti-p-actin or anti-tubulin monoclonal antibody. The Nitrocellulose membranes were developed using horseradish peroxidase-conjugated secondary antibodies and the ECL detection system (Pierce, Rockford, IL). Quantitative evaluation of E protein calibrated against tubulin or β-actin was done by scanning and analysis using the Image-J program (www.//rsb.info.nih.gov/ij).

***Cell attachment assay:***

The cell attachment assays were performed in sextuplicate in 96-well plates. The 1 μg of C16 or AG73 peptide in 60 μl double-distilled water was added to each well, and this volume was allowed to air dry overnight in a biosafety hood at room temperature (or fibronectin 20 ng/80 μl for 1 hour at 37 °C). Thereafter, nonspecific binding sites of the tissue culture plastic were blocked by adding 200 μl of 1 % bovine serum albumin (BSA) in PBS for 1 hour at 37 °C. LAN-1, B16F10 and SVEC4-10 cells were harvested by agitation, washed three times in serum-free DMEM and resuspended in 1 % BSA-DMEM. After 20 minutes incubation in 1 % BSA in a tube, cells were washed and resuspended in 0.1 % BSA. The cells were counted using a hemocytometer and brought to a final cell count of 1 x $10^6$/ml in the 0.1 % BSA medium. Cells were divided into tubes and each tube was treated with one of the following in DMEM: ECGS (endothelial cell growth supplement) (50 μg/ml), AS101 (1-5 μg/ml), SAS (1-5 μg/ml), ECGS (50 μg/ml) + SAS (1-5 μg/ml), ECGS (50 μg/ml) + AS101 (1-5 μg/ml), 10 % serum, 10 % serum + AS101 (1-5 μg/ml) or 10 % serum + SAS (1-5 μg/ml).

Next, 150 μl of the treated cell suspension was added to each well (20,000 cells/well), which had been coated with peptides, and incubated for 50 minutes at 37 °C. After 50 minutes, the unattached cells were removed by aspiration and the remaining attached cells were fixed and stained by crystal violet. 100 μl aliquot of 10 % SDS was thereafter added to each well for cell lysis and dye release and absorption was read with a TECAN spectrophotometer at 570 nm.

***Crystal violet staining:***

Attached cells were washed with PBS without $Ca^{+2}/Mg^{+2}$. 100 μl of 0.2 % crystal violet in 20 % methanol was thereafter added and the cells were incubated for 10 minutes at room temperature. At the end of incubation time, cells were washed twice with double-distilled water and 100 μl of 10 % SDS was added. Dye bound to the cell is released by SDS and the absorption by the dye is measured by colorimetry at 570 nm. The amount of stained cells is then considered to be proportional to the measured absorption by the dye

***Fluorescence staining of cells:***

Before the staining procedure, cells were adhered to cover slips loaded in the wells of a 6-well plate and treated as described above in the Experimental procedures section. At the end point, cells were washed with PBS without $Ca^{+2}/Mg^{+2}$ and fixed in 4 % paraformaldehyde, permeabilized with 0.1 % Triton X-100 and blocked with 5 % fetal calf serum/1 % BSA in PBS (phosphate buffer saline). Next, primary antibody anti-α-tubulin was added for 1 hour, washed three times with PBS-T, and then FITC-labeled anti-mouse secondary antibody was added for 1 hour. Cells were thereafter washed three times and stained with TRITC-phalloidin for 1 hour, washed again, and Hoechst dye, a nuclear stain, was added for 15 minutes. Finally, cells were washed and cover slips were stuck to a slide with mounting solution. Cells with actin fiber stress in randomly chosen fields were scored and expressed as a percentage of the totals amount of cells. Stained cells were counted and photographed with Zeiss ApoTome fluorescent microscope.

***Chick aortic arch assay:***

Aortic arches were removed from fertilized chicken eggs at day 13 of embryonic development. The eggs were cracked into a sterile 100 mm culture dish. The embryo was removed from its surroundings by cutting away the associated membranes and yolk sac. The chicken embryo was placed ventral side up to surgically expose the heart and aortic arches. The heart and aortic arch were removed and placed into a sterile culture dish containing PBS to which 1 % penicillin-streptomycin was added. Arches, from which the surrounding adventitia had been removed, were cut into 0.8 mm sections. Each arch was placed onto drop of Matrigel that was deposited on the bottom of a 48-well culture plate just prior to adding the ring. An additional drop of ice-cold Matrigel was spread on each aortic arch. Matrigel was allowed to solidify before adding 500 μl of human endothelial-SFM basal growth medium. AS101 or SAS (0.1-5 μg/ml in DMEM) was added to wells alone or with ECGS (50 μg/ml) and incubated at 37 °C, 5 % $CO_2$ for 24-48 hours. Quantitative evaluation of tubule formation was performed by a blinded observer on a scale of 1-3 (least to maximum sprouting).

***Chick Chorioallantoic Membrane (CAM) assay:***

Collagen I solution was prepared by mixing 1.8 ml of collagen I, 4.14 μl of 10N NaOH and 0.2 ml of 10 x PBS/200 mM HEPES (pH 7.4). 10 μl of a mixture containing 0.1 ml of the collagen I solution and 0.2 ml of PBS either with or without AS101 (5 μg/ml) or SAS (5 μg/ml) was loaded onto a quarter piece of a Thermonox disc and gelled by warming to room temperature for 15 minutes. The disc was then applied to the chorioallantoic membrane of a 10-days-old embryo, following preparation of the eggs by removing 2 ml of albumin from the 3-days-old embryo and by "opening a window" to the 7-day-old embryo. ECGS (50 μg/ml) in PBS was used to induce angiogenesis and 5μg/ml of AS101 or SAS were mixed with ECGS. After 3-days incubation, the negative or positive response was assessed under a binocular. Positive response, namely the appearance of a typical spokewheel pattern of new blood vessels around the loaded samples, was determined by a blinded observer. Assays for each test sample were carried out three times, and each experiment contains 10 to 12 eggs/data point.

***Tumor Growth in vivo on CAM:***

B16F10 melanoma cells ($10^6$ cells/0.05 μl) in Matrigel were added directly onto the CAM of the 7-days-old embryo (embryos were prepared as described hereinabove). AS101 and SAS (5 μg) were added daily. After a 7-days incubation, with treatment performed each day (PBS, AS 101 or SAS), tumors were excised and weighed. Formalin-fixed tumor tissues were paraffin embedded, sectioned at 10 μm, and stained with hematoxylin and eosin (H&E) for examination of tumor cell proliferation and vessel density.

***Tube formation assay:***

Plates (48 wells) were coated with 150 μl of Matrigel and incubated at 37 °C for 1 hour to promote gelling. Svec4-10 or HUVEC cells (40,000 cells) in 0.5 ml of serum-free DMEM with varying concentration of AS101 or SAS

were added to each well. For positive control, ECGS was added at a concentration of 50 μg/ml. All samples were performed in sextuplicate. After 3 hours incubation, the plates were fixed with methanol and stained with crystal violet. Randomly chosen fields were photographed and density of tube formation was analyzed by imageJ.

***Boyden Chamber migration assay:***

Migration assay was performed in a 48-well microchemotaxis chamber. Polyester membranes with 8 μm pores were coated with 0.1 mg/ml of collagen I in DMEM and then dried for 1 hour. B16F10 and Svec4-10 cells were harvested using TE and resuspended in DMEM containing 0.1 % BSA. The bottom chamber was loaded with Svec4-10 supernatant/DMEM (1:5) containing varying concentration of AS101 or SAS and the filter was laid over the treatment solutions. Non-diluted Svec4-10 supernatant was used as a positive control. The upper wells were then loaded with 30,000 cells, the chamber was incubated at 37 °C for 6 hours and the filters were fixed and stained using Diff-Quick. The cells that migrated through the filter were quantified by counting the center of each well in a 36-box grid at x 20 using an Olympic CK2 microscope. Each condition was studied in triplicate wells, and each experiment was performed three times.

***"Scratch" wound closure assay:***

Confluent Svec4-10 monolayers in 6 mm plates were "scratch" wounded using the tip of a universal blue pipette tip and rinsed with PBS. AS101 or SAS (1 μg/ml final concentration) or ECGS (100 μg/ml) were added to the plates containing serum-free DMEM. The plates were incubated at 37 °C and fixed and stained at several time points. Migrated cells at 12 hours were counted along the scratch. The centers of plates at all times were photographed for presented data. The experiment was repeated three times.

***Mouse plug in vivo migration and angiogenesis assay:***

Matrigel was mixed with ECGS (500 μg/ml) and injected subcutaneously into C57B1/6N female mice on the ventral surface of the animal. During 10 days, every day, AS101 or SAS (20 μg/mouse in Matrigel) were injected subcutaneously. After 10 days, the plugs were removed with some surrounding tissue, fixed in 10 % formalin, and paraffin-embedded. Sections were deparaffinized, and stained with hematoxylin and eosin dyes. In the most positive regions, stained cells were counted by a blinded observer. In each treatment group there were 5-6 mice, and the experiment was performed twice. Images from the sections were captured using a 10X objective of an Olympus microscope.

***ELISA-integrin:***

Plates coated with C16 or fibronectin were blocked with 10 % BSA for 1 hour and washed with PBS. 20 ng/well of integrin with or without AS101/SAS was applied on fibronectin or 2 ng C16 coated well and incubated for 1.5 hours at 37 °C. The wells were thereafter washed with PBS-T and mouse anti-integrin was added thereto for 1 hour at 37 °C. Cells were then washed again and anti-mouse-horseradish peroxidase was added for another 1 hour at 37 °C. Finally, cells were washed, TBM-substrate was added for color development, and absorption was read at 595 nm. Data presented as ± SE, bar *=p<0.005, evaluated by T-test.

***West Nile Virus (WNV) cytopathic effect assay:***

Vero cells were seeded at the density of 2 x $10^4$ cells/well in 96-well plates, 20 hours before infection. The cells were treated with PBS or with various concentrations of AS101 before (1 hour or 5-10 minutes), or after (1 hour or 24 hours) virus inoculation. WNV at MOI (multiplicity of infection) of 0.005 PFU (plaque forming units) per cell was added to the cells and allowed undergo absorption for 1 hour at 37 °C. The medium was removed and the cells were washed twice with PBS and overlaid with fresh medium. Cells were then incubated at 37 °C with 5 % $CO_2$ for 3-5 days. The development of a cytopathic effect was monitored by light microscopy.

***WNV yield assay:***

Vero cells were seeded as described above in 96-well plates. Cells were treated with AS101 and infected with WNV at MOI of 5 PFU/cell. The cells were washed six times with PBS, and fresh medium was added. The virus titer in harvested culture supernatants was determined by a microtitration assay as follows [*Cottey et al.*, *in* Colgan et al., Current Protocols in Immunology, John Wiley & Sons, New York, NY, 2003]: 10-fold serial dilutions of supernatants were used to infect Vero cells in 96-well plates. After 5 day, wells were scored for the presence of a cytopathic effect and the $TCID_{50}$

(tissue culture infectious dose), the dilution at which 50 % of the wells showed a cytopathic effect, was determined. Viral titers (expressed as log10 of $TCID_{50}$/ml) were calculated according to the Spearman-Karber method [*Hierholzer and Killington, in* Mahy and Kangro, Virology Methods Manual, Academic Press, San Diego, CA, 1996].

***WNV plaque assay:***

Vero cells were seeded at a density of 1 x $10^6$ cells in 10 cm dishes 20 hours prior to infection. WNV (5x$10^3$ PFU/ml, MOI = 0.005 PFU/cell) was added to the cultures, and the plates were incubated for 1 hour at 37 °C with 5 % $CO_2$. After incubation, the medium was removed and the cells were washed 6 times with PBS and overlaid with fresh EMEM medium containing 4 % FCS (fetal calf serum) and 1 % (w/v) agar. Cells were then incubated at 37 °C with 5 % $CO_2$ for 3 days until plaques appeared, and then fixed and stained with a solution of 0.5 % crystal violet in a mixture of formalin (1 % v/v) and ethanol (1.65 % v/v). Plaques were counted over a light box after removal of the agar overlay.

***Cell-free virus assay:***

Cells were plated 20 hours before virus infection in 6-well plates at a density of 2 x $10^5$ cells/plate, and treated with AS101 (1-10 μg/ml) for 1 hour or for 5-10 minutes before WNV infection. The cells were infected at MOI = 5 PFU/cell and incubated for 1 hour at 37 °C, 5 % $CO_2$. Supernatants were collected immediately following the incubation, and the concentration of the virus which remained cell-free in the culture supernatant was measured by viral plaque assay, as described above.

***Anti-αVβ3 integrin antibody binding assay:***

An anti-αVβ3 integrin mediated cell adhesion kit (Millipore [Chemicon], Temecula, CA) was used to determine cells attachment to anti-αVβ3 integrin antibody, in the presence or absence of AS101. Briefly, Vero or BGM cells were mixed with PBS or with AS101 (1-10 μg/ml) and were added to anti-αVβ3 integrin coated 96-well plates, and to a control 96-well plate (containing anti-mouse IgG) in triplicate. The plates were incubated at 37 °C, 5 % $CO_2$, for 2 hours, and the cells were then stained and extracted according to the kit instructions, using the staining solution and extraction buffer supplied with the kit. Cell concentration was measured by a spectrophotometer at 540 nm.

***Integrin-WNV attachment assay:***

A solid-phase binding assay was performed according to a method previously described by Sharma et al [Virology, 239:150, 1997]. WNV was diluted in 50 mM carbonate-bicarbonate buffer, pH 9.6, to a concentration of $10^6$ PFU/ml, and 100 μl aliquots were added to the wells of 96-well Nunc-Maxisorp ELISA plates. The plates were incubated overnight at 4 °C, washed three times with washing buffer (20 mM Tris-HCL [pH 7.5], 150 mM NaCl), and then blocked for 2 hours at room temperature with 200 μl of blocking buffer containing 20 mM Tris-HCL, pH 7.5, 150 mM NaCl, 5 % Bovine serum albumin (BSA). The wells were then washed again, and purified αVβ3 integrin (75 ng/well), diluted in binding buffer (20 mM Tris-HCL, pH 7.5, 150 mM NaCl, 5 % BSA, 2 mM $CaCl_2$, 1mM $MgCl_2$) containing various concentrations of AS101, was added to the wells. After 2 hours of incubation at 37 °C, the wells were washed three times with washing buffer. Bound integrin was detected with mouse monoclonal anti-human αVβ3 integrin antibody (Millipore [Chemicon]) diluted 1:1000 in 100 μl binding buffer. Incubation continued for 1 hour at 37 C, and then the wells were washed again. Horseradish peroxidase-conjugated secondary anti-mouse IgG antibody diluted 1:5000 in binding buffer was added to the wells and incubation continued for 45 minutes at 37 °C. The wells were then washed, and o-phenylenediamine dihydrochloride (OPD) tablets diluted in citric acid to the concentration of 1 mg/ml were added for 10 minutes. Colour development was stopped by the addition of 1 N $H_2SO_4$, and plates were read in a spectrophotometer at 492 nm.

***Cell viability assay:***

Cell viability was determined using a cell proliferation kit (Roche), which colors viable cells using the tetrazolium salt XTT.
2x$10^4$ per 200 μl cells were seeded in 96-microwell flat bottom plates. After 24 hours, the tested compound(s) (e.g., AS 101, ARA-C, DTNB, anti-VLA-4 antibody) was added, and the cells were incubated overnight with the tested compound(s). The XTT solution was then added and the cells were incubated for 4 hours at 37 °C. Cell viability was than evaluated by measuring absorbance at 450 nm using a Microplate Spectrophotometer (Spectra MAX Plus, Molecular Devices).

***Statistical analysis:***

Significance of each treatment alone or each combination of treatments was examined by T-test. Data were expressed as mean ± SE and a value of $p<0.05$ was considered to be significant.

## ***EXPERIMENTAL RESULTS***

### ***EXAMPLE 1***

***In vivo angiogenesis assays***

***Chick Chorioallantoic Membrane (CAM) assay:***

Using the CAM assay as an *in vivo* model, AS101 and SAS were tested for possible anti-angiogenic properties. ECGS was used as an angiogenic stimulus on chorioallantoic membranes, showing a 70 % positive response.

The results are presented in FIG's 1A and 1B, and clearly show that AS101 or SAS in combined treatment with ECGS reduced the angiogenic capability of ECGS to baseline levels. *=p<0.05, **=p<0.001. Results express means ± standard error obtained from 10-12 eggs per data point.

***Mouse plug in vivo migration and angiogenesis assay:***

AS 101 or SAS were tested further for anti-angiogenic properties with a mouse plug assay. Matrigel plugs were implanted under mouse skin, and ECGS was used as a chemoattractant for endothelial cells. The endothelial cells migrate, invade and differentiate to induce capillary tubes in the plug. Stained cells were counted by a blinded observer.

The results are presented in FIG 2, and clearly show that AS 101 and SAS were able to inhibit endothelial cell invasion and migration, thus inhibiting angiogenesis.

Results express means ± standard error of two experiments, and p values.

***Tumor Growth in vivo on CAM:***

Cancer cells cause profound changes in the normal neighboring tissue. This altered tissue, also referred to as tumor stroma, provides an environment that favors local tumor growth, invasion and metastatic spreading. Strong evidence indicates that the formation of new blood vessels in the tumor stroma (a process known as tumor angiogenesis) is a critical event promoting tumor progression [www.chuv.ch/cpo_research/integrin.html]. It is known that inhibition of blood vessel formation suppresses tumor growth in experimental tumor models. Therefore, the ability of AS101 or SAS to reduce tumor angiogenesis was tested in a chick *in vivo* model.

The results are presented in FIG's 3A and 3B, and clearly show that AS101 or SAS reduced tumor growth and weight on a chorioallantoic membrane.

Results express means ± standard error. *=p<0.05.

### ***EXAMPLE 2***

***In vitro angiogenesis assays***

***Tube formation assay:***

AS 101 and SAS were tested for the possible ability to prevent capillary-like tube formation in the endothelial cell lines, SVEC4-10 and HUVEC, in a Matrigel matrix.

The results are presented in FIG 4, and clearly show that AS 101 and SAS reduced the number of tubes formed in both cell lines.

***Chick aortic arch assay:***

The chick aortic arch assay was used to investigate the *ex vivo* angiogenic activity of AS101 or SAS. 0.8 mm rings of aortic arches from 13 day old chicken embryos were placed in Matrigel - an extract of basement membrane derived from a murine tumor with components identical, both chemically and immunologically, to authentic basement membrane components. Tubes sprout from arches in Matrigel without any stimuli, and ECGS, which accelerates sprouting, was used as a control. Sprouting vessel length and number were quantified by a blinded observer.

The results are presented in FIG's 5A, 5B and 5C, and clearly show that 0.5-5 μg/ml AS101 or SAS did not inhibit the number of tubes exiting from arches, but reduced their length. AS101 and SAS were found to be non-toxic in this assay, because after washing the AS101 or SAS treated rings they again sprouted vessels (data not shown).
Results express means ± standard error from 4 different experiments. *=p<0.05
The effect of 0.5-5 μg/ml AS 101 and SAS on tube sprouting was also tested in the presence of 100 μg/ml ECGS. The results are presented in FIG's 6A and 6B, and clearly show that AS101 and SAS were found to inhibit the vessel length and number for vessel sprouting induced by ECGS.
Results express means ± standard error from 4 different experiments. *=p<0.05.

***Boyden chamber migration assay:***

The process of angiogenesis includes the migration of endothelial cells through tissue. Therefore, AS101 and SAS were tested for possible effects on the migration of endothelial cells. Cell migration was tested in a Boyden chamber.
The results are presented in FIG 7, and clearly show that AS101 and SAS inhibited the directional migration of murine endothelial cells through the membrane to the chemoattractant. Migration is expressed as a percentage of the migration observed in untreated cells in cell medium.
Results express means ± standard error from 3 different experiments. *=p<0.05, **=p<0.001.

***"Scratch" wound closure assay:***

To confirm the results of the Boyden chamber assay, the effect of AS101 and SAS on cell migration was tested by a "scratch" wound closure assay. Cells were photographed 12, 24 and 36 hours after the wound was formed. Cells were counted 12 hours after the wound was formed.
The results are presented in FIG's 8A and 8B, and clearly show that AS101 and SAS reduce ECGS-induced scratch wound repair by SVEC4-10 cell migration in a monolayer. ECGS was added at 100 μg/ml, AS101 or SAS concentration was 1 μg/ml.
Results express means ± standard error from 3 different experiments. **=p<0.001.

***Cell attachment assay:***

Laminin is essential for basement membrane assembly, promoting cell attachment and angiogenesis. *In vitro,* most structural and functional studies have been performed with laminin-1, the main component of Matrigel. Laminin-1 appears early during epithelial morphogenesis in most tissue of the embryo, and remains present as a major epithelial laminin in some adult tissues.
Synthetic laminin-1 peptides, C16 and AG73, the gift of Dr. H. Kleinmann and Dr. M. Nomizu, already showed their angiogenic properties and ability to attach different cells to basement membrane. Moreover, it is known that C16 peptide works through αVβ3 integrin, the most angiogenic integrin. As AS101 and SAS inhibit blood vessel sprouting from aortic arches and tube formation on Matrigel, it was investigated whether AS101 or SAS can block the attachment of cells to those peptides or inhibit C16 binding to αVβ3 integrin.
The ability of AS101 and SAS to reduce cell attachment to fibronectin and to specific peptides of laminin, C16 and AG73, was investigated by coating plates with the peptides, as shown in FIG 9. After incubating the cells on the plates, unattached cells are removed, and attached cells are quantified by staining with crystal violet, lysing the cells, and reading absorption at 570 nm.
The results are presented in FIG 10, and clearly show that AS101 and SAS inhibited cell attachment to fibronectin in SVEC4-10, LAN-1, BV-2 and RAW cells.
Results express means ± standard error. *=p<0.005.
Further results are presented in FIG's 11A and 11B, and clearly show that AS101 and SAS slightly decrease SVEC4-10 attachment to C16 peptide, as well as to AG73. Cell attachment is quantified as a percentage of the attached cells observed for untreated cells. Results obtained from cell attachment assays for melanoma B16F10 cells and neuroblastoma LAN-1 are similar (data not shown).
Results express means ± standard error. *=p<0.005.

***ELISA-integrin:***

This *in vitro* assay was designed to study the ability of AS101 or SAS to disrupt fibronectin or C16 peptide binding to αVβ3 integrin.
The results are presented in FIG's 12A and 12B, and clearly show that both AS 101 an SAS block fibronectin and C16 peptide binding to αVβ3 integrin at concentrations of 0.5-10 μg/ml.

Results express means ± standard error. *=$p<0.05$.

***Western blot analysis of FAK phosphorylation:***

Cell migration requires continuous assembly and disassembly of cell-ECM or cell-cell contacts, and constant remodeling of the associated actin cytoskeleton. Communication and interaction between adhesion receptors and polymerized actin is therefore essential for important cell responses, such as migration and intracellular signaling that controls cell growth and survival. FAK, which is reported to bind the intracellular regions of β-integrin subunits, plays a pivotal role as a signal integrator downstream of cell-ECM interactions and other receptor and non-receptor tyrosine kinases. FAK responds to integrin engagement by autophosphorylation of its tyrosine-397 which, in turn, creates a binding site for the Src family kinases.

As AS101 and SAS decrease cell attachment to C16 and block C16 binding to αVβ3 integrin, it was necessary to examine the effect of AS101 or SAS on FAK phosphorylation, as a downstream signal from αVβ3 integrin.

The results are presented in FIG's 13A, 13B, 13C and 13D, and clearly show that incubation of resuspended cells in a tube with AS101 or SAS for 5 to 10 minutes causes inhibition of ECGS-induced FAK phosphorylation at Y397. These results were obtained in SVEC4-10, B16F10 and LAN-1 cells.

***EXAMPLE 3***

***Inhibition of West Nile Virus (WNV)-infection***

***West Nile Virus (WNV) cytopathic effect assay:***

The effect of AS101 on WNV infection of Vero cells was determined by observing via light microscopy the effect of AS101 on the cytopathic effect caused by the virus.

As shown in FIG 14, a cytopathic effect caused by West Nile Virus (WNV) is observed, but no cytopathic effect is observed when 2.5 μg/ml AS101 is added along with WNV (WNV+AS101). PBS (phosphate buffer saline) and 2.5 μg/ml AS101 were given as controls. The shown inhibition occurred when AS101 was added 5-10 minutes before inoculation of the cells with the virus. However, AS101 did not exhibit such an inhibitory effect when added 1 hour or 24 hours after inoculation (data not shown).

The effect of AS101 on WNV infection of Vero cells was also determined by measuring cell viability of the infected cells, using crystal violet staining, as described hereinabove.

As shown in FIG 15, AS101 exhibited a dose-dependent protective effect. These results indicate that AS101 protected Vero cells from WNV-mediated cell lysis.

The results express means ± standard error from four experiments are presented as percentages of the viability in PBS-treated, uninfected controls. *=$p<0.05$; **=$p<0.005$.

***WNV plaque assay:***

The effect of AS101 on WNV infection of Vero cells was determined by measuring plaque formation in infected cells. AS101 was added to the cells 1 hour before inoculation with the virus.

Results expressing means ± standard error from six experiments are presented in FIG 16A. *=$p<0.05$; **=$p<0.005$.

Results of a representative experiment of PBS-treated control cells (PBS), WNF-infected cells (WNV) and WNV infected cells treated with 10 μg/ml AS101 (AS101+WNV) are shown in FIG 16B.

As shown in FIG's 16A and 16B, AS101 exhibited a dose-dependent protective effect against the plaque formation caused by WNV infection.

These results confirm that AS101 protected Vero cells from WNV-mediated cell lysis.

***WNV yield assay:***

The effect of AS101 on WNV infection of Vero cells was determined by measuring the yield of WNV released by infected cells into the supernatant.

The cells were treated with 5 μg/ml AS101 1 hour before inoculation with the virus. Following infection, the inoculum was removed and the cells were washed thoroughly. Culture supernatants were collected from infected cells at 12, 24 and 36 hours after infection, and infectious virus yield was determined as described hereinabove.

As shown in FIG 17, AS101 resulted in a 1 log and 2 log decrease in virus yield at 24 hours and 36 hours post-infection, respectively.

Results expressing means ± standard error from three experiments are presented in FIG 17. *=p<0.05; **=p<0.005.

***Western blot analysis of WNV protein synthesis:***

Western Blot analysis was employed to measure the effect of AS101 on WNV protein expression in Vero cells harvested 8 hours after WNV infection. WNV envelope (WNV-E) protein was detected using a specific monoclonal antibody. Expression of WNV-E was normalized relative to tubulin expression.

Results of a representative Western blot are shown in FIG 18A. Results expressing means ± standard error from four experiments are presented in FIG 18B.

As shown in FIG's 18A and 18B, treatment with 2 and 5 μg/ml of AS101 resulted in a dose-dependent 2-3 fold inhibition of virus protein expression as compared to untreated cells. Similar results were obtained in BHK-21 cells, under the same assay conditions (data not shown).

As inhibition was observed during the first replication cycle, these results suggest that AS101 interferes with early events in the virus growth cycle, which is also in line with our finding that AS101 did not inhibit a cytopathic effect in Vero cells when added 1 or 24 hours post-infection, as described herein.

To test the hypothesis that AS101 inhibits virus adsorption and entry into the Vero cells, the level of WNV-E protein was measured in cells immediately (5 and 15 minutes) after virus adsorption. The cells were washed thoroughly and cell extracts were prepared and analyzed by Western blotting. The 5-15 minute time interval allows virus penetration, but is not sufficient for *de-novo* viral protein synthesis [Chu and Ng, J. Virol. 78:10543, 2004]. Therefore, the WNV-E protein detected when assaying after such a time interval originated from infecting cell-bound particles. As shown in FIG 19, treatment with 5 μg/ml of AS101 marked reduced the level of WNV-E protein as compared with untreated cells. These results indicated that AS101 inhibits WNV attachment to Vero cells.

***Cell-free virus assay:***

To further test the hypothesis that AS101 inhibits virus adsorption and entry into the Vero cells, the effect of AS101 on the level of cell-free virus which remained in the culture supernatants 1 hour after virus adsorption to Vero cells was measured.

As shown in FIG 20, culture supernatants collected from cells treated 1 hour before infection with 5 μg/ml and 10 μg/ml of AS101 contained approximately twice the amount of WNV infectious units observed in untreated cells. Similar results were obtained when AS101 was added 5-10 minutes before infection (data not shown).

Results expressing means ± standard error from three experiments are presented in FIG 20. *=p<0.05.

These results indicated that AS101 inhibits WNV attachment to Vero cells.

***Anti-αVβ3 integrin antibody binding assay:***

The αVβ3 integrin presented on the cells' surfaces serves as the cellular receptor for WNV [Chu and Ng, J. Biol. Chem. 279:54533, 2004]. The effect of AS101 on binding of anti-αVβ3 integrin antibody to Vero cells was therefore measured.

As shown in FIG 21, AS101 inhibited the binding of anti-αVβ3 integrin antibody to Vero cells in a dose-dependent manner. Similar results were obtained with BGM cells, with 64 % inhibition by 10 μg/ml of AS101 (data not shown).

Results expressing means ± standard error from three experiments are presented in FIG 21. *=p<0.05.

These results suggest that AS101 interacted with αVβ3 integrin located on the cell surface, and that AS101 acts as an inhibitor of αVβ3 integrin functions.

***Integrin-WNV attachment assay:***

In order to test the hypothesis that the interaction of AS101 with αVβ3 integrin interferes with WNV attachment to its cell receptor, the effect of AS101 on the attachment of immobilized WNV to αVβ3 integrin was measured.

As shown in FIG 22, AS101 directly inhibited the attachment of αVβ3 integrin to WNV in a dose dependent manner.

Each bar in FIG 22 represents the mean ± standard error of three to six experiments. *=p<0.05.

These results confirm that AS101 interferes with attachment to and penetration of cells by WNV, which leads to inhibition of the growth of the virus in the cells.

***EXAMPLE 4***

***Multiple anti-integrin antibody binding assay:***

Although a wealth of evidence shows that inside-out signaling can control activation state, it has been postulated that integrin function may be directly modulated by redox rearrangements within the cysteine-rich domain of the extracellular integrin domain. A disulfide bond reshuffling mechanism is proposed in which resting and active integrins differ in the number and position of unpaired cysteine residues (free thiols). Although the physiological significance of these mechanistic observations, obtained using bifunctional reducing agents, are not clear, the fact that purified integrins have been shown to possess endogenous thiol isomerase activity suggests flexibility in the intermolecular disulfide bonding patterns of a single integrin species. Collectively, these observations suggest that disulfide bonds may not be fixed in integrins and that changes in bonding patterns may be associated with different states of integrin activity.

The specificity of tellurium-containing compounds to integrins was tested by examining the effect of AS101 and SAS on the attachment of SVEC cells to specific anti-integrin antibodies. Commercially available kits (R&D Biosystems) with anti-integrin coated wells were used, and attachment was determined according to the manufacturer's instructions.

As shown in FIG 23, both AS 101 and SAS exhibited considerable inhibition of cell attachment to antibodies against all integrin β subunits of tested, but little if any inhibition of attachment to integrin α subunits.

The results point to an activity of tellurium-containing compounds against β but not α subunits of integrins. Although these results do not provide information about the target of the tellurium-containing compounds' action, nor to their mechanism of action, they suggest an ability to inactivate β subunit activity (containing cysteine-rich domains in the extracellular tail), pointing towards the possibility that deactivation of integrins may be controlled directly by a redox site in the extracellular domain via integrin disulfide exchange. Such a mechanism is characteristic of known activities of AS 1 01 and SAS.

***EXAMPLE 5***

***Inhibition of Acute myeloid leukemia (AML) chemotherapy resistance Acute myeloid leukemia (AML) chemotherapy resistance assay:***

Resistance of many tumors to established treatment regimens constitutes a major problem in cancer therapy. Therefore novel strategies to target tumor cell resistance are essential to improve patient outcome. There exists substantial evidence that the tumor microenvironment may contribute to the failure of standard chemotherapy to eradicate the entire tumor population and may facilitate the emergence of acquired drug resistance. Bone marrow minimal residual disease (MRD) causes relapse after chemotherapy in patients with acute myelogenous leukemia (AML) due to acquired drug resistance. Studies have shown that this resistance is induced by the attachment of the integrin VLA-4 (α4β1 integrin) on leukemic cells to its ligand fibronectin on bone marrow stromal cells.

It was thus hypothesized that tellurium-containing compounds may sensitize human leukemic cells of AML origin to chemotherapy-induced death, via inhibition of resistance induced by the integrin's activity.

It was first ascertained that leukemic cells from AML origin (HL-60 and U937) are more resistant to the chemotherapeutic agents daunorubicin (DRB) and cytosine arabinoside (ARA-C) when cultured on fibronectin (FN) than when cultured on VCAM-1- or BSA-coated plates.

Cell viability, as measured colorimetrically using an XTT assay kit, was determined following treatment, , as described hereinabove.

As shown in FIG's 24A and 24B, both HL-60 and U937 cells are more resistant to ARA-C when cultured on fibronectin-coated plates, in comparison with VCAM-1- and BSA-coated plates.

Similarly, as shown in FIG's 24C and 24D, both HL-60 and U937 cells are more resistant to daunorubicin when cultured on fibronectin-coated plates, in comparison with VCAM-1- and BSA-coated plates.

These results show that the above model is suitable for studying the effect of tellurium-containing compounds on resistance to chemotherapy in AML.

The effect of AS101 on daunorubicin- and ARA-C-induced cell death was then examined.

As shown in FIG 25A, AS101 sensitized HL-60 cells to daunorubicin-induced cell death on fibronectin-coated plates in a dose-dependent manner. *=$p<0.01$ between results with 0.5 or 1 μg/ml AS101 and results without AS101.

However, as shown in FIG's 25B and 25C, AS101 had no visible effect on BSA-coated plates or VCAM-1-coated plates. # =$p<0.01$ relative to control.

Similarly, as shown in FIG's 26A, 26B and 26C, AS101 sensitized HL-60 cells to ARA-C-induced cell death on fibronectin-coated plates, but not on BSA- or VCAM-1-coated plates. *=$p<0.001$ between results with 0.5 or 1 μg/ml AS101 and results without AS101. # =$p<0.001$ relative to control.

Similarly, as shown in FIG's 27A, 27B and 27C, AS101 sensitized U937 cells to ARA-C-induced cell death on fibronectin-coated plates, but not on BSA- or VCAM-1-coated plates. *=$p<0.001$ between results with 0.5 or 1 μg/ml AS101 and results without AS101. # =$p<0.001$ relative to control.

The results suggest that tellurium-containing compounds sensitize AML cancer cells to chemotherapy-induced cell death on fibronectin, thereby reversing the resistance to chemotherapy induced by fibronectin. The failure of AS101 to sensitize cells to cell death on VCAM-1 and BSA may be due to the high sensitivity (i.e., minimal resistance) to chemotherapy in cells grown on VCAM-1 and BSA substrates.

***Fluorescence-basedflow cytometry analysis:***

Fluorescence-based flow cytometry was used to measure the effect of tellurium-containing compounds on chemotherapy-induced cell death. Accumulation of cells in the sub-G1 fraction, increased proportion of annexin V-positive and propidium iodide-positive cells and increased caspase activity were each used as indicators of increased cell death, using standard procedures.

As shown in FIG 28, ARA-C caused a slight increase in the percentage of U937 cells in the sub-G1 fraction (the fraction on the left side of each graph) when the cells were grown on fibronectin, whereas AS101, which had no effect alone, increased the percentage to over 80 % of the cells when combined with ARA-C.

In contrast, ARA-C caused a large percentage of the cells grown on BSA to be observed in the sub-G1 fraction, and the combination of AS101 and ARA-C did not provide significantly different results than ARA-C alone.

As shown in FIG 29A, ARA-C caused a slight increase in the percentage of annexin V-positive (the fraction on the right side of each graph) and propidium iodide-positive (the fraction on the upper half of each graph) U937 cells when the cells were grown on fibronectin, whereas AS101, which had no effect alone, increased the percentage of annexin V-positive and propidium iodide-positive cells considerably when combined with ARA-C.

As shown in FIG 29B, ARA-C caused a large percentage of the cells grown on BSA to be annexin V-positive and propidium iodide-positive, and the combination of AS101 and ARA-C increased the percentage of annexin V-positive and propidium iodide-positive cells only slightly above the percentage obtained with ARA-C alone.

As shown in FIG 30A, ARA-C alone and AS 101 alone had little or no effect on the percentage of U937 cells grown on fibronectin which exhibited high caspase-3 activity, whereas combination of ARA-C and AS101 resulted in a high percentage of cells with increased caspase-3 activity.

As shown in FIG 30B, when grown on BSA, U937 cells exhibited the same degrees of caspase-3 activity when treated with ARA-C alone as when treated with ARA-C and AS101 in combination.

These results confirm that AS101 sensitizes AML cancer cells to chemotherapy-induced cell death when the cells are grown on fibronectin, but not when the cells are grown on BSA.

***Western blot analysis of Akt and Bcl2:***

The effect of tellurium-containing compounds on chemotherapy-induced cell death was further studied by examining the effect of AS101 on the survival factors Akt and Bcl2 in U937 cells.

Phosphorylation of Akt was determined by Western blot analysis.

As shown in FIG 31, AS101 reduced the level of phosphorylated Akt (pAkt), the activated form of Akt, in a dose-dependent manner in cells grown on fibronectin (FN), but not in cells grown on BSA. Total amounts of Akt remained unchanged.

The expression of the survival factor Bcl2 was examined via Western blot analysis in U937 cells treated with the chemotherapeutic agent ARA-C.

As shown in FIG 32, ARA-C did not decrease expression of Bcl2 in cells plated on fibronectin (FN) unless AS101 was added. In contrast, ARA-C reduced expression of Bcl2 considerably in cells plated on BSA, and addition of AS 101 had no effect.

These results are consistent with AS 101 sensitizing cancer cells to chemotherapy in when grown on fibronectin, but not when grown on BSA.

***Effect of anti-VLA antibodies on AML chemotherapy resistance:***

The role of VLA protein in fibronectin-induced resistance to chemotherapy was investigated by performing a chemotherapy resistance assay, as described hereinabove, with anti-VLA-4 and anti-VLA-5 antibodies. U937 cells were grown in fibronectin- and BSA-coated plates. Antibodies were added at a concentration of 1 μg/ml.

As shown in FIG 33A, anti-VLA-4 antibodies did not affect cell viability when given alone, but sensitized cells to ARA-C in cells grown on fibronectin-coated plates. In addition, AS101 had no additional effect in cells sensitized by anti-VLA-4 antibodies. *=$p<0.001$ relative to control. # =$p<0.001$ between results with 0.5 or 1 μg/ml AS101 and results

without AS101.

In contrast, as shown in FIG 33B, neither AS 101 nor anti-VLA-4 antibodies had an effect on cells grown in BSA-coated plates. ## = p<0.001.

These results suggest that VLA-4 ($\alpha 4\beta 1$ integrin), a fibronectin receptor, mediates fibronectin-induced resistance to ARA-C, and that anti-VLA-4 antibodies inhibit this effect of VLA-4, thereby sensitizing cells to ARA-C. The failure of AS101 to add to the effect of anti-VLA-4 antibodies suggests that AS 101 may also act by inhibiting VLA-4.

As shown in FIG's 34A, 34B and 34C, anti-VLA-5 antibodies did not have a significantly different effect than PBS, in cells grown on either fibronectin (FIG 34A), VCAM-1 (FIG 34B) or BSA (FIG 34C).

These results suggest that VLA-5, unlike VLA-4, does not mediate fibronectin-induced resistance to chemotherapy.

***Sensitization of leukemic cells from AML patients to chemotherapy:***

Bone marrow samples from patients diagnosed, with AML, but not yet treated, were obtained and grown *in vitro.* The leukemic cells obtained therefrom were tested for levels of VLA-4 and VLA-5 via fluorescence based cytometry, using FITC (fluorescein isothiocyanate)-conjugated anti-VLA-4 and anti-VLA-5 antibodies, using standard procedures. The cells were grown on both fibronectin and BSA, and the effect of AS101 on chemotherapy resistance was measured.

Representative results are shown in FIG 35.

As shown in FIG 35A, cells from Patient A expressed low levels of VLA-4 and cells from Patient B expressed high levels of VLA-4. The cells from both patients expressed high levels of VLA-5.

As shown in FIG 35B, the viability of cells expressing low levels of VLA-4 (from Patient A) was decreased effectively by ARA-C, when grown on both fibronectin and BSA, whereas cells expressing high levels of VLA-4 (from Patient B) were relatively resistant to ARA-C when grown on fibronectin. As further shown in FIG 35B, AS101 inhibited the resistance to ARA-C of high-VLA-4 cells on fibronectin in a dose-dependent manner.

The mean results $\pm$ standard error obtained with leukemic cells from 8 AML patients with high VLA-4 levels are presented in FIG 36A. The mean results $\pm$ standard error obtained with leukemic cells from 4 AML patients with low VLA-4 levels are presented in FIG 36B.

As shown in FIG 36A, cells expressing high levels of VLA-4 were relatively resistant to ARA-C when grown on fibronectin, and AS101 inhibited the resistance to ARA-C of high-VLA-4 cells on fibronectin in a dose-dependent manner.

As shown in FIG 36B, the viability of cells expressing low levels of VLA-4 was decreased effectively by ARA-C, when grown on both fibronectin and BSA (i.e., no resistance to ARA-C was observed).

# =p<0.001 relative to control. *=p<0.001 between results with 0.5 or 1 $\mu$g/ml AS101 and results without AS101.

These results suggest that only cancer cells expressing high levels of VLA-4 are susceptible to fibronectin-induced resistance to chemotherapy, and that VLA-4 may be an important target for AS101 in preventing this resistance.

***Effect of DTNB (5, 5'-dithiobis-(2-nitrobenzoic acid)) on AML chemotherapy resistance:***

Although a wealth of evidence shows that inside-out signaling via factors in the cytoplasm can control the integrin activation state, an alternative concept now suggests that integrin activation could be controlled directly by a redox site in the extracellular domain, independently of factors in the cytoplasm. Studies have suggested a pivotal role for free sulfhydryls in platelet integrin function, and enzyme-mediated reduction of disulfide bonds on platelets has been implicated.

The role of free thiols in VLA-4/fibronectin-induced resistance to chemotherapy was therefore investigated by performing a chemotherapy resistance assay, as described hereinabove, with the membrane-impermeable thiol blocker DTNB (5mM).

As shown in FIG 37A, 5 mM DTNB sensitizes leukemic cells grown in fibronectin-coated plates to ARA-C-induced cell death.

As shown in FIG 37B, no effect of DTNB is observed in leukemic cells grown in BSA-coated plates.

As AS101 has thiol blocking activity, and the thiol blocker DTNB exhibited similar sensitization activity to that of AS101, it was hypothesized that the sensitization by AS101 of leukemic cells on fibronectin is mediated by blocking free thiol groups.

To test this hypothesis, AS101 was administered along with DTNB to leukemic cells treated with ARA-C. U937 cells grown in either fibronectin- or BSA-coated plates, as well as low-VLA-4 and high-VLA-4 leukemic cells from AML patients were used.

As shown in FIG 38A, the combination of AS101 and DTNB did not sensitize U937 cells grown on fibronectin to a greater degree than did either AS101 or DTNB alone.

As shown in FIG 38B, AS101 and DTNB had no effect either alone or in combination on U937 cells grown on BSA.

Similarly, as shown in FIG 39A, the combination of AS101 and DTNB did not sensitize high-VLA-4 cells grown

on fibronectin to a greater degree than did either AS101 or DTNB alone.

As shown in FIG 39B, AS101 and DTNB had no effect either alone or in combination on low VLA-4 leukemic cells.

These results suggest that AS101-induced sensitization is mediated by the same mechanism as DTNB-induced sensitization, i.e., the blocking of free membranal thiols on VLA-4.

***Western blot analysis of VLA-4 thiols:***

In order to confirm that free thiols are present on the integrin VLA-4 in leukemic cells, and that these are blocked by AS101, U937 leukemic cells cultured on fibronectin or BSA were treated with BMCC (1-biotinamido-4-(4'-[maleimidoethylcyclohexane]-carboxamido)butane), a thiol blocker linked to biotin. Some cultures were treated with AS101. The α and β chains of the VLA-4 (α4β1 integrin) were then immunoprecipitated from the treated cells using either anti-α4 or anti-β1 antibodies and blotted with anti-biotin antibody. The presence of free thiols was indicated by bands observed by anti-biotin blotting. The presence of α4 and β1 chains was determined by blotting with anti-α4 and anti-β1 antibodies.

Dithiothreitol (DTT), a reducing agent, was added to some cultures to reduce disulfides to thiols.

FIG 20 presents results obtained by immunoprecipitation with anti-α4 antibodies (Rows A and B) and with anti-β1 antibodies (Rows C and D). Western blots using antibiotin antibodies are presented in Rows B and D. Blots using anti-α4 and anti-β1 antibodies are presented in Rows A and C, respectively.

As shown in FIG 20, free thiols are found on the α chain, but not the β chain, of the integrin VLA-4 and these are present when cells are cultured in the presence of fibronectin, but not in the presence of BSA. Furthermore, treatment with AS101 eliminated these free thiols.

These results suggest that elimination by AS101 of free thiols on the α4 chain of VLA-4 mediates the AS101-induced sensitization of cancer cells on fibronectin to chemotherapy.

Although considerable evidence suggests that inside-out signalling via factors in the cytoplasm can control integrin activation state, the results presented herein suggest an alternative concept, that integrin activation can be controlled directly by a redox site in the extracellular domain, independent of factors in the cytoplasm. Thus, integrin disulfide exchange may be involved in aspects of integrin activation, altering integrin conformation, unshielding the integrin ligand-binding site.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

In addition, citation or identification of any reference in this application shall not be construed as an admission that such reference is available as prior art to the present invention.

## Claims

1. Use of at least one tellurium-containing compound in the preparation of a medicament for the treatment of an eye disease or disorder **characterized by** ocular neovascularization;
   wherein said at least one tellurium-containing compound is selected from the group consisting of tellurium dioxide ($TeO_2$), a complex of $TeO_2$, a compound having general Formula I:

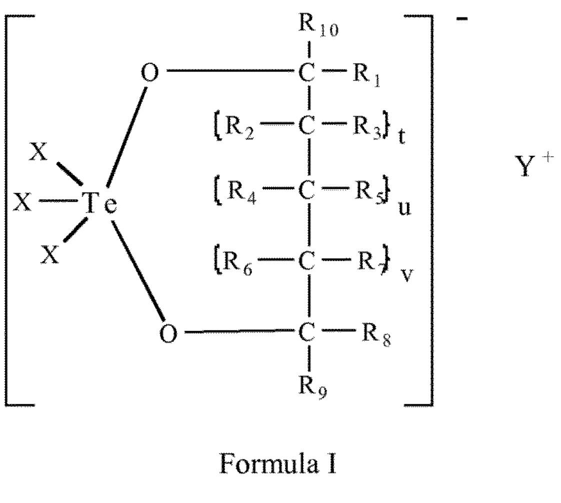

Formula I

a compound having general Formula II:

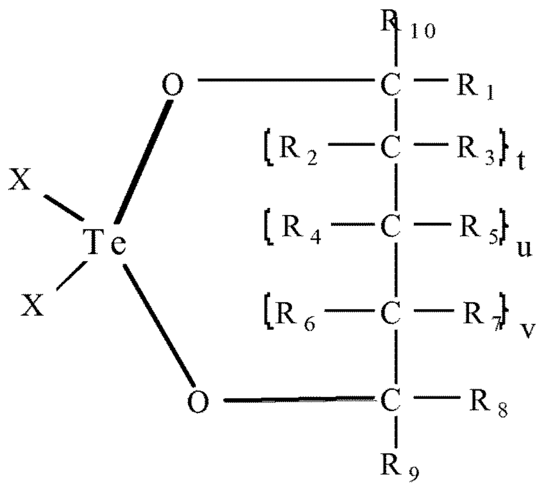

Formula II

a compound having general Formula III:

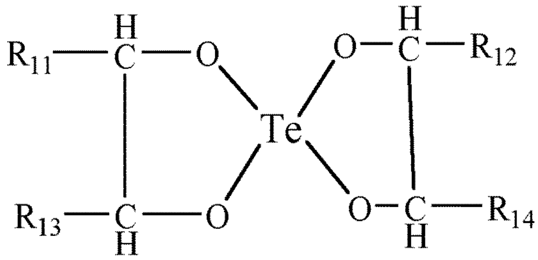

Formula III

and
a compound having general Formula IV:

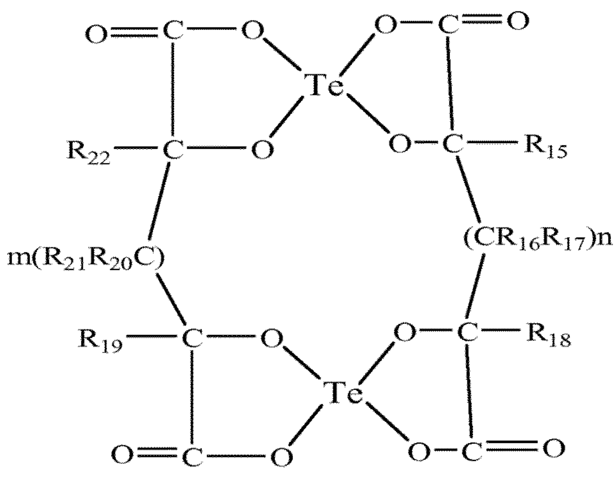

Formula IV

wherein:

each of t, u and v is independently 0 or 1;
each of m and n is independently 0, 1, 2 or 3;
Y is selected from the group consisting of ammonium, phosphonium, potassium, sodium and lithium;
X is a halogen atom; and
each of $R_1$-$R_{22}$ is independently selected from the group consisting of hydrogen, hydroxyalkyl, hydroxy, thiohydroxy, alkyl, alkenyl, alkynyl, alkoxy, thioalkoxy, halogen, haloalkyl, carboxy, carbonyl, alkylcarbonylalkyl, carboxyalkyl, acyl, amido, cyano, N-monoalkylamidoalkyl, N,N-dialkylamidoalkyl, cyanoalkyl, alkoxyalkyl, carbamyl, cycloalkyl, heteroalicyclic, sulfonyl, sulfinyl, sulfate, amine, aryl, heteroaryl, phosphate, phosphonate and sulfoneamido.

**2.** The use of claim 1, wherein said tellurium-containing compound inhibits an integrin comprising an $\alpha$V, $\beta$1, $\beta$2, $\beta$3 or $\beta$6 integrin subunit.

**3.** The use of claim 2, wherein said integrin is $\alpha_v\beta_3$ integrin or VLA-4.

**4.** The use of claim 1, wherein said tellurium-containing compound has general Formula I.

**5.** The use of claim 1, wherein t, u and v are each 0 and wherein each of $R_1$, $R_8$, $R_9$ and $R_{10}$ is hydrogen, wherein X is chloro and wherein Y is ammonium.

**6.** The use of claim 1, wherein said compound has general Formula IV, wherein each of m and n is 0 and wherein each of $R_{15}$, $R_{18}$, $R_{19}$ and $R_{22}$ is hydrogen.

**7.** The use of any of claims 1-6, wherein said eye disease or disorder is diabetic retinopathy, retinopathy of prematurity, neovascular glaucoma, retinoblastoma, retrolental fibroplasia, rubeosis, uveitis, macular degeneration, a disease associated with choroidal neovascularization or iris neovascularization, corneal graft neovascularization, ocular inflammation or an ocular tumor.

**8.** At least one tellurium-containing compound as defined in any of claims 1-6 for use in the treatment of an eye disease or disorder **characterized by** ocular neovascularization in a subject.

**9.** At least one tellurium-containing compound for use according to claim 8 wherein said eye disease or disorder is diabetic retinopathy, retinopathy of prematurity, neovascular glaucoma, retinoblastoma, retrolental fibroplasia, rubeosis, uveitis, macular degeneration, a disease associated with choroidal neovascularization or iris neovascularization, corneal graft neovascularization, ocular inflammation or an ocular tumor.

## Patentansprüche

**1.** Verwendung mindestens einer tellurhaltigen Verbindung bei der Herstellung eines Medikaments zur Behandlung einer durch okulare Neovaskularisation gekennzeichneten Augenerkrankung oder -störung;
wobei die mindestens eine tellurhaltige Verbindung aus der Gruppe bestehend aus Tellurdioxid ($TeO_2$), einem Komplex von $TeO_2$, einer Verbindung der allgemeinen Formel I:

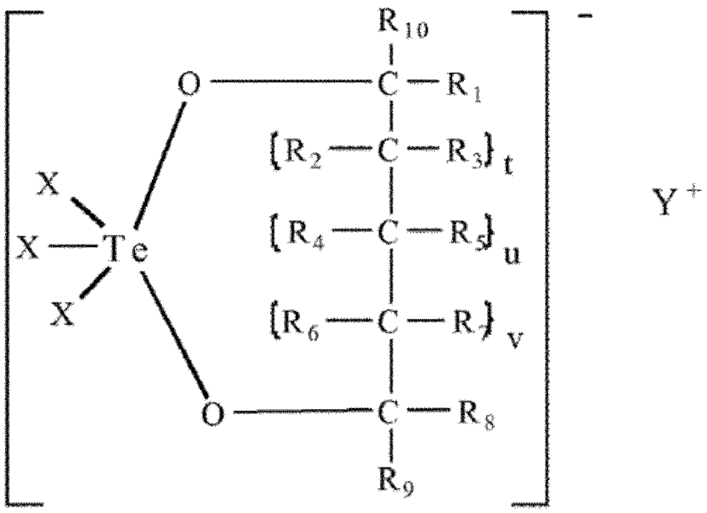

Formel I

einer Verbindung der allgemeinen Formel II:

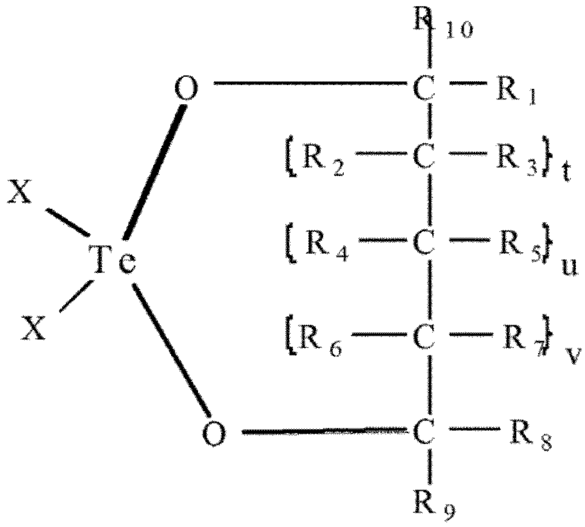

Formel II

einer Verbindung der allgemeinen Formel III:

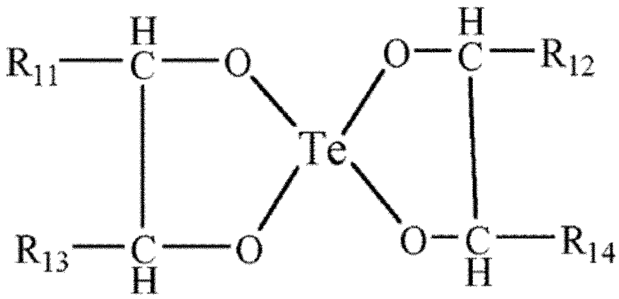

Formel III

und
einer Verbindung der allgemeinen Formel IV:

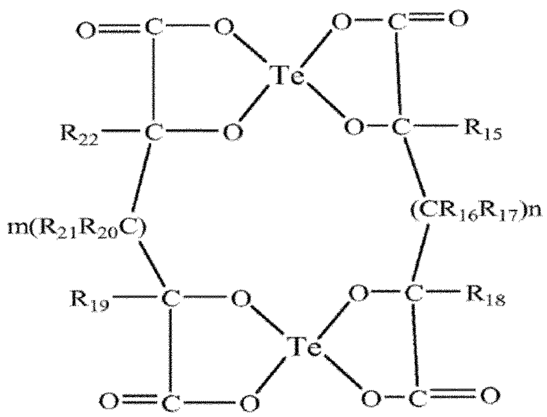

Formel IV

ausgewählt ist,
wobei:

t, u und v jeweils unabhängig 0 oder 1 sind;
m und n jeweils unabhängig 0, 1, 2 oder 3 sind;
Y aus der Gruppe bestehend aus Ammonium, Phosphonium, Kalium, Natrium und Lithium ausgewählt ist;
X ein Halogenatom ist; und
$R_1$-$R_{22}$ jeweils unabhängig aus der Gruppe bestehend aus Wasserstoff, Hydroxyalkyl, Hydroxy, Thiohydroxy, Alkyl, Alkenyl, Alkinyl, Alkoxy, Thioalkoxy, Halogen, Halogenalkyl, Carboxy, Carbonyl, Alkylcarbonylalkyl, Carboxyalkyl, Acyl, Amido, Cyano, N-monoalkylamidoalkyl, N,N-Dialkylamidoalkyl, Cyanoalkyl, Alkoxyalkyl, Carbamyl, Cycloalkyl, Heteroalicyl, Sulfonyl, Sulfinyl, Sulfat, Amin, Aryl, Heteroaryl, Phosphat, Phosphonat und Sulfonamido ausgewählt sind.

**2.** Verwendung nach Anspruch 1, wobei die tellurhaltige Verbindung ein Integrin, das eine $\alpha$V-, $\beta$1-, $\beta$2-, $\beta$3- oder $\beta$6-Integrin-Untereinheit umfasst, hemmt.

**3.** Verwendung nach Anspruch 2, wobei das Integrin $\alpha_v\beta_3$-Integrin oder VLA-4 ist.

**4.** Verwendung nach Anspruch 1, wobei die tellurhaltige Verbindung die allgemeine Formel I hat.

**5.** Verwendung nach Anspruch 1, wobei t, u und v jeweils 0 sind und wobei $R_1$, $R_8$, $R_9$ und $R_{10}$ Wasserstoff sind, wobei X Chlor ist und wobei Y Ammonium ist.

**6.** Verwendung nach Anspruch 1, wobei die Verbindung die allgemeine Formel IV hat, wobei m und n jeweils 0 sind und wobei $R_{15}$, $R_{18}$, $R_{19}$ und $R_{22}$ jeweils Wasserstoff sind.

**7.** Verwendung nach einem der Ansprüche 1 bis 6, wobei die Augenerkrankung oder -störung diabetische Retinopathie, Frühgeborenenretinopathie, ein neovaskuläres Glaukom, ein Retinoblastom, retrolentale Fibroplasie, Rubeose, Uveitis, Makuladegeneration, eine mit Aderhautneovaskularisation oder Irisneovaskularisation assoziierte Erkrankung, Hornhauttransplantatsneovaskularisation, okuläre Entzündung oder ein Augentumor ist.

**8.** Mindestens eine nach einem der Ansprüche 1-6 definierte tellurhaltige Verbindung zur Verwendung bei der Behandlung einer Augenerkrankung oder -störung, die durch eine okuläre Neovaskularisation gekennzeichnet ist, bei einem Subjekt.

**9.** Mindestens eine tellurhaltige Verbindung zur Verwendung nach Anspruch 8, wobei die Augenerkrankung oder -störung diabetische Retinopathie, Frühgeborenenretinopathie, ein neovaskuläres Glaukom, ein Retinoblastom,

retrolentale Fibroplasie, Rubeose, Uveitis, Makuladegeneration, eine mit Aderhautneovaskularisation oder Irisneovaskularisation assoziierte Erkrankung, Hornhauttransplantatsneovaskularisation, okuläre Entzündung oder ein Augentumor ist.

## Revendications

1. Utilisation d'au moins un composé contenant du tellure dans la préparation d'un médicament pour le traitement d'une maladie ou d'un trouble de la vue **caractérisé par** une néovascularisation oculaire ;
dans lequel ledit au moins un composé contenant du tellure est choisi dans le groupe constitué par du dioxyde de tellure (TeO2), un complexe de TeO2, un composé de formule générale I :

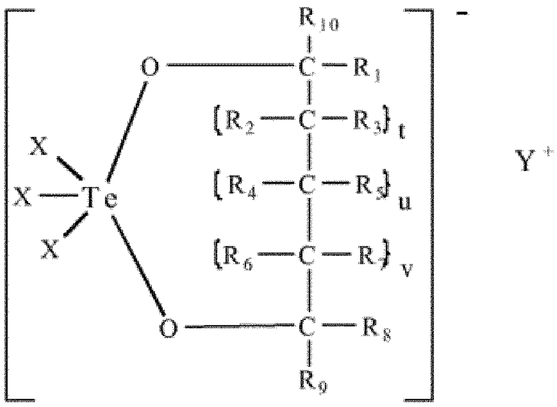

Formule I

un composé de formule générale II :

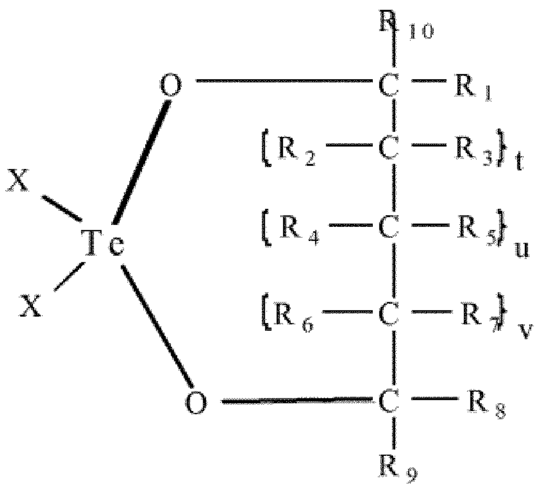

Formule II

un composé de formule générale III :

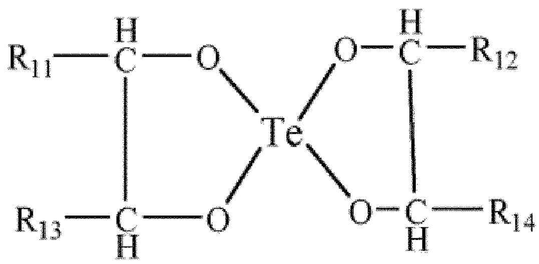

Formule III

et

un composé de formule générale IV:

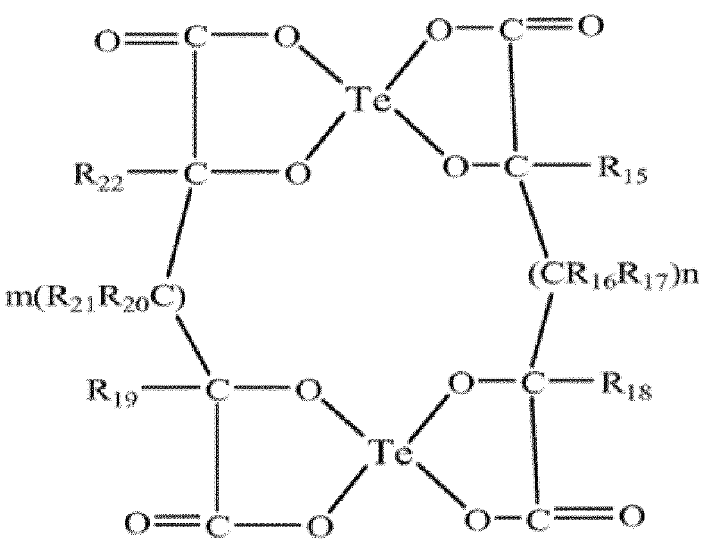

Formule IV

dans lequel :

chacun de t, u et v est indépendamment 0 ou 1 ;
chacun de m et n est indépendamment 0, 1, 2 ou 3 ;
Y est choisi dans le groupe constitué d'ammonium, de phosphonium, de potassium, de sodium et de lithium ;
X est un atome d'halogène ; et
chacun de $R_1$ à $R_{22}$ est indépendamment choisi dans le groupe comprenant hydrogène, hydroxyalkyle, hydroxy, thiohydroxy, alkyle, alcényle, alcynyle, alcoxy, thioalcoxy, halogène, haloalkyle, carboxy, carbonyle, alkylcarbonylalkyle, carboxyalkyle, acyle, amido, cyano, N-monoalkylamidoalkyle, N,N-dialkylamidoalkyle, cyanoalkyle, alcoxyalkyle, carbamyle, cycloalkyle, hétéroalicyclique, sulfonyle, sulfinyle, sulfate, amine, aryle, hétéroaryle, phosphate, phosphonate et sulfoneamido.

**2.** Utilisation selon la revendication 1, dans laquelle ledit composé contenant du tellure inhibe une intégrine comprenant une sous-unité $\alpha$V, $\beta$1, $\beta$2, $\beta$3 ou $\beta$6.

**3.** Utilisation selon la revendication 2, dans laquelle ladite intégrine est l'intégrine $\alpha_v\beta_3$ ou VLA-4.

**4.** Utilisation selon la revendication 1, dans laquelle ledit composé contenant du tellure a la formule générale I.

**5.** Utilisation selon la revendication 1, dans laquelle t, u et v valent chacun 0 et dans laquelle chacun de $R_1$, $R_8$, $R_9$ et $R_{10}$ est de l'hydrogène, dans lequel X est du chlore et dans lequel Y est de l'ammonium.

**6.** Utilisation selon la revendication 1, dans laquelle ledit composé répond à la formule générale IV, dans laquelle

chacun de m et n vaut 0 et dans lequel chacun de $R_{15}$, $R_{18}$, $R_{19}$ et $R_{22}$ est de l'hydrogène.

**7.** Utilisation selon l'une quelconque des revendications 1 à 6, dans laquelle ladite maladie ou ledit trouble de l'oeil est une rétinopathie diabétique, une rétinopathie de prématurité, un glaucome néovasculaire, un rétinoblastome, une fibroplasie rétrolentale, une rubéose, une uvéite, une dégénérescence maculaire, une maladie associée à une néovascularisation choroïdienne ou à une néovascularisation iridienne, à une néovascularisation d'un greffon cornéen, à une inflammation oculaire ou à une tumeur oculaire.

**8.** Composé contenant au moins un tellure tel que défini dans l'une quelconque des revendications 1-6, pour utilisation dans le traitement d'une maladie ou d'un trouble oculaire **caractérisé par** une néovascularisation oculaire chez un sujet.

**9.** Composé contenant au moins un tellure pour utilisation selon la revendication 8, dans lequel ladite maladie ou ledit trouble de la vue est une rétinopathie diabétique, une rétinopathie de prématurité, un glaucome néovasculaire, un rétinoblastome, une fibroplasie rétrolentale, une rubéose, une uvéite, une dégénérescence maculaire, une maladie associée à une néovascularisation choroïdienne ou à une néovascularisation iridienne, à une néovascularisation d'un greffon cornéen, à une inflammation oculaire ou à une tumeur oculaire.

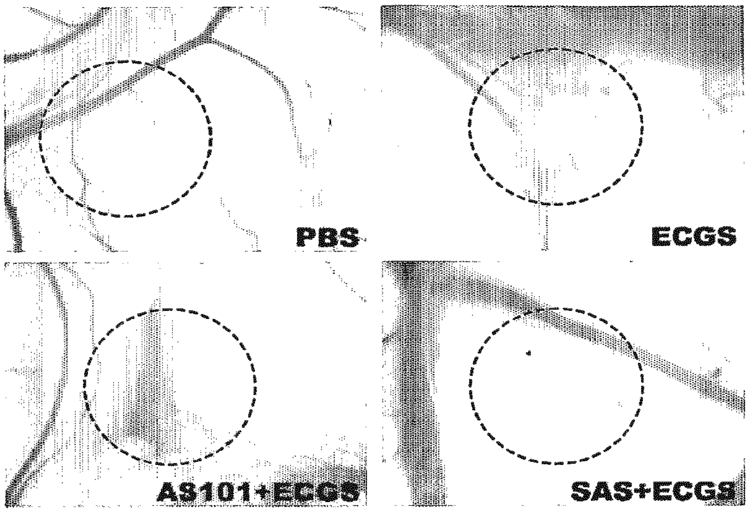
PBS
ECGS
AS101+ECGS
SAS+ECGS

FIG.1A

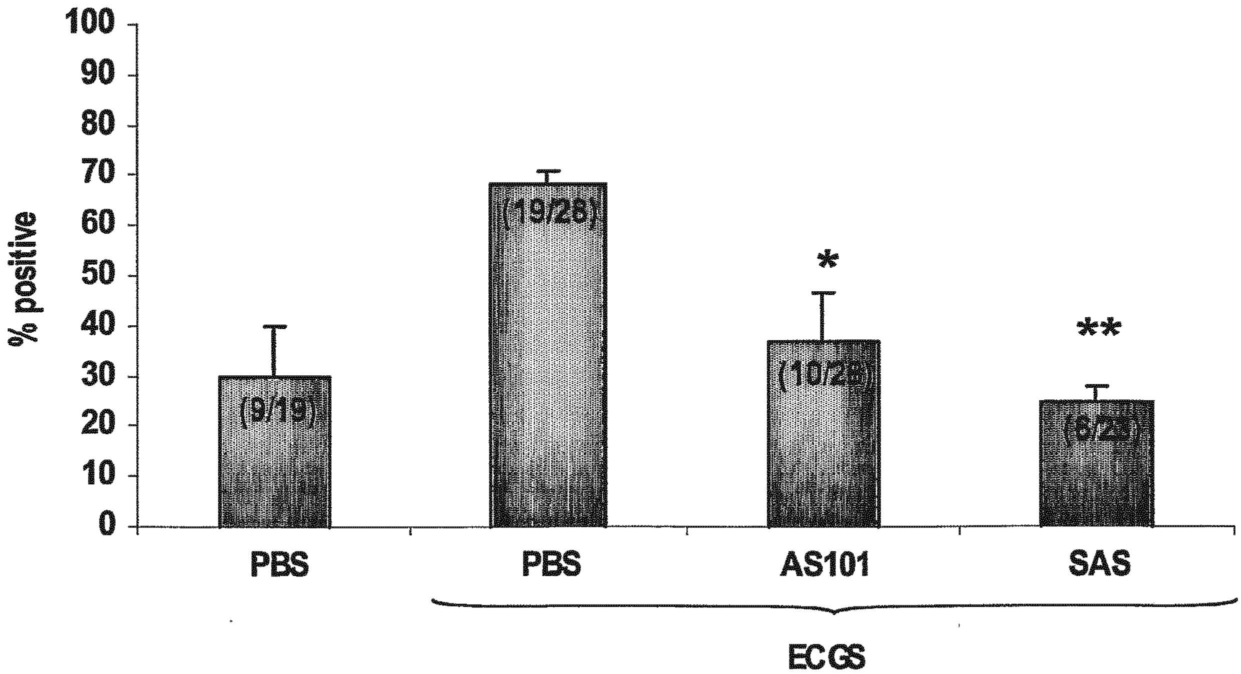
100
90
80
70
60
50
40
30
20
10
0
% positive
(9/19)
(19/28)
(10/28)
(6/28)
*
**
PBS
PBS
AS101
SAS
ECGS

FIG. 1B

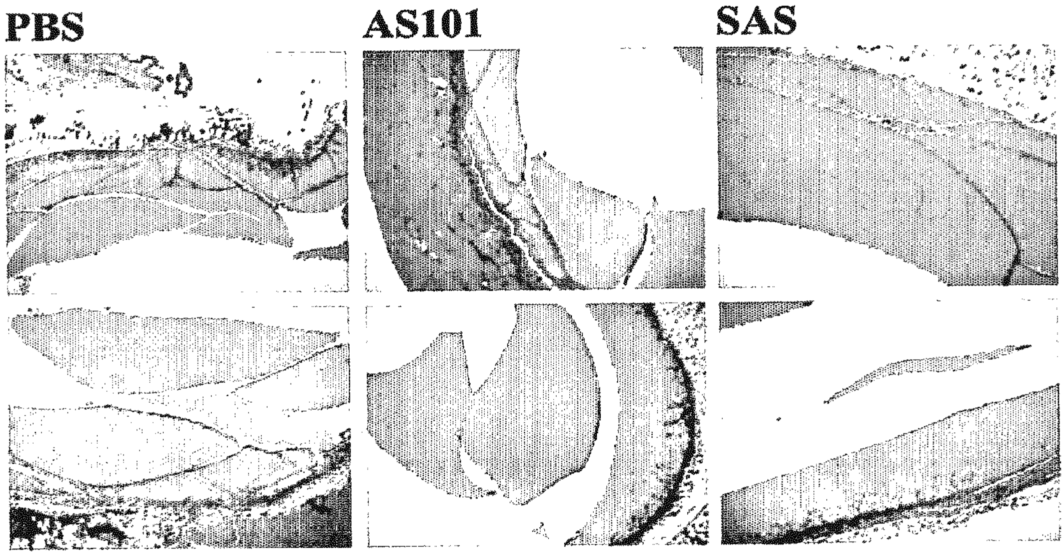


| | PBS | AS101 | SAS |
|---|---|---|---|
| | 50.5+/-18.266 | 20.36+/-3.74 | 26.33+/-3.74 |
| p | | 0.0448 | 0.076 |

CD31/CD34 staining

FIG. 2

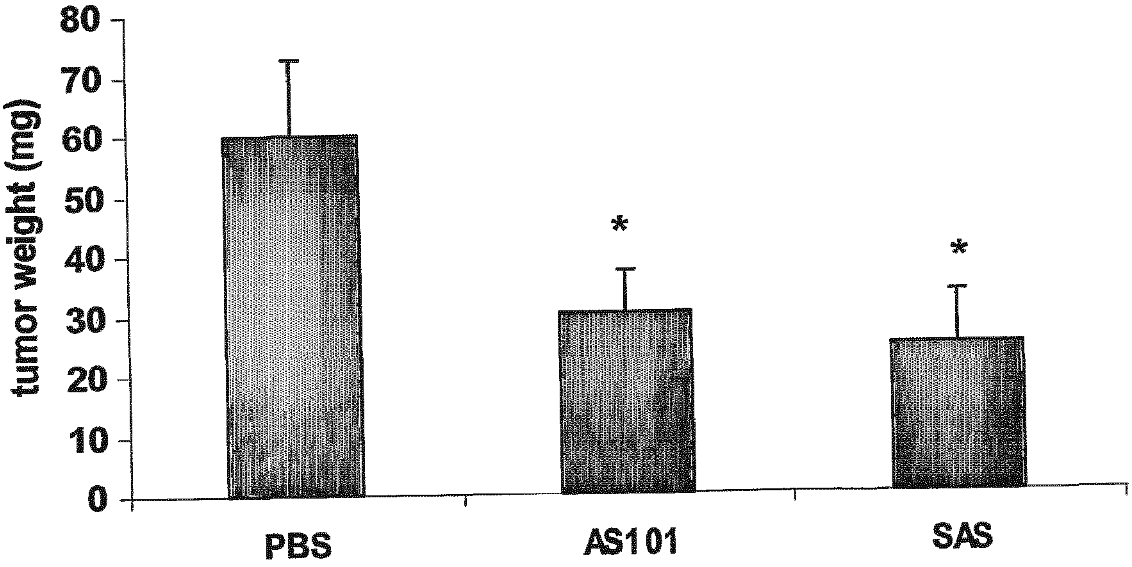


FIG.3A

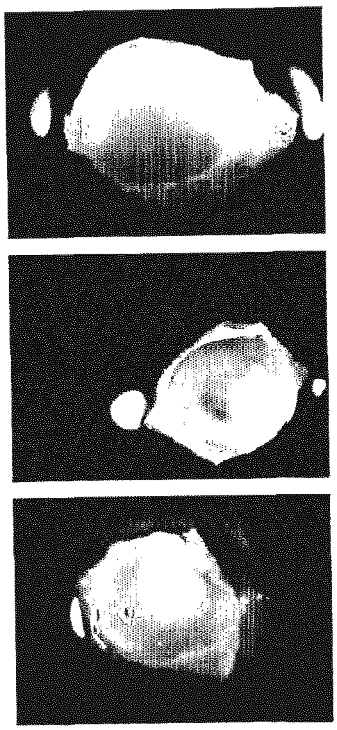

FIG. 3B

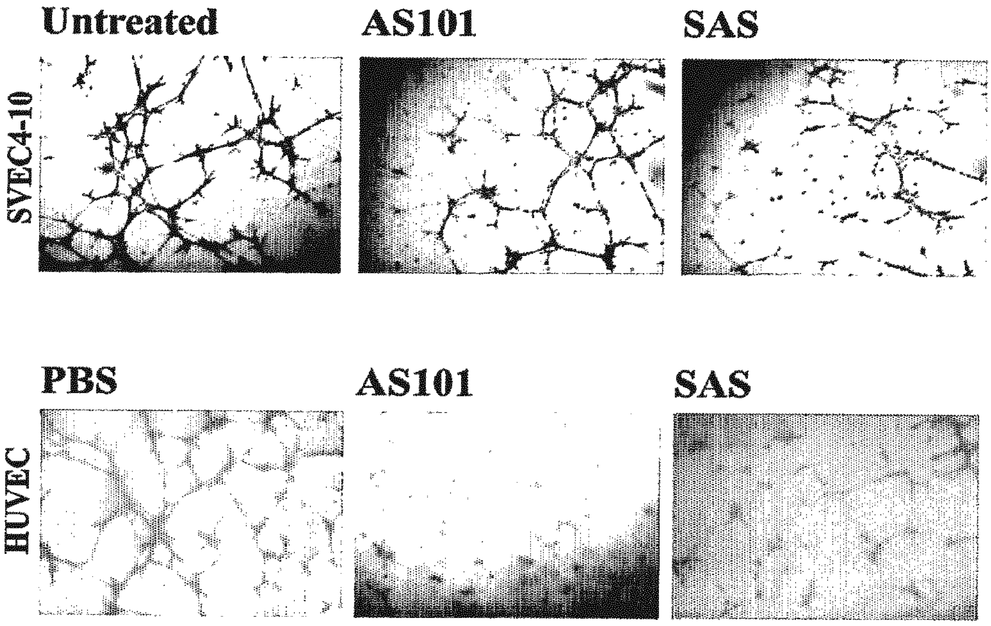


FIG. 4

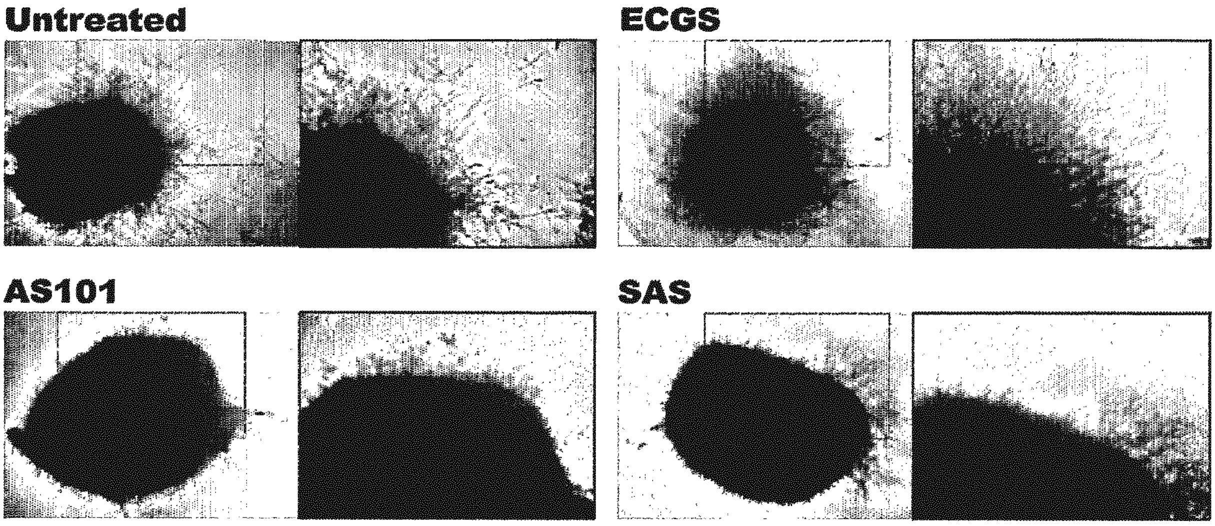
Untreated
ECGS
AS101
SAS

FIG. 5A

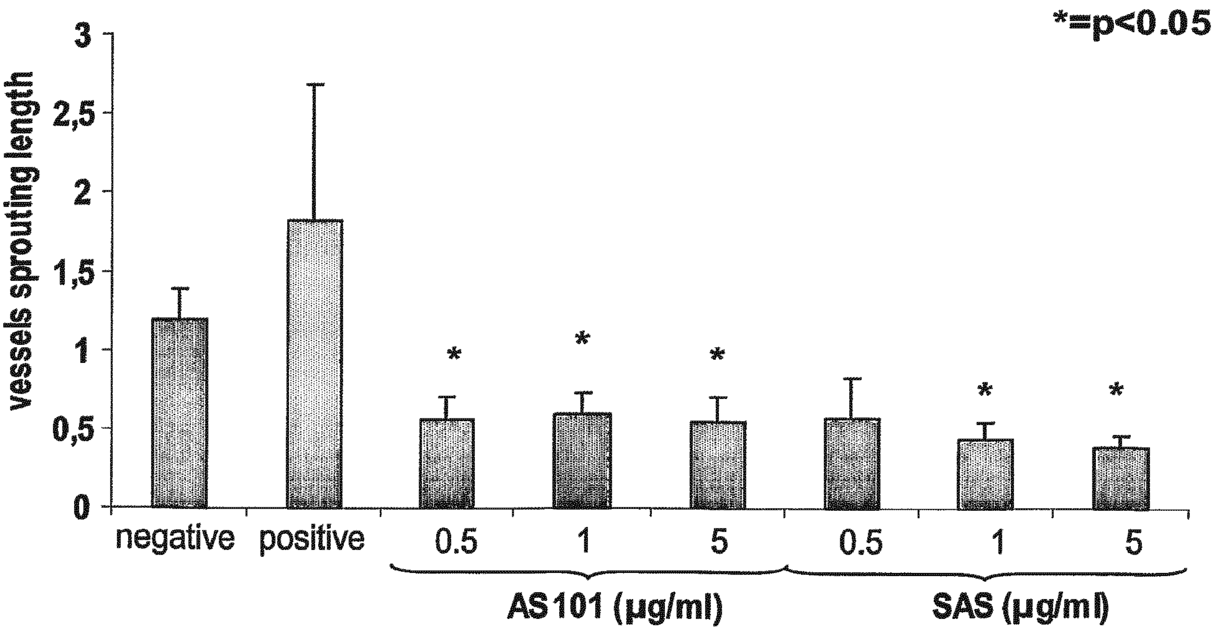
*=p<0.05
vessels sprouting length
3
2,5
2
1,5
1
0,5
0
negative
positive
0.5
1
5
0.5
1
5
AS101 (µg/ml)
SAS (µg/ml)

FIG. 5B

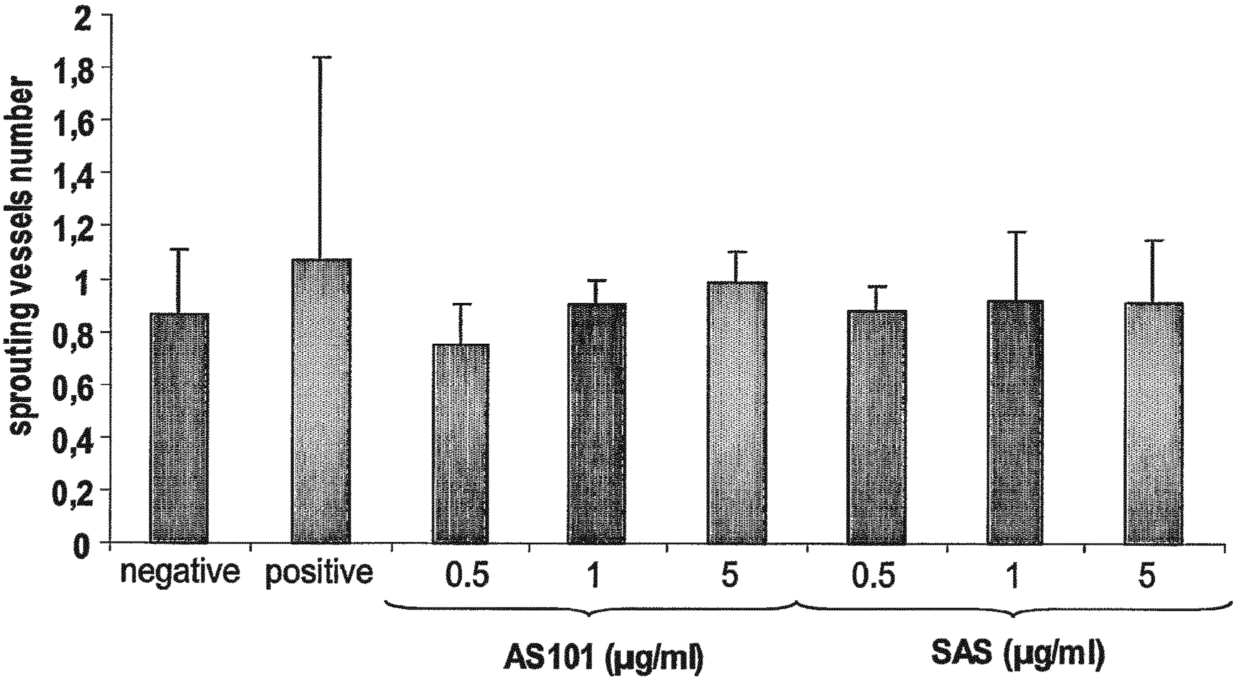

2
1,8
1,6
1,4
1,2
1
0,8
0,6
0,4
0,2
0
sprouting vessels number
negative
positive
0.5
1
5
0.5
1
5
AS101 (µg/ml)
SAS (µg/ml)


FIG. 5C

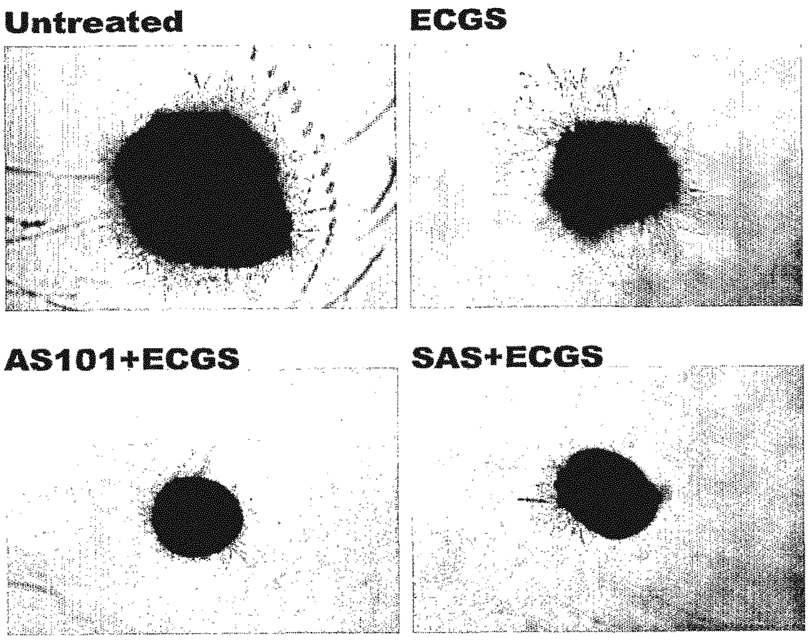
Untreated
ECGS
AS101+ECGS
SAS+ECGS

FIG. 6A

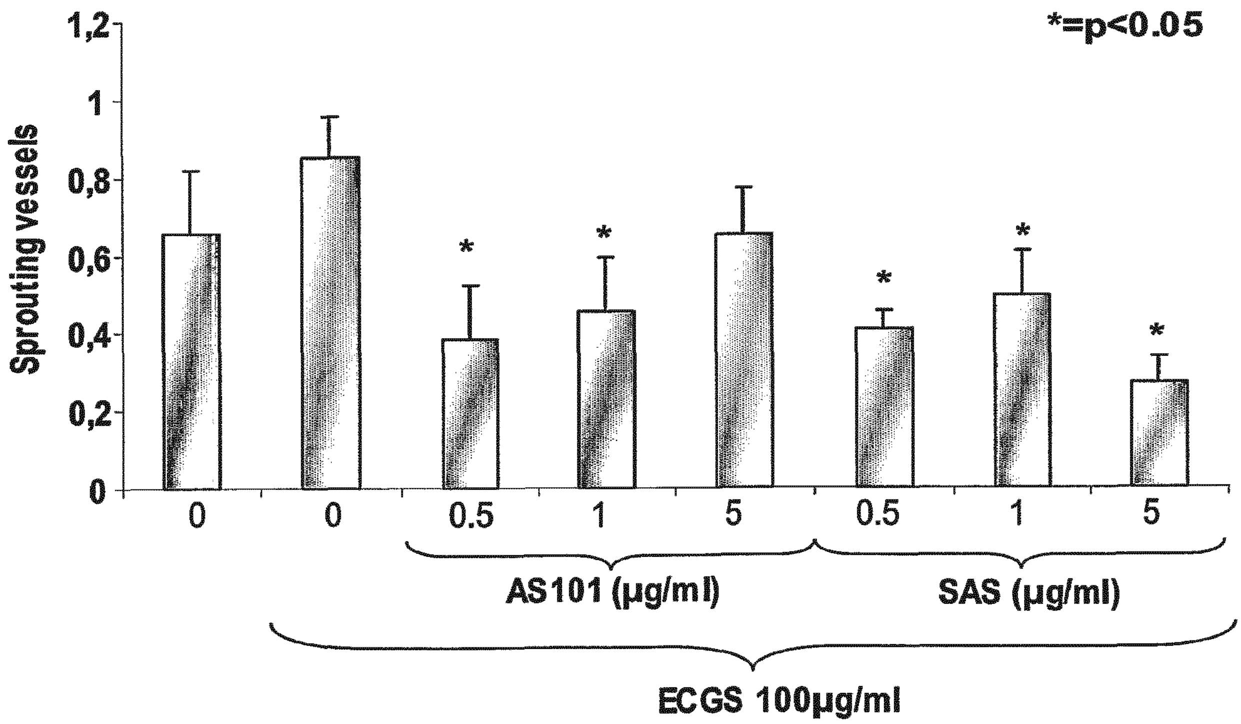
*=p<0.05
Sprouting vessels
1,2
1
0,8
0,6
0,4
0,2
0
0
0
0.5
1
5
0.5
1
5
AS101 (µg/ml)
SAS (µg/ml)
ECGS 100µg/ml

FIG. 6B

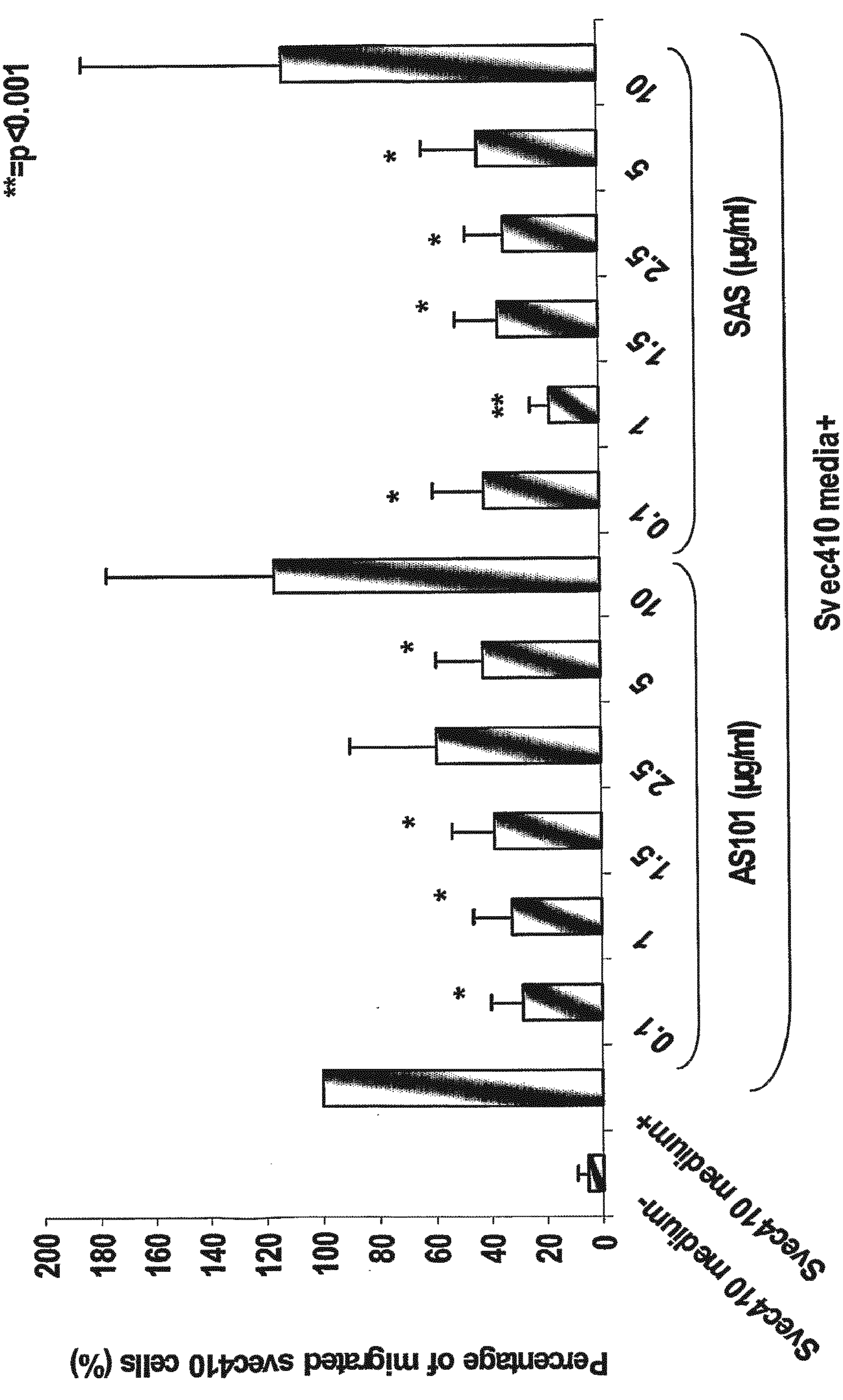
* =p<0.05
**=p<0.001
Percentage of migrated svec410 cells (%)
200
180
160
140
120
100
80
60
40
20
0
Svec410 medium-
Svec410 medium+
0.1
1
1.5
2.5
5
10
AS101 (µg/ml)
SAS (µg/ml)
Svec410 media+

FIG. 7

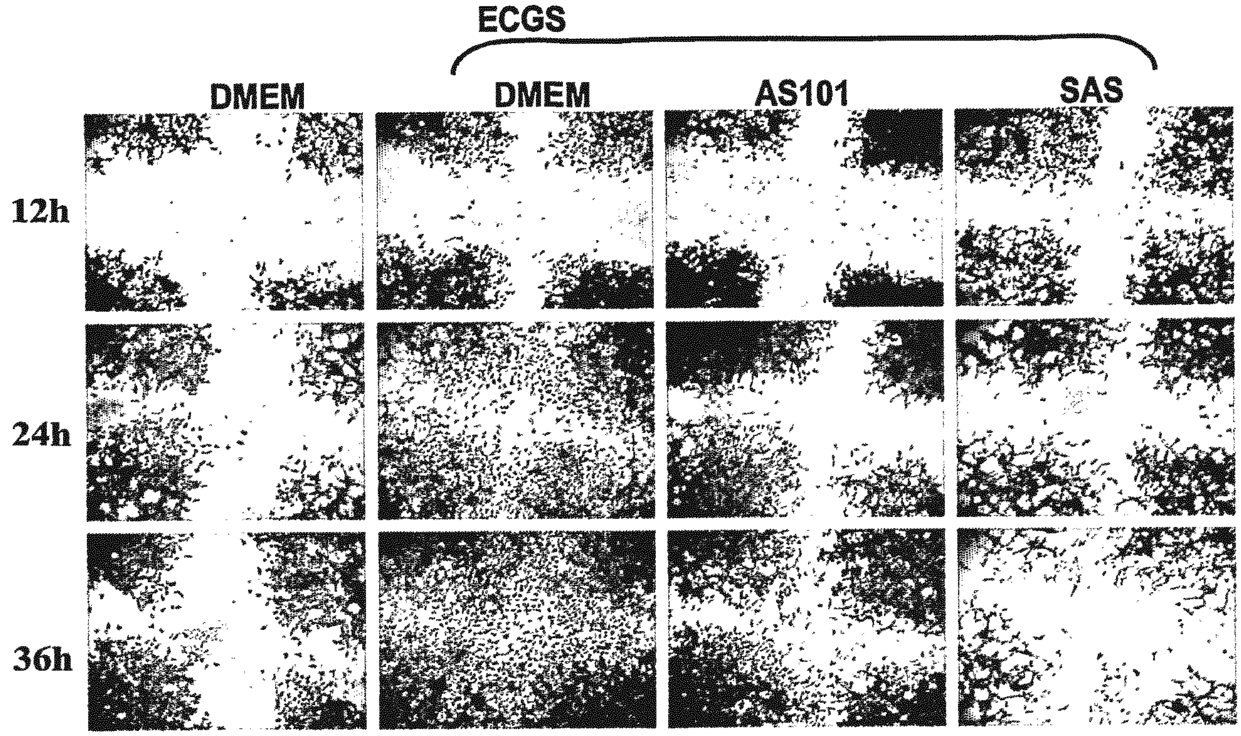
ECGS
DMEM
DMEM
AS101
SAS
12h
24h
36h

FIG.8A

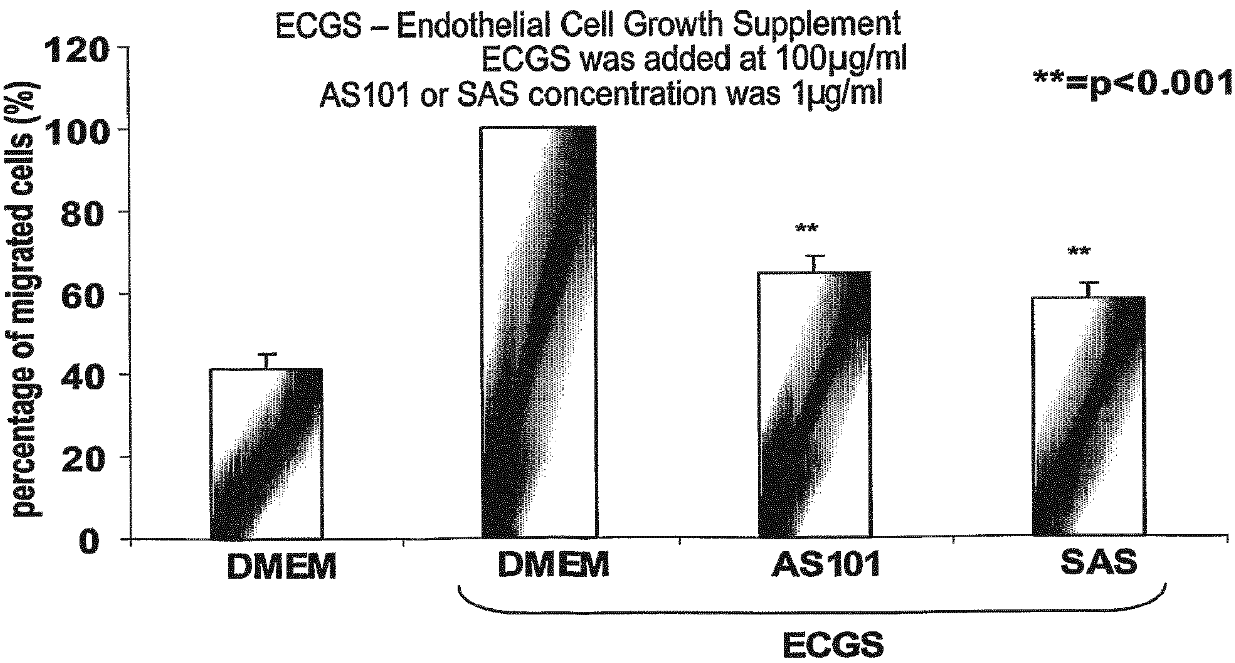
ECGS – Endothelial Cell Growth Supplement
ECGS was added at 100µg/ml
AS101 or SAS concentration was 1µg/ml
**=p<0.001
percentage of migrated cells (%)
120
100
80
60
40
20
0
**
**
DMEM
DMEM
AS101
SAS
ECGS

FIG.8B

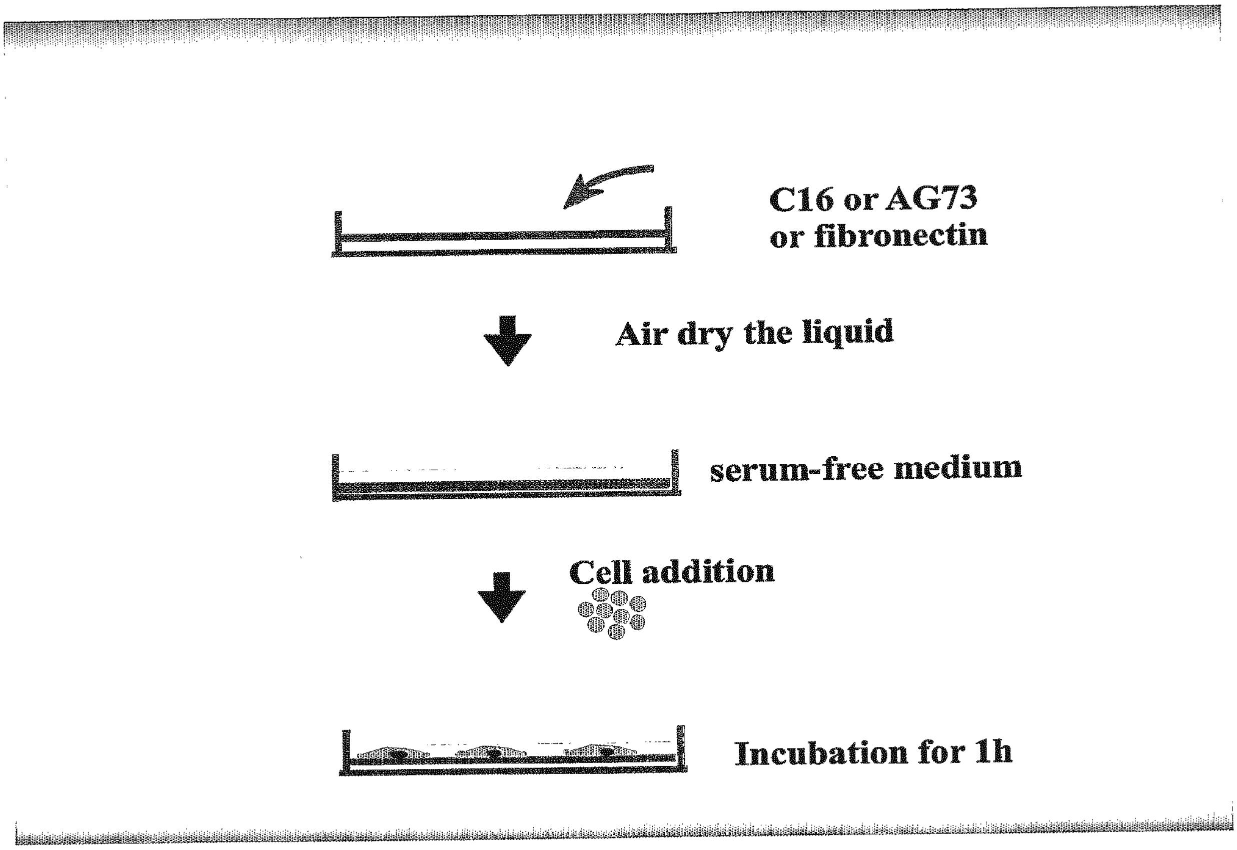
C16 or AG73
or fibronectin
Air dry the liquid
serum-free medium
Cell addition
Incubation for 1h

FIG. 9

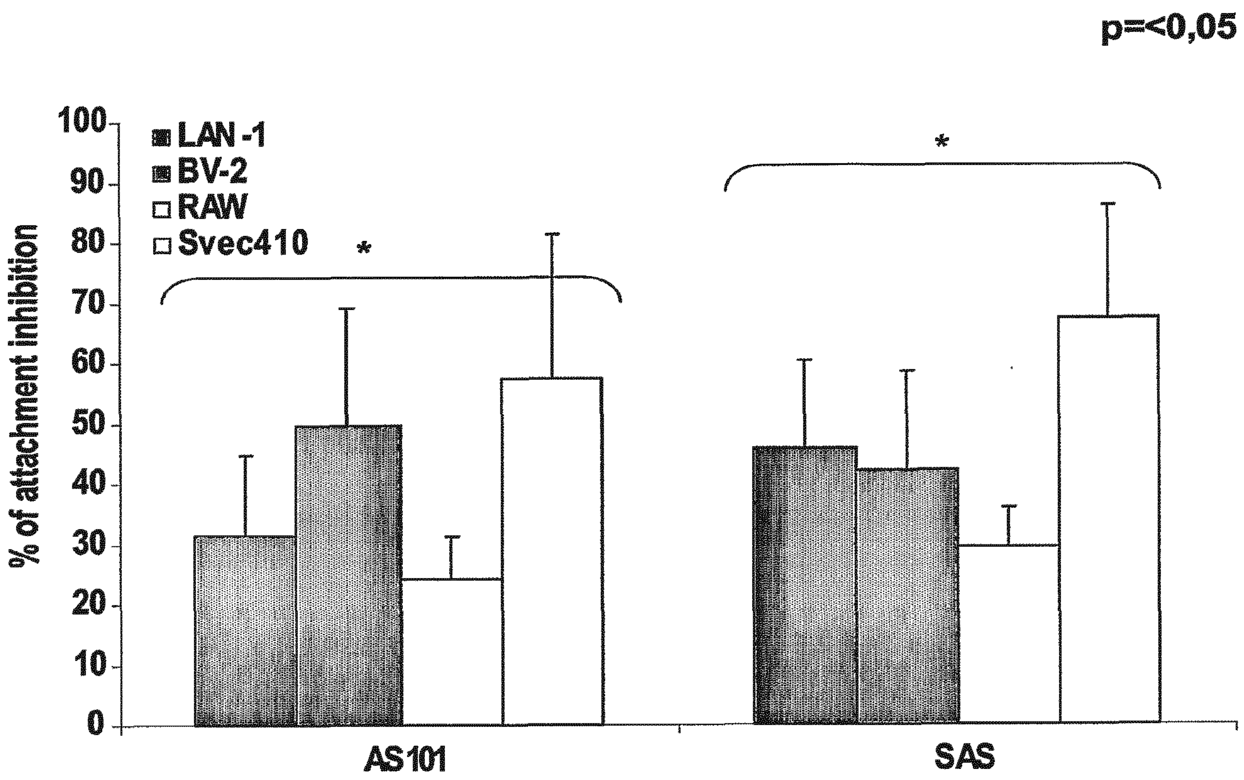
p=<0,05
100
90
80
70
60
50
40
30
20
10
0
% of attachment inhibition
LAN -1
BV-2
RAW
Svec410
*
*
AS101
SAS

FIG. 10

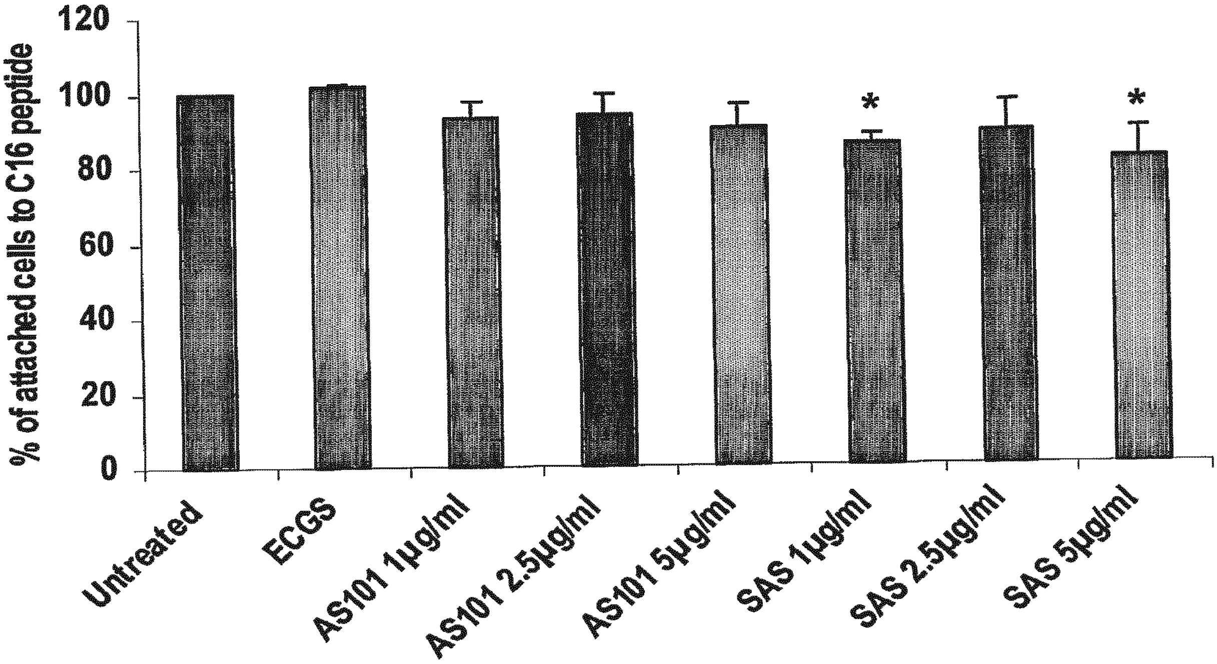


FIG. 11A

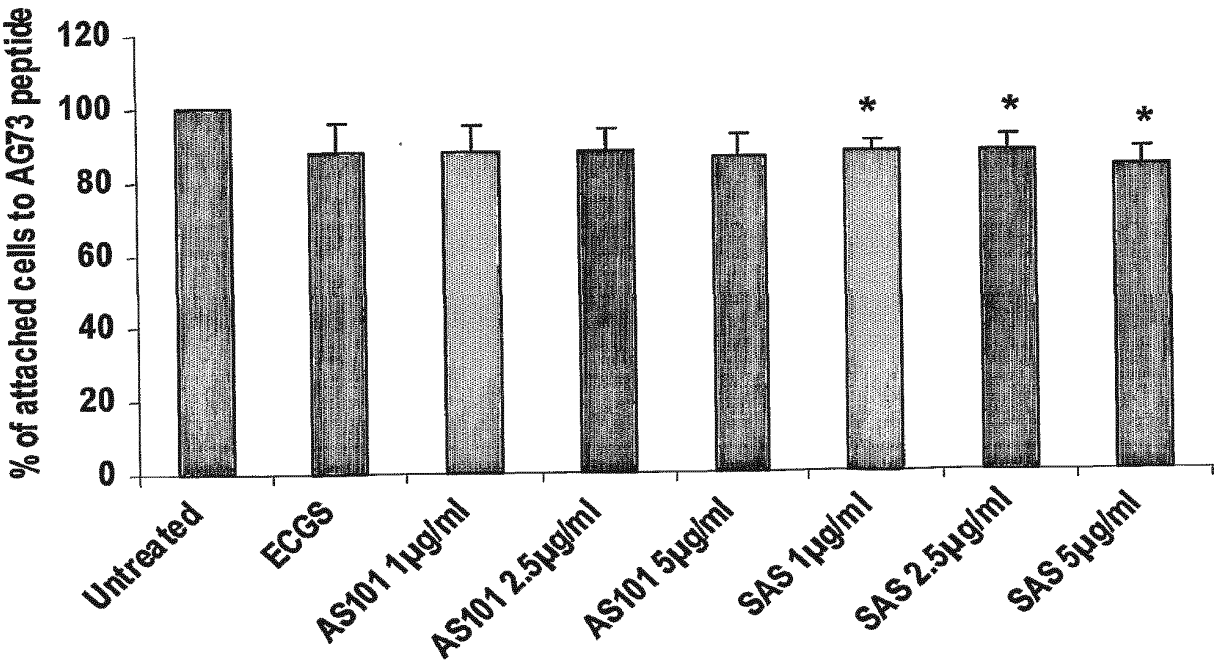


FIG.11B

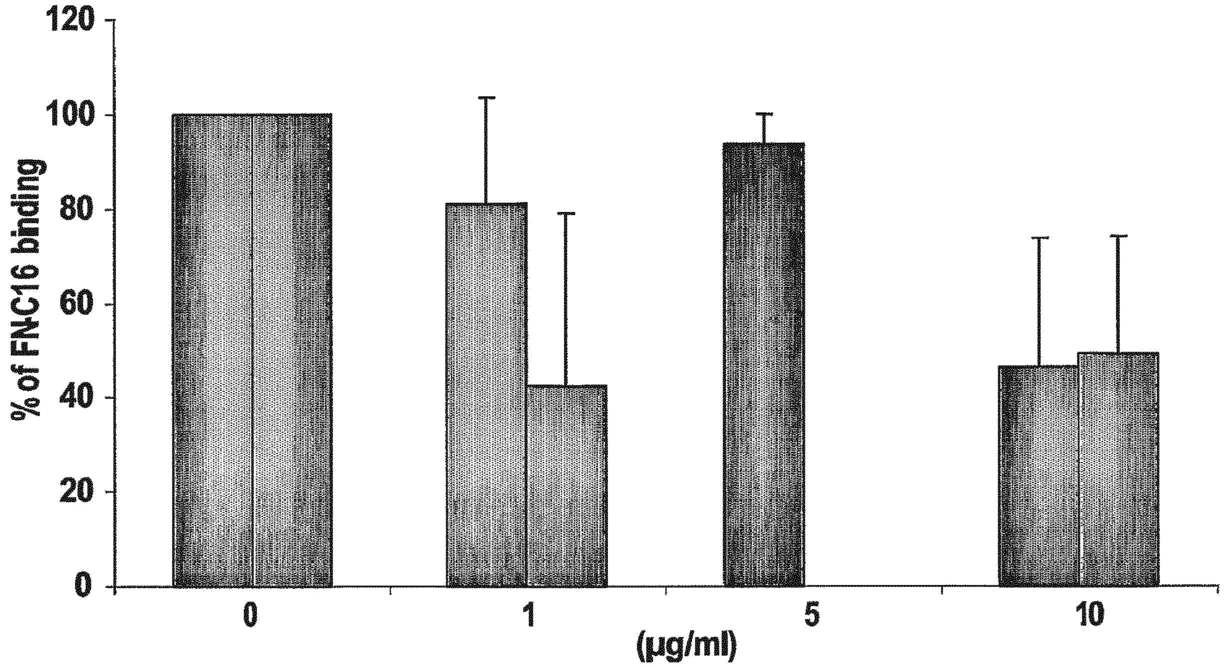


FIG.12A

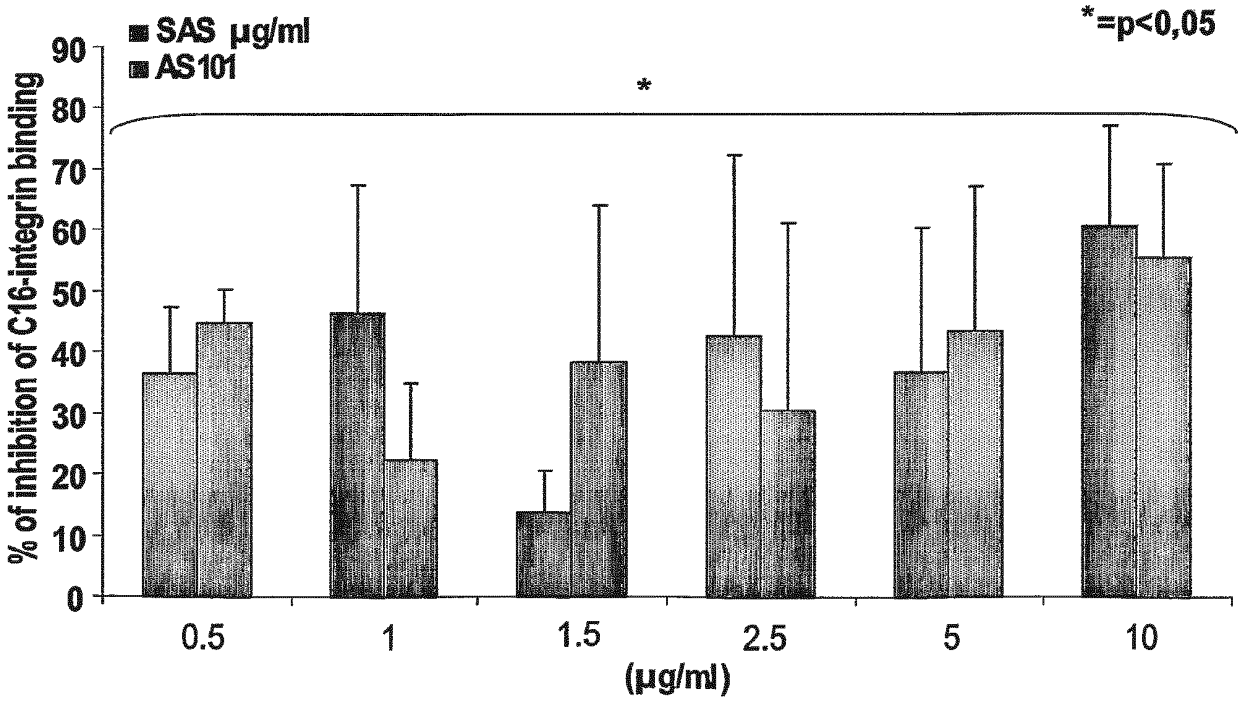


FIG. 12B

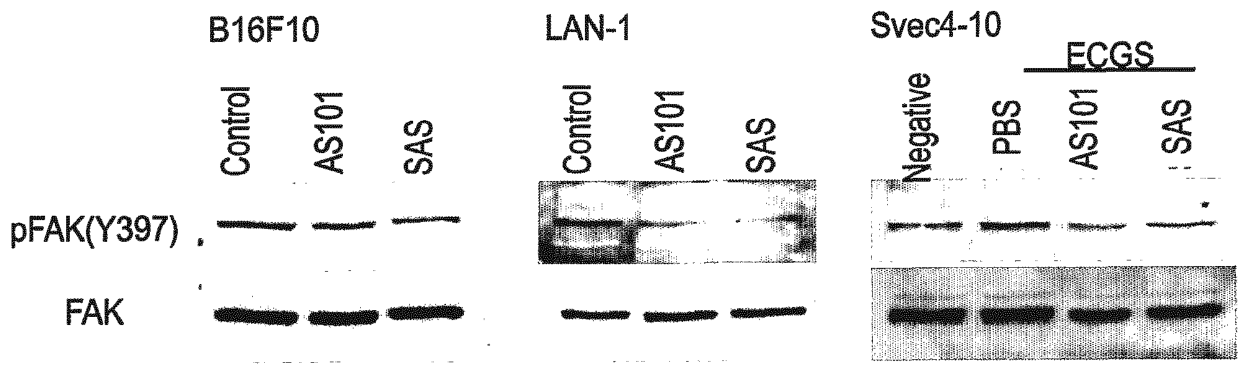


FIG. 13A

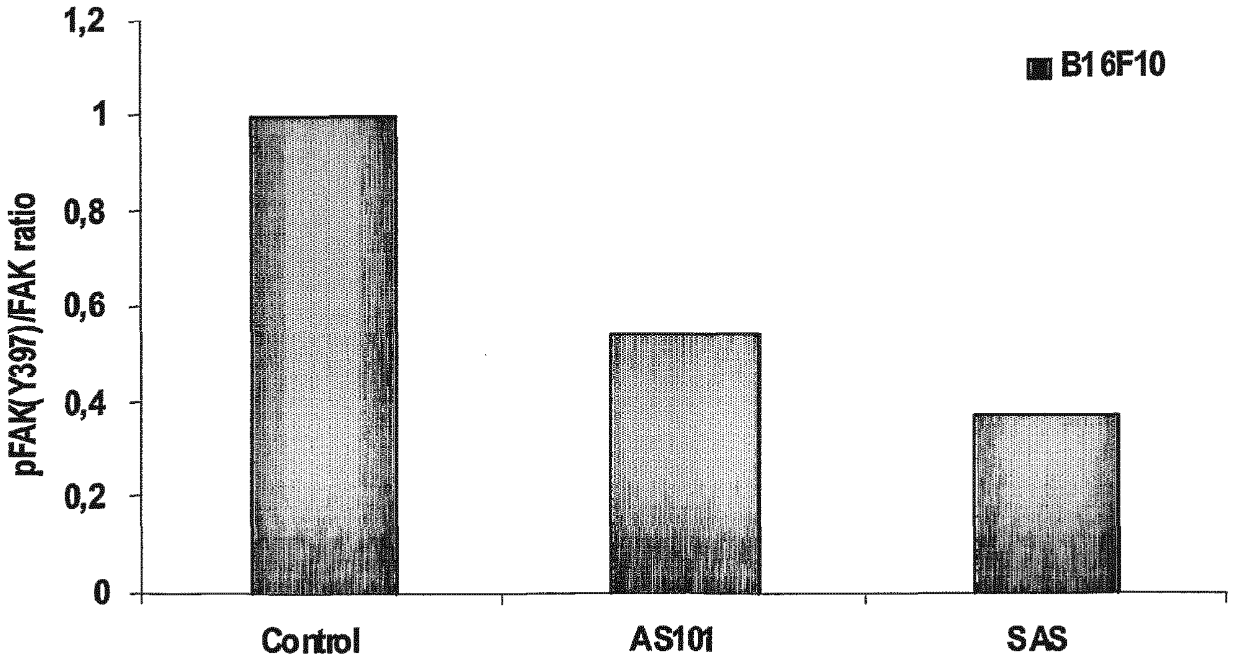


FIG. 13B

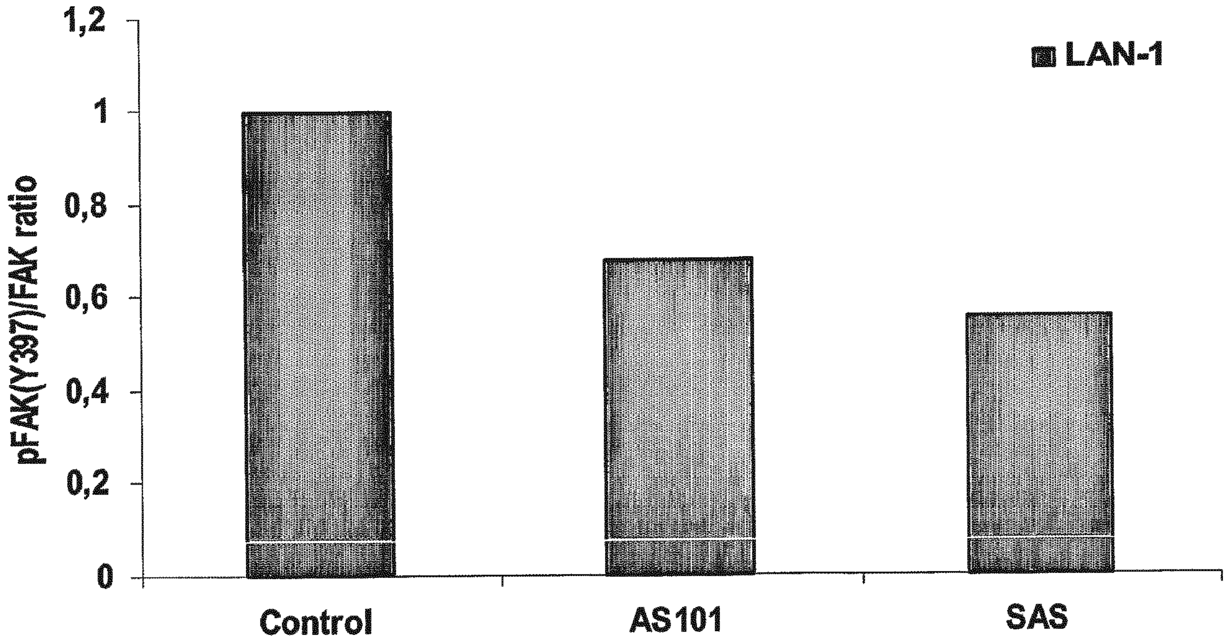


FIG. 13C

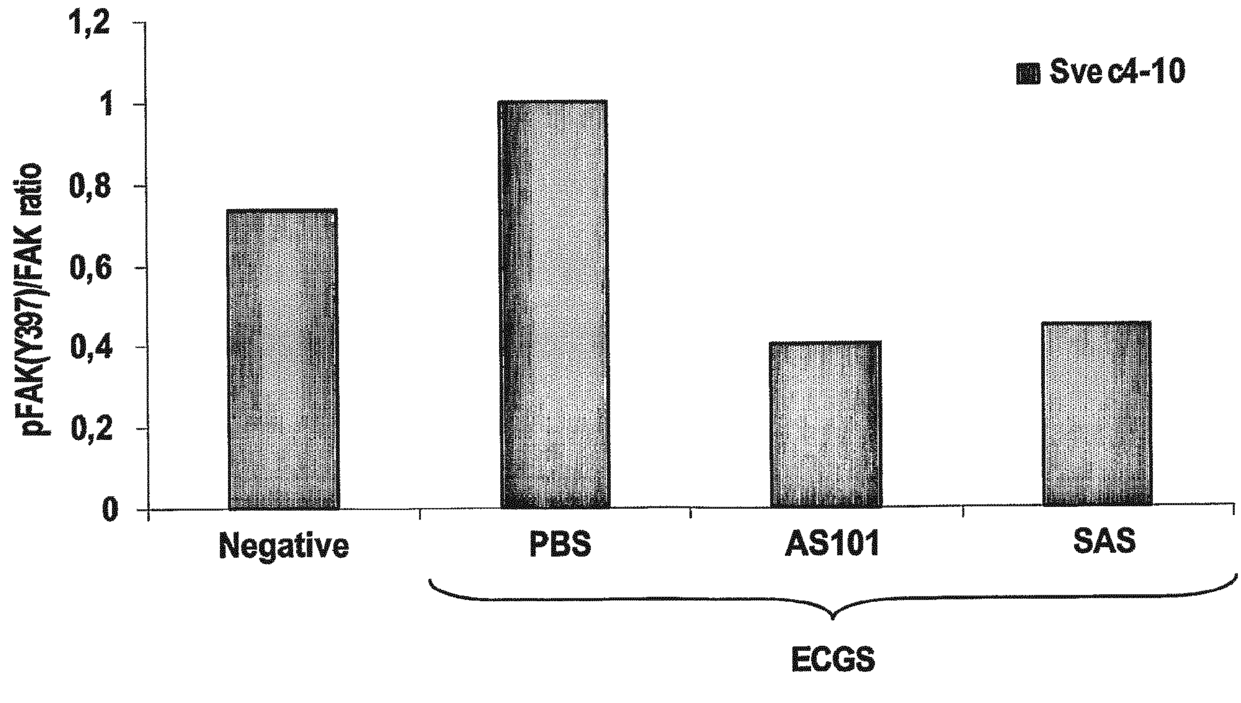


FIG. 13D

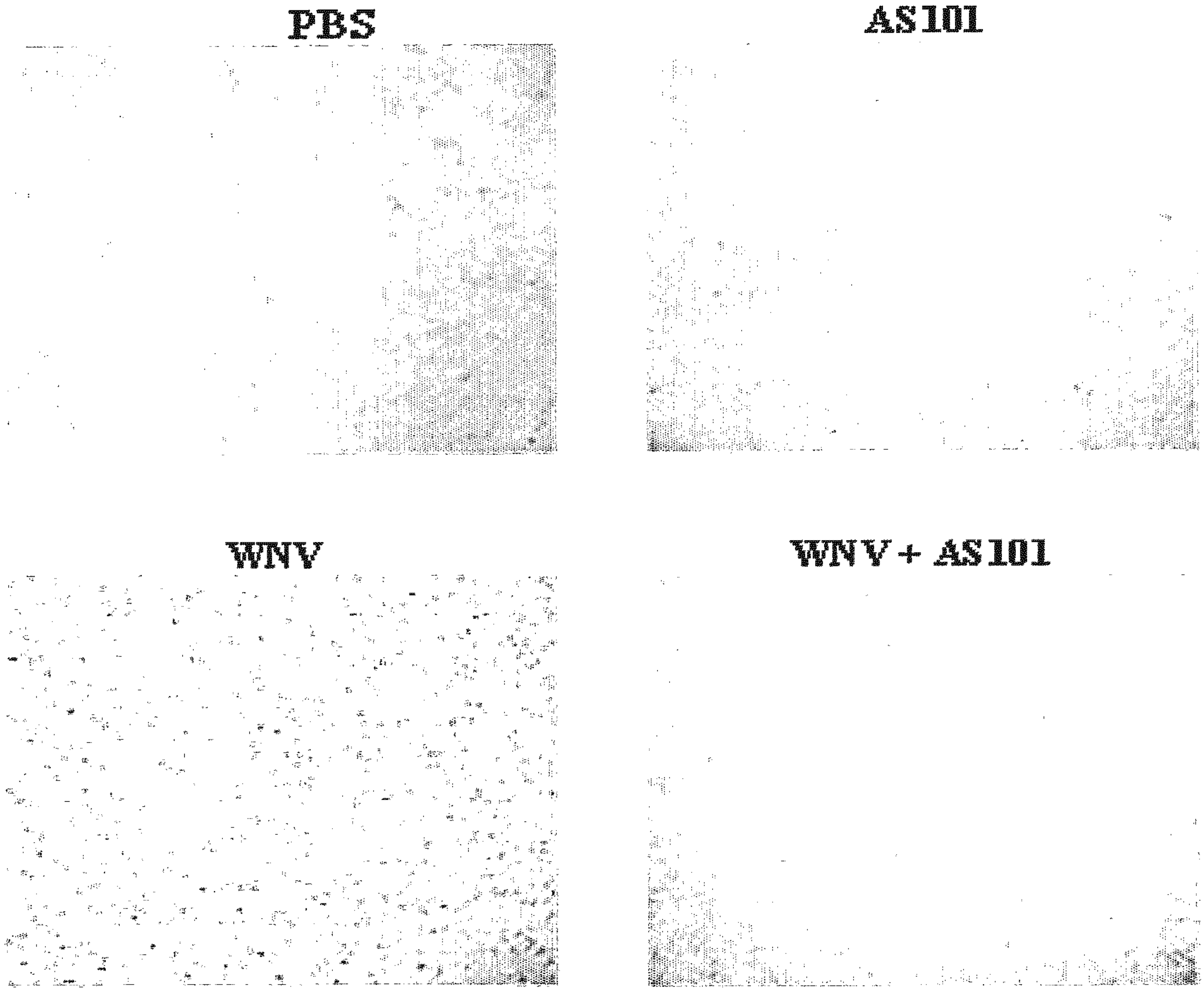


FIG. 14

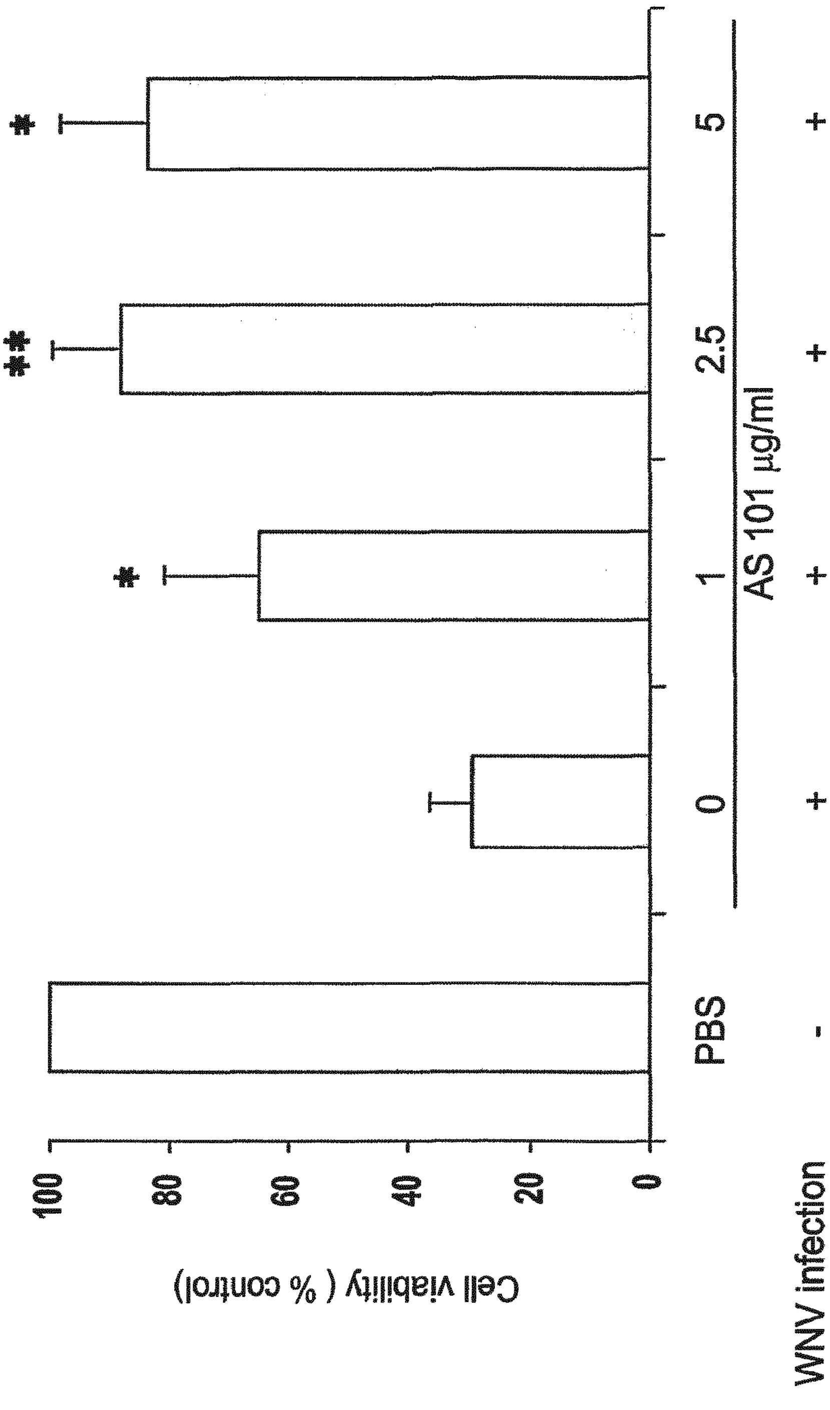
Cell viability ( % control)
100
80
60
40
20
0
PBS
0
1
2.5
5
AS 101 μg/ml
WNV infection
-
+
+
+
+
*
**
*

FIG. 15

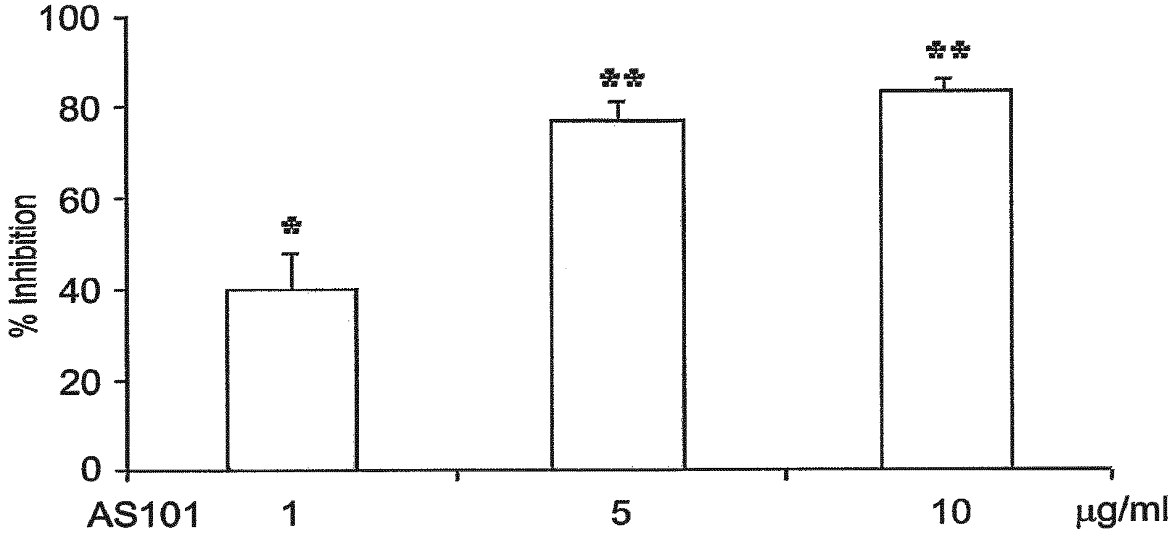


FIG. 16A

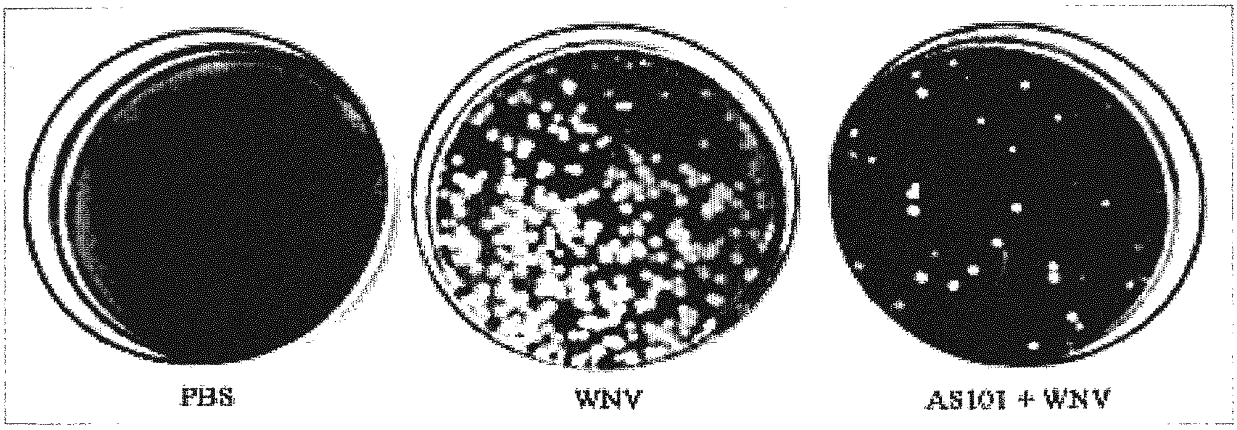


FIG.16B

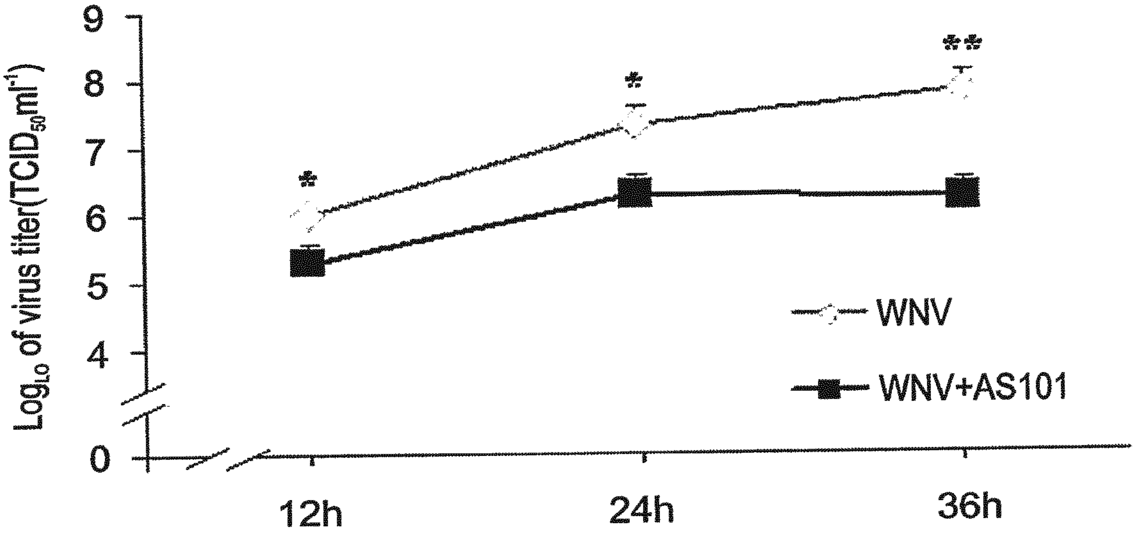
9
8
7
6
5
4
0
Log10 of virus titer(TCID50ml-1)
*
*
**
WNV
WNV+AS101
12h
24h
36h

FIG. 17

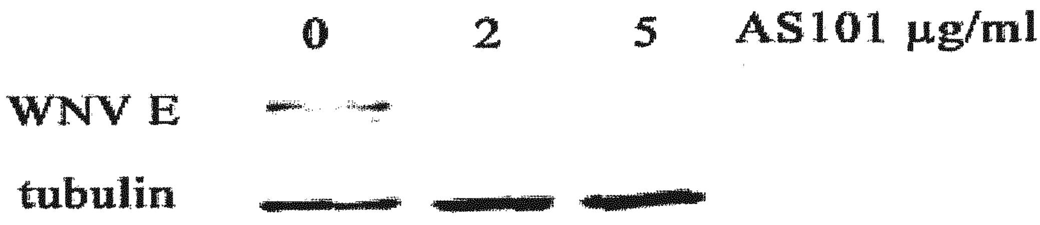


FIG.18A

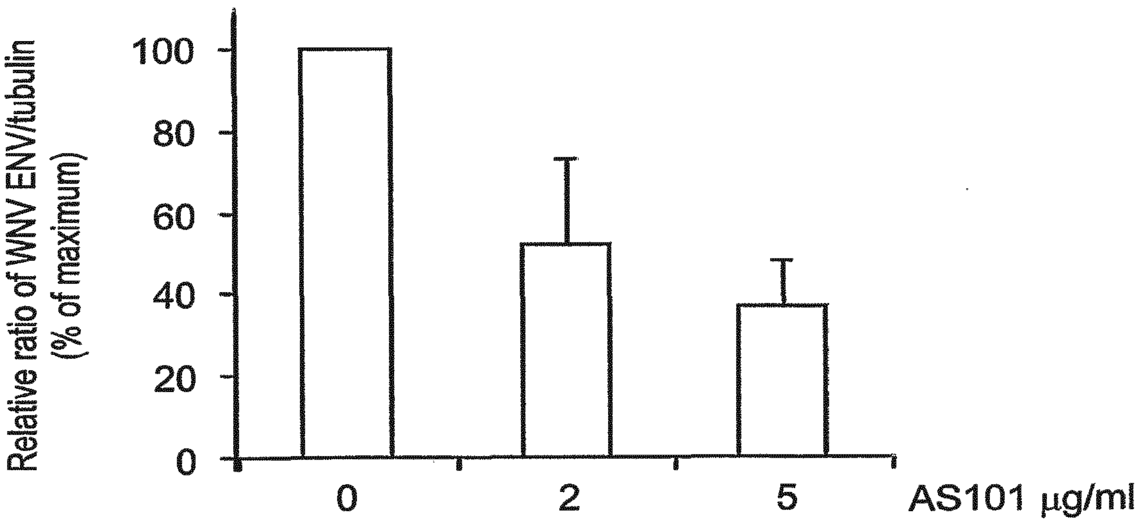


FIG.18B

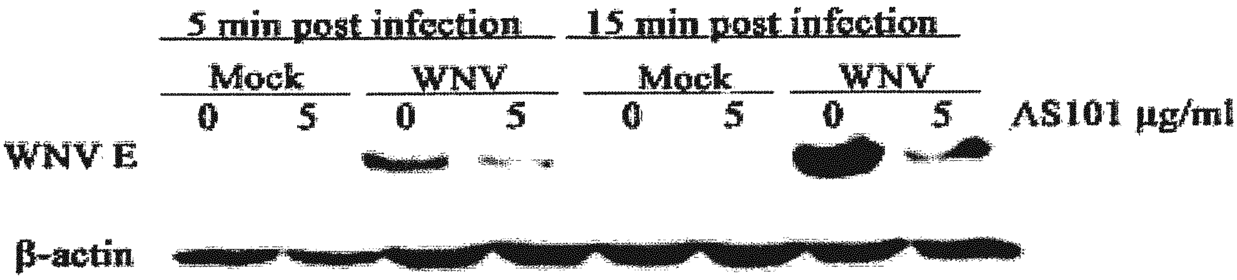
5 min post infection
15 min post infection
Mock
WNV
Mock
WNV
0
5
0
5
0
5
0
5
AS101 µg/ml
WNV E
β-actin

FIG. 19

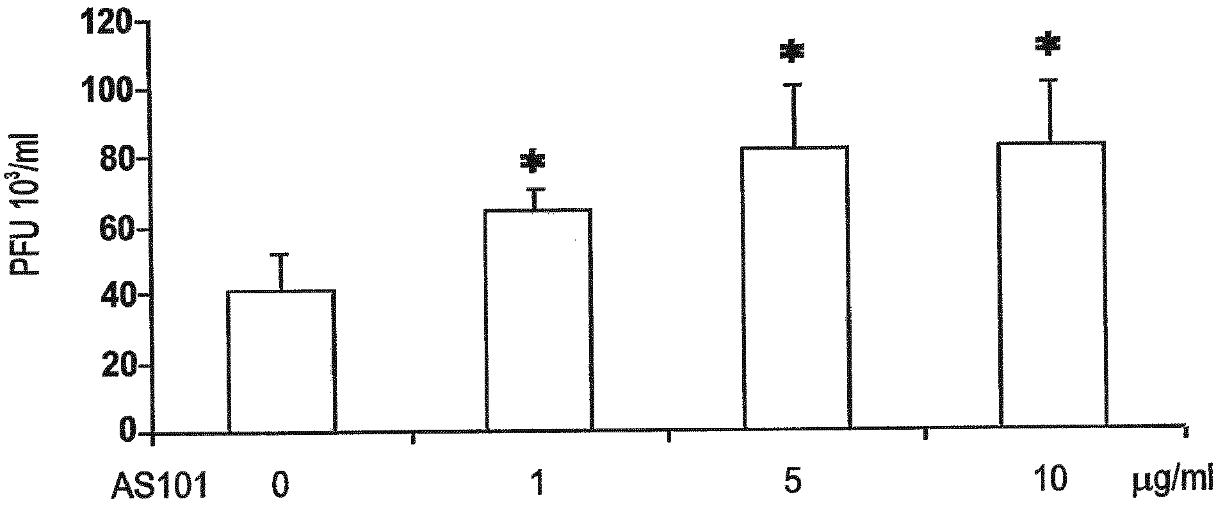


FIG.20

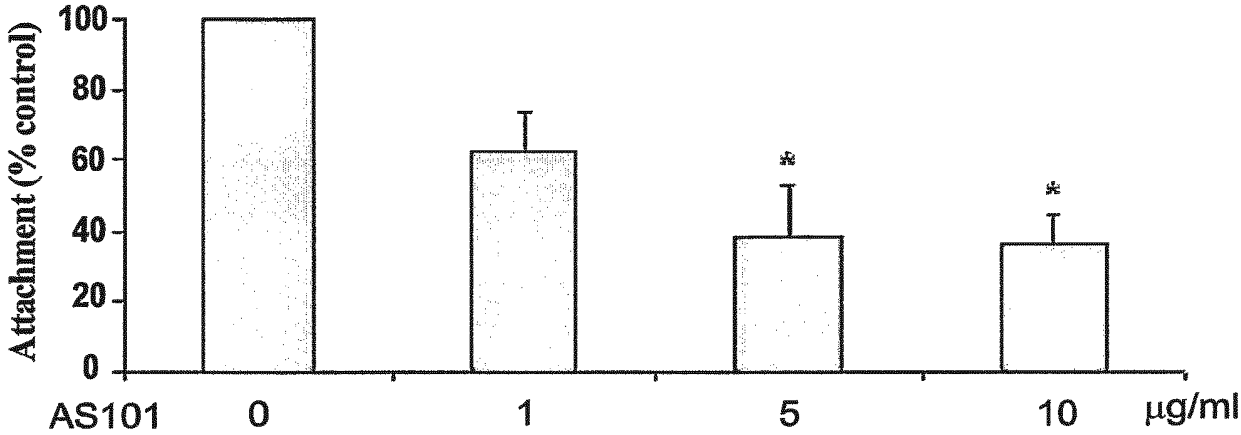


FIG. 21

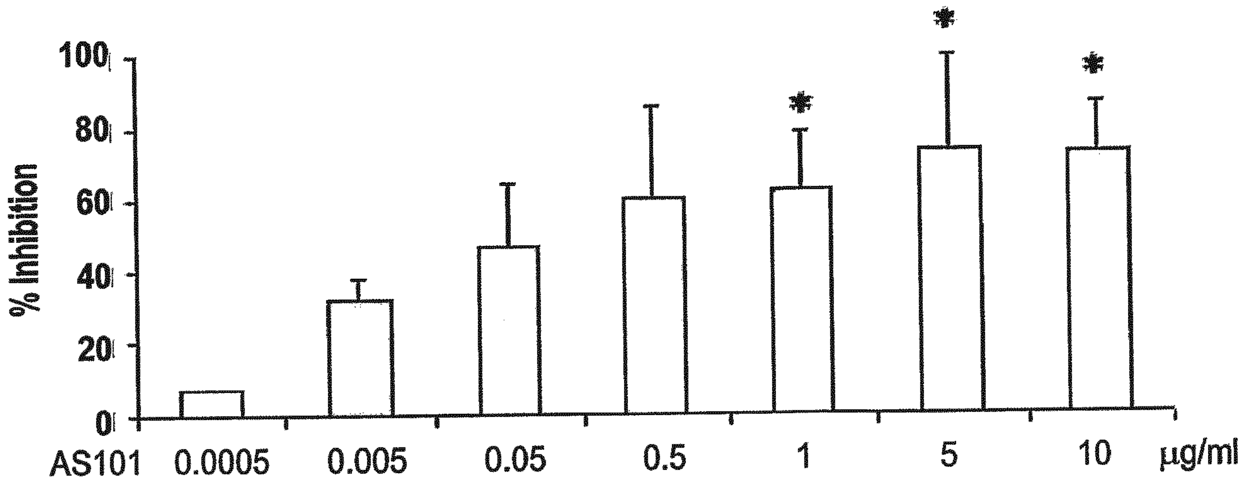


FIG. 22

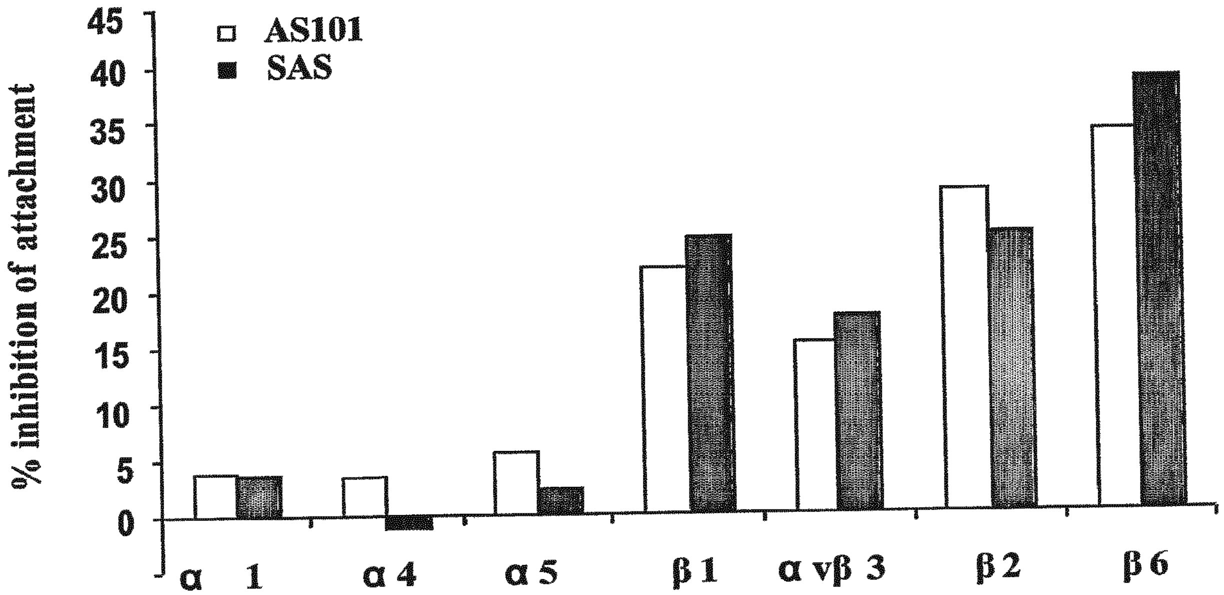
% inhibition of attachment
45
40
35
30
25
20
15
10
5
0
AS101
SAS
α 1
α 4
α 5
β 1
α vβ 3
β 2
β 6

FIG. 23

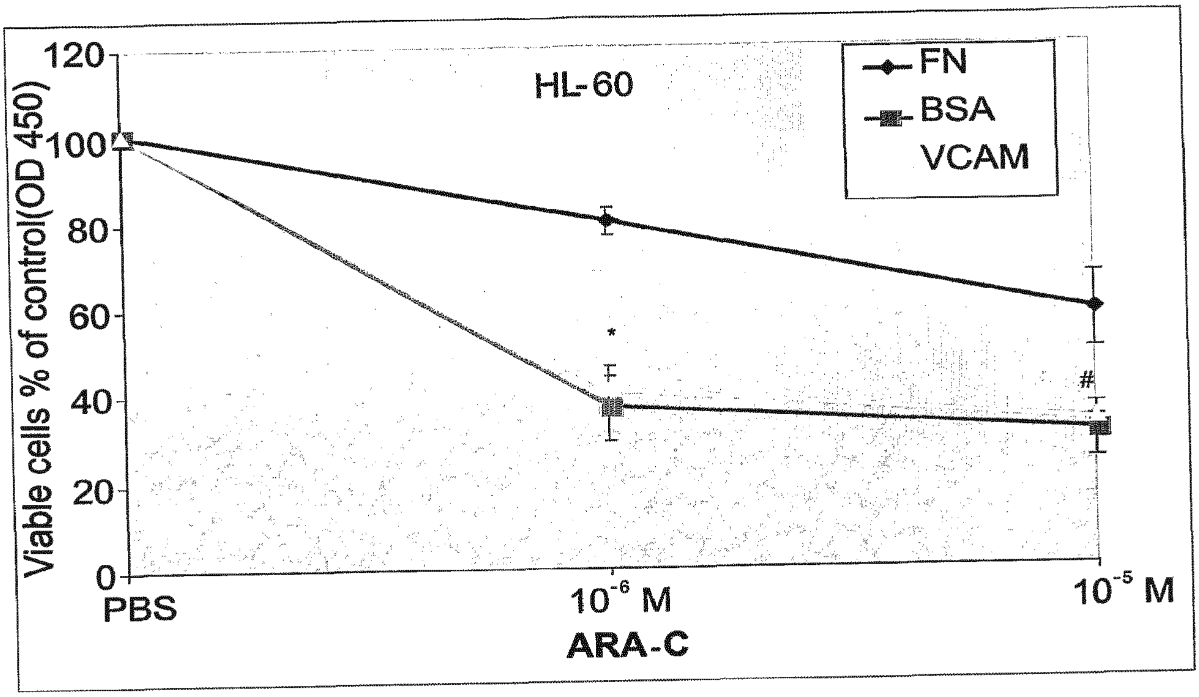


FIG. 24A

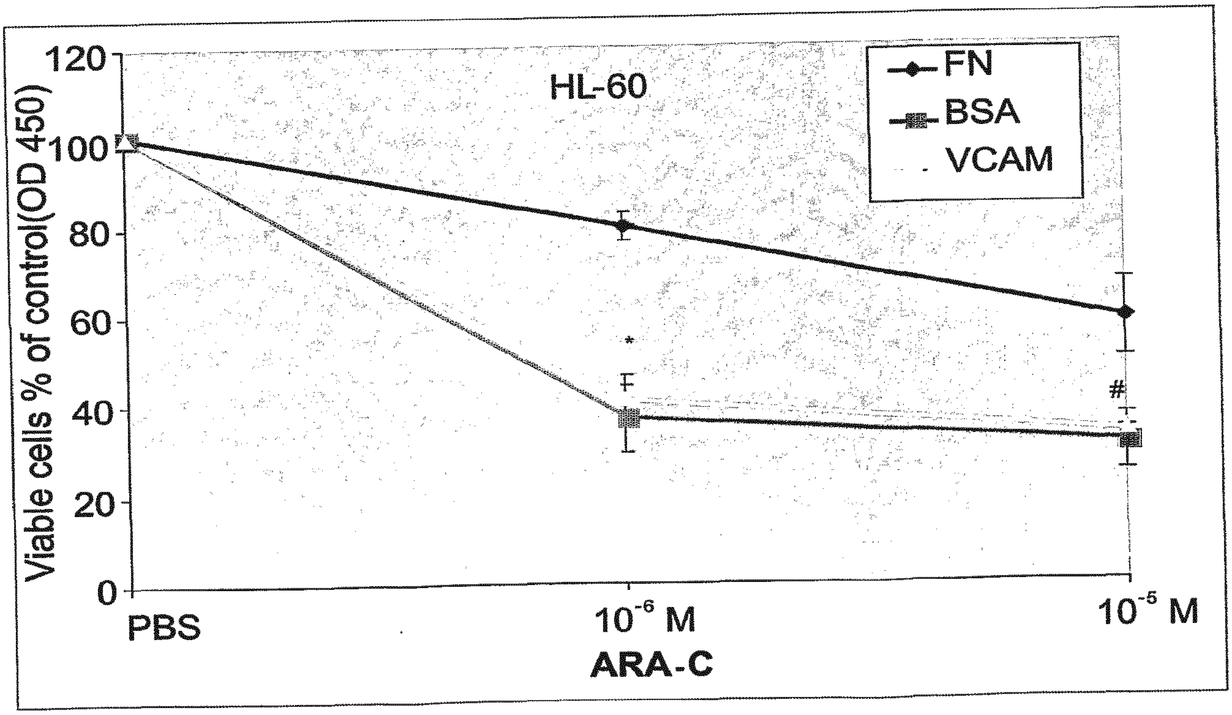


FIG. 24B

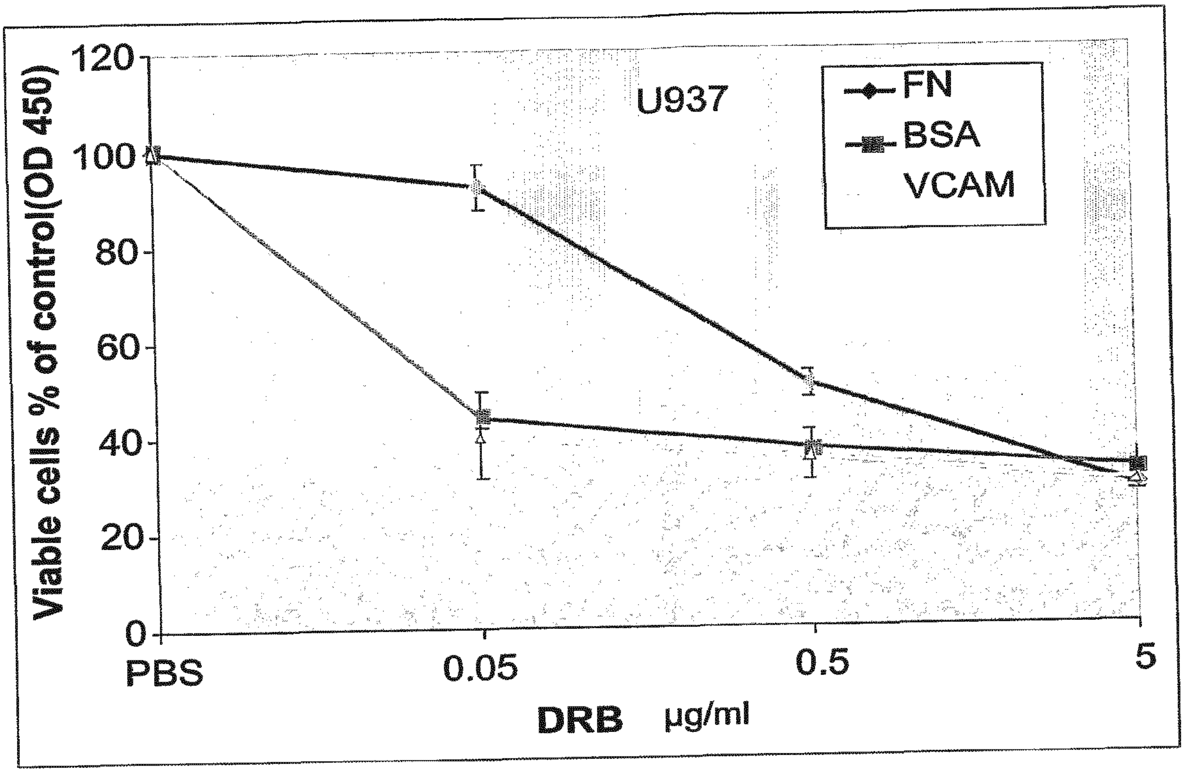


FIG. 24C

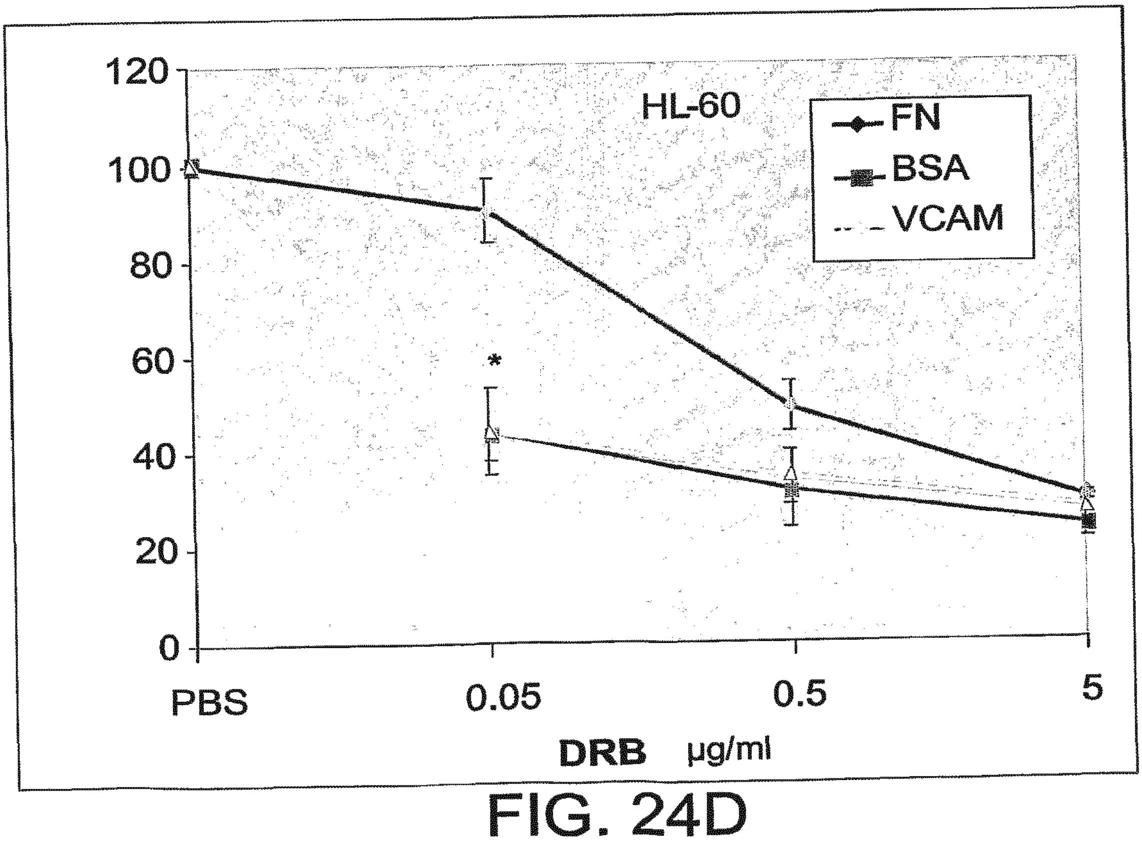


FIG. 24D

FIG. 25A

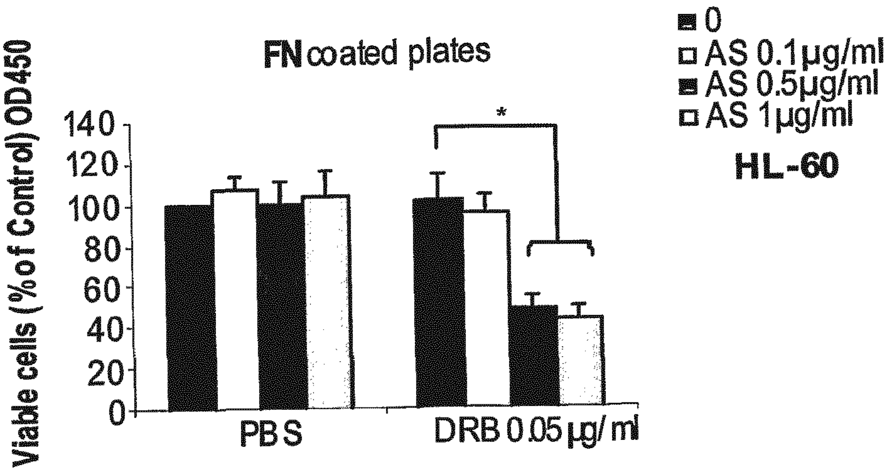


FIG. 25B

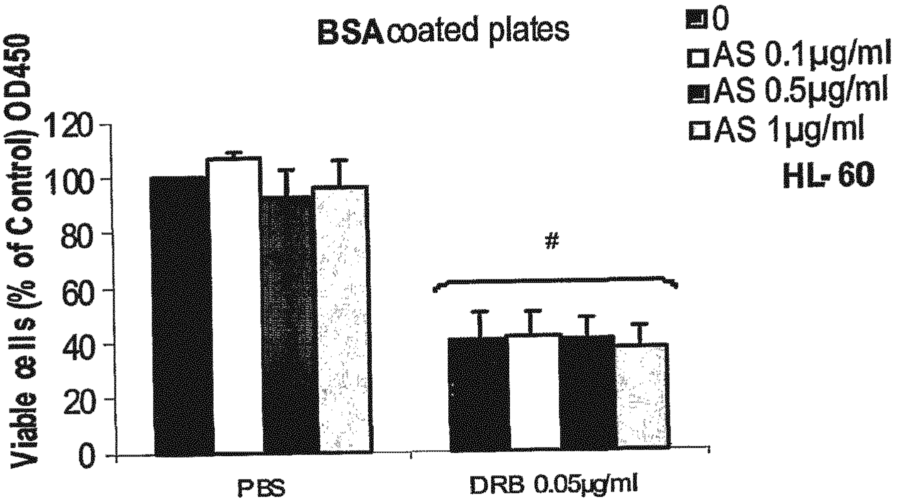


FIG. 25C

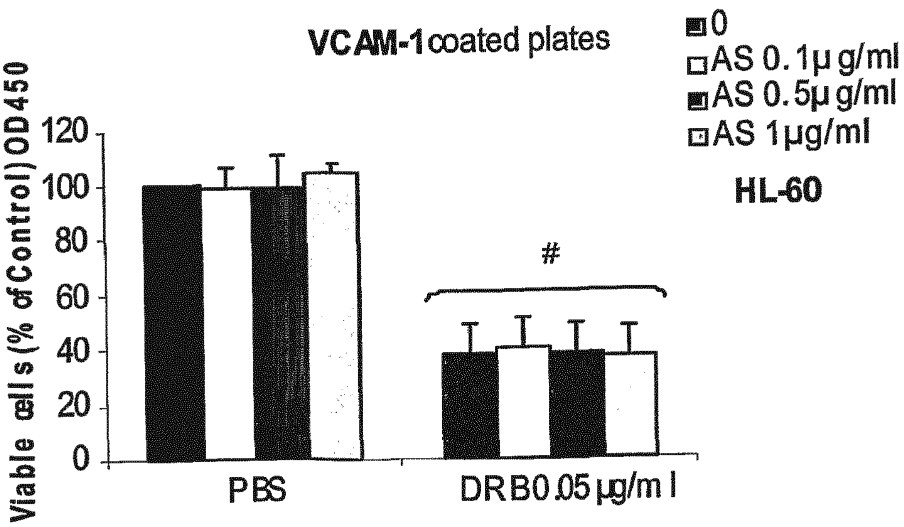

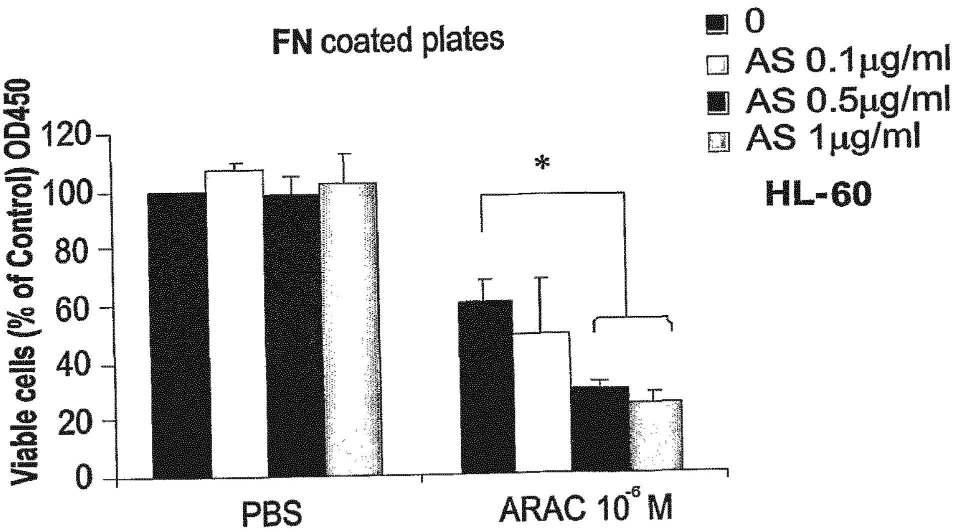


FIG. 26A

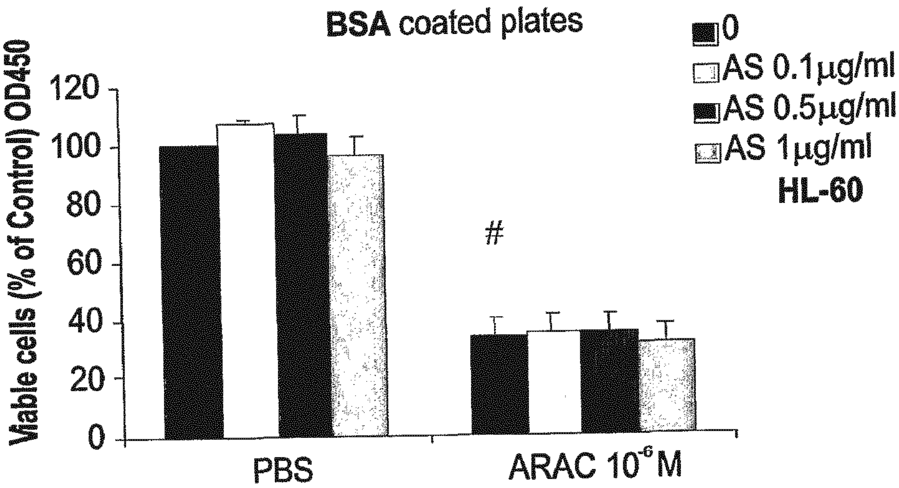


FIG. 26B

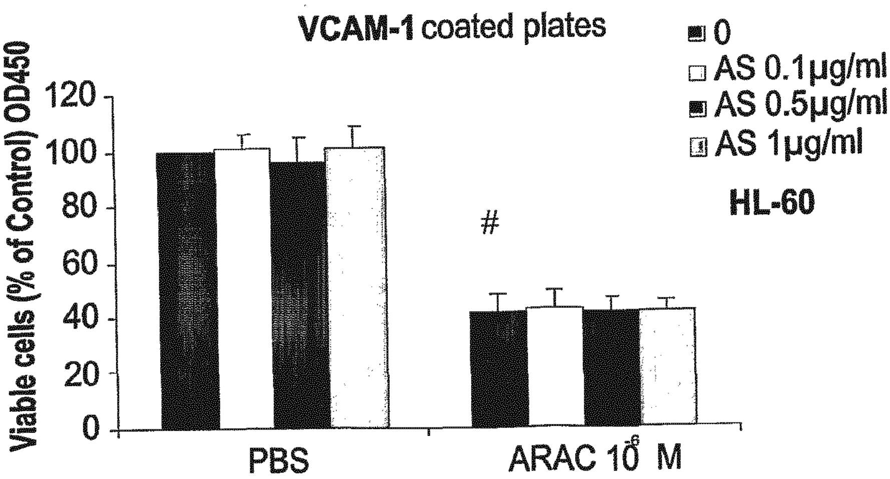


FIG. 26C

FIG. 27A

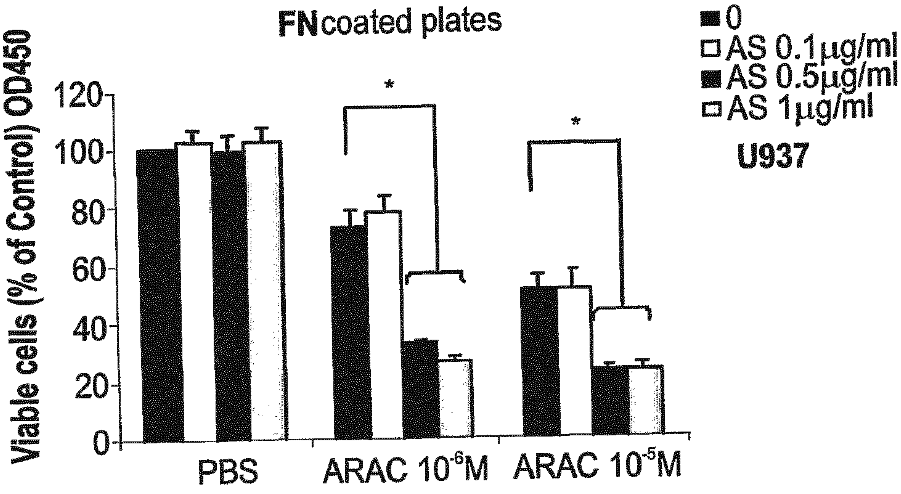


FIG. 27B

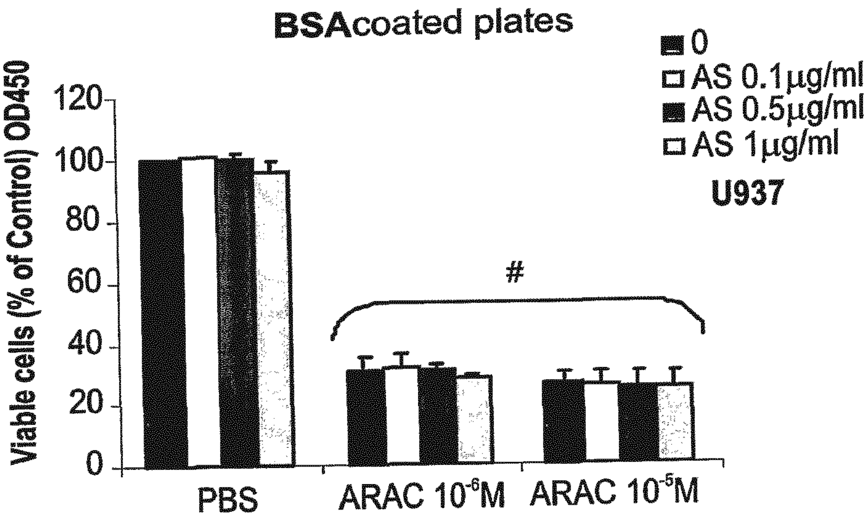


FIG. 27C

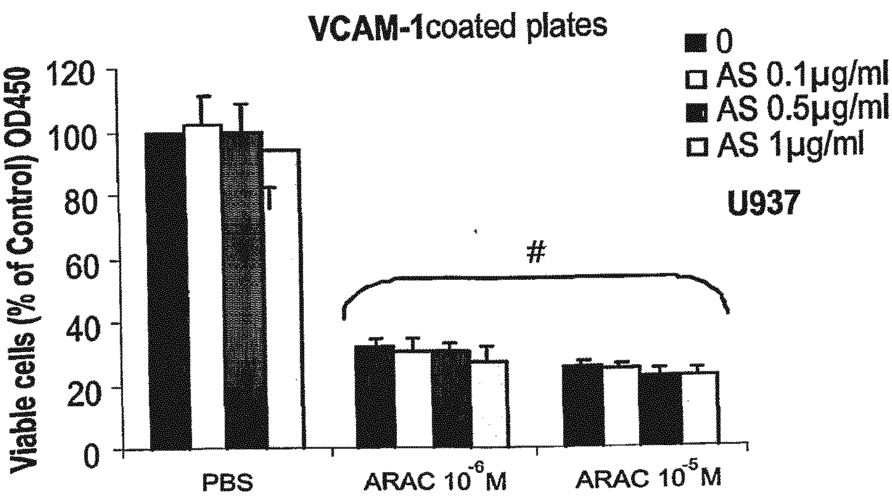

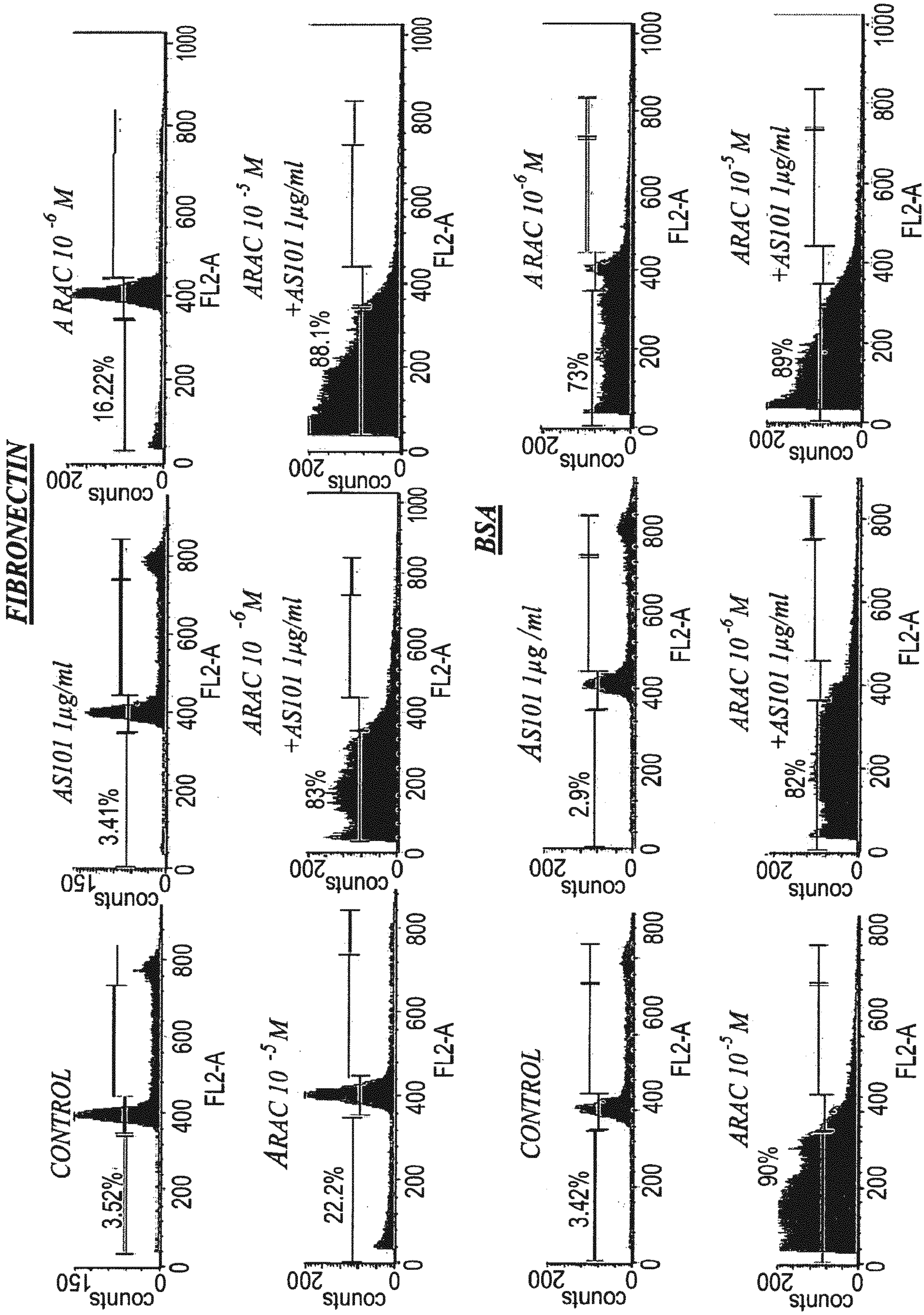
FIBRONECTIN
CONTROL
3.52%
AS101 1μg/ml
3.41%
A RAC 10 -6 M
16.22%
ARAC 10 -5 M
22.2%
ARAC 10 -6 M +AS101 1μg/ml
83%
ARAC 10 -5 M +AS101 1μg/ml
88.1%
BSA
CONTROL
3.42%
AS101 1μg /ml
2.9%
A RAC 10 -6 M
73%
ARAC 10 -5 M
90%
ARAC 10 -6 M +AS101 1μg/ml
82%
ARAC 10 -5 M +AS101 1μg/ml
89%
counts
FL2-A

FIG. 28

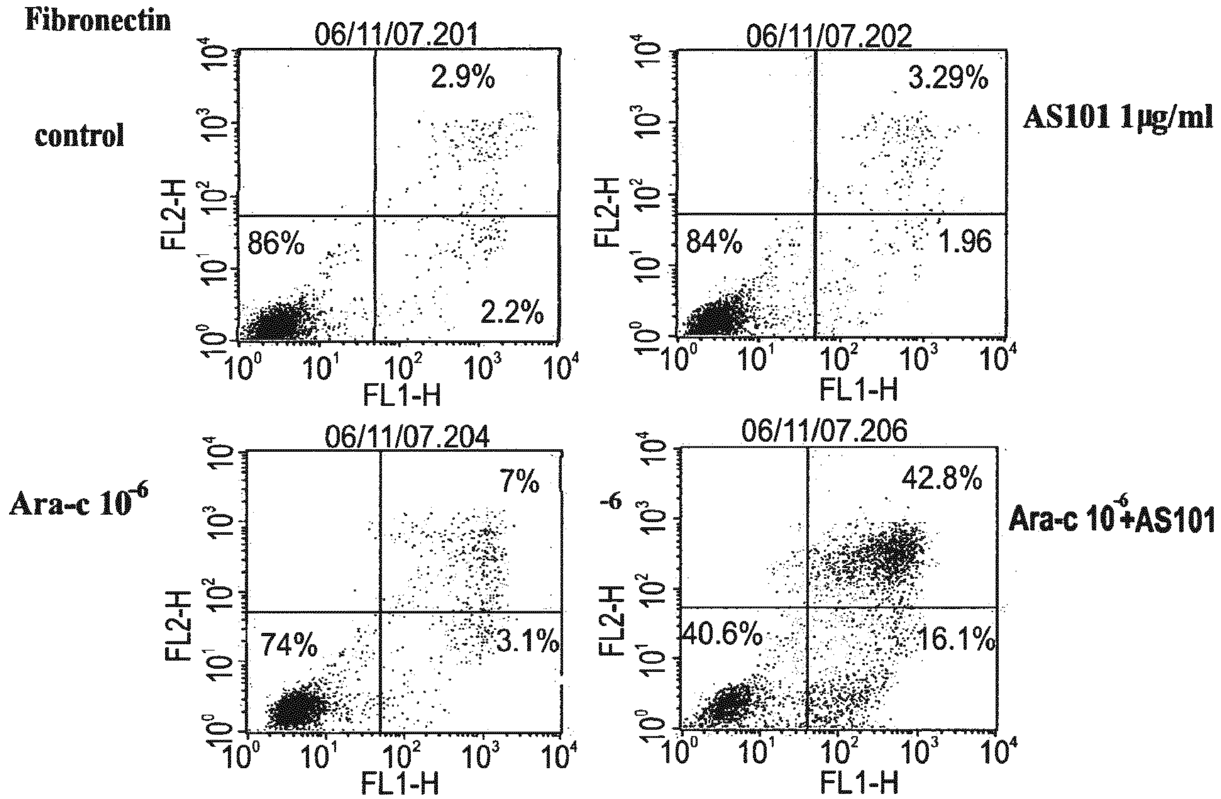


FIG. 29A

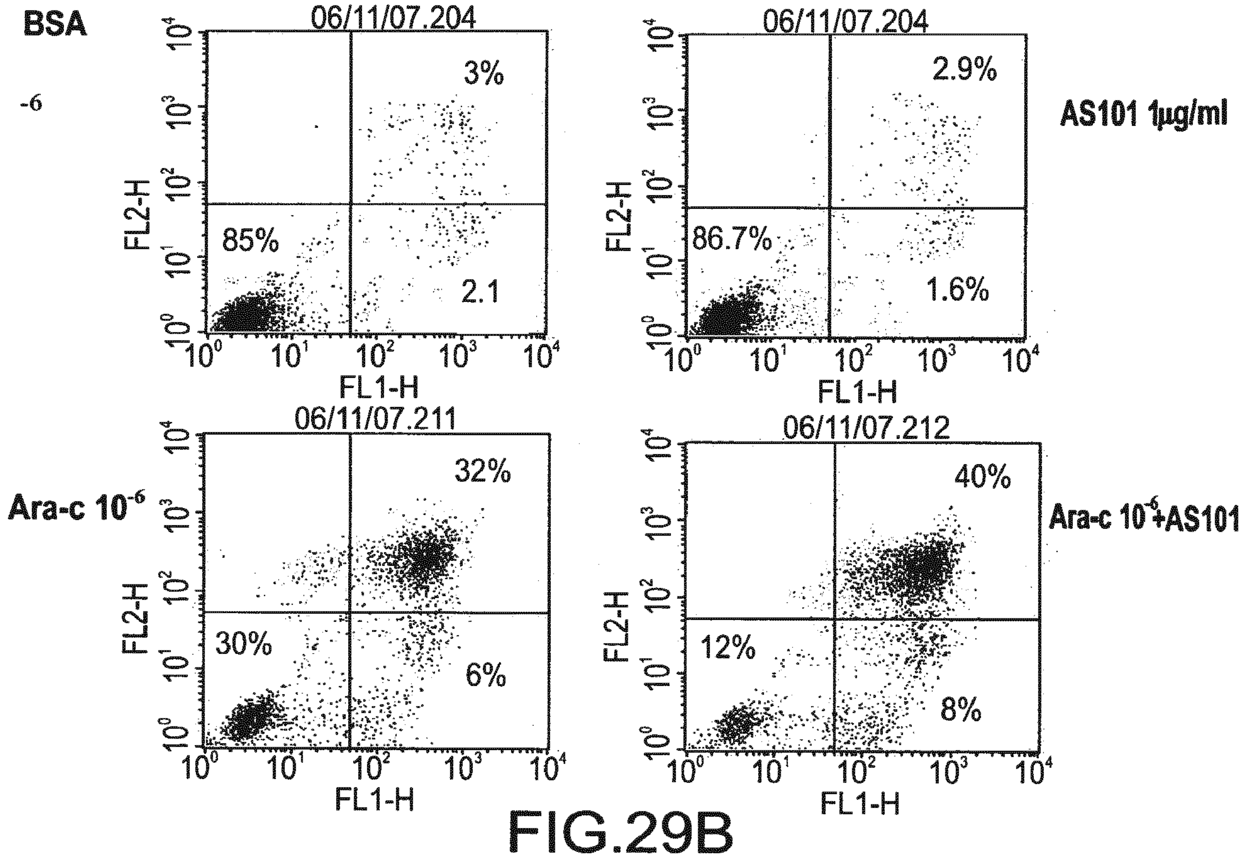


FIG.29B

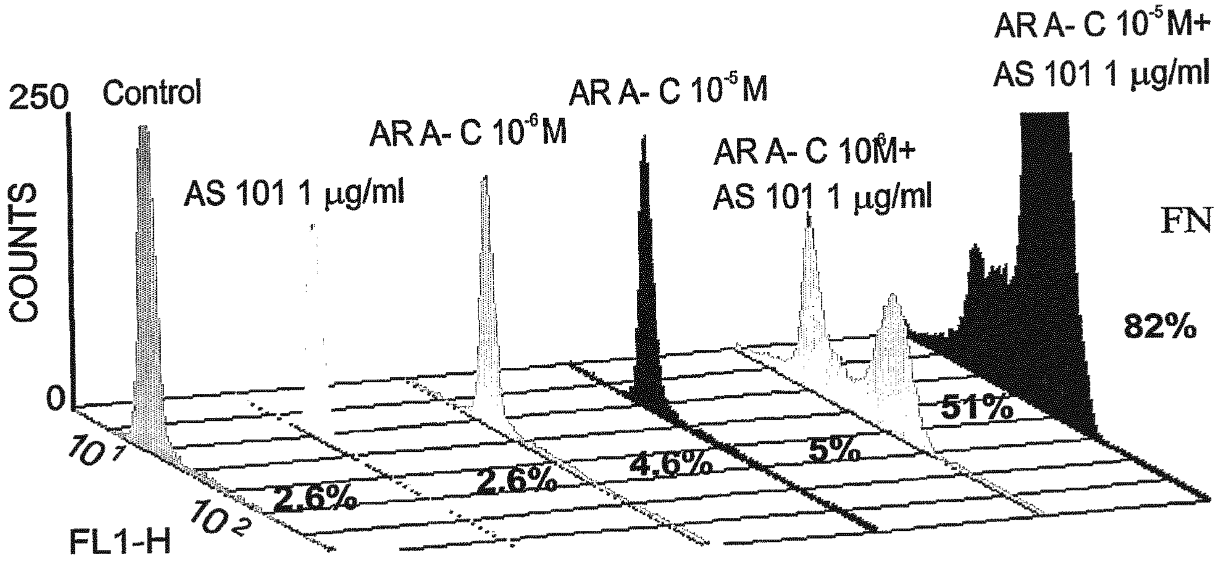


FIG. 30A

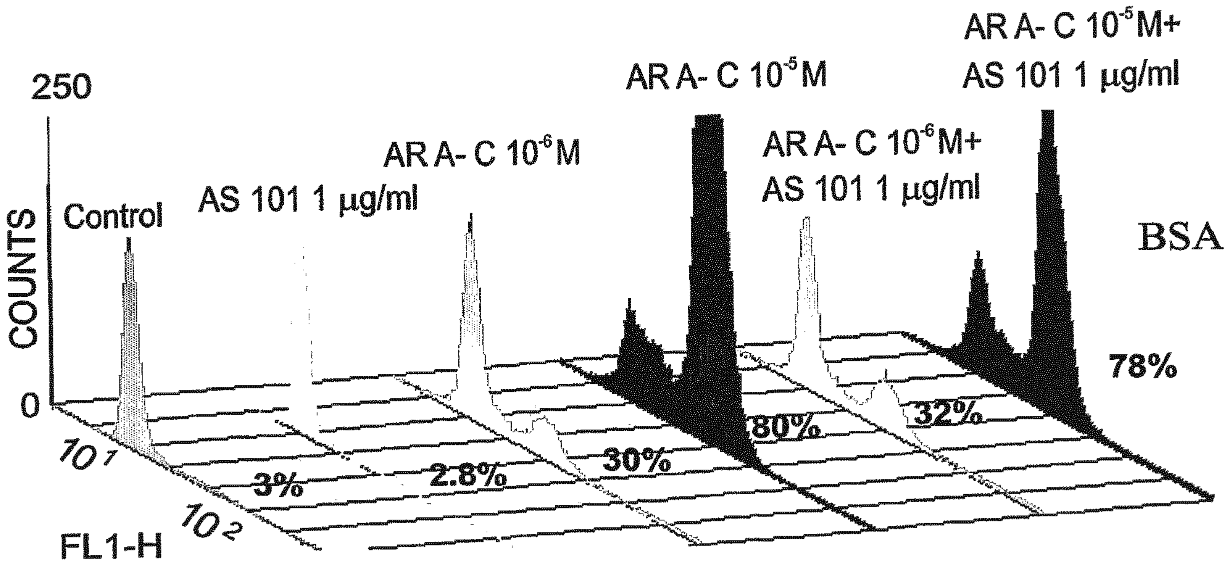


FIG. 30B

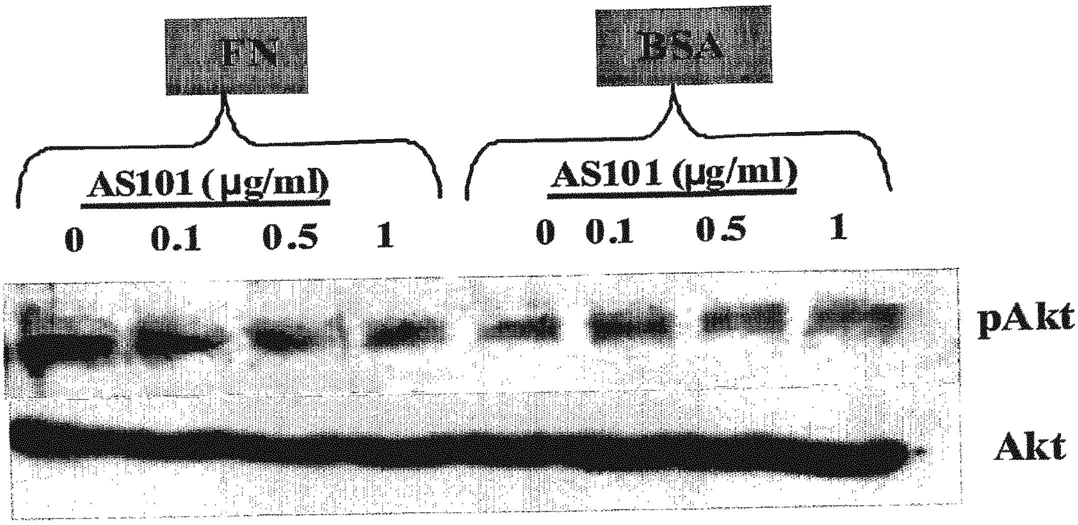


FIG. 31

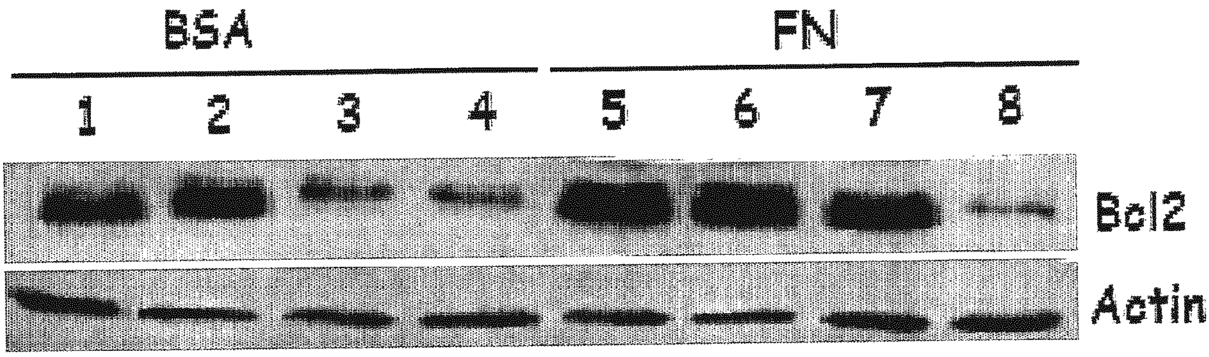


FIG. 32

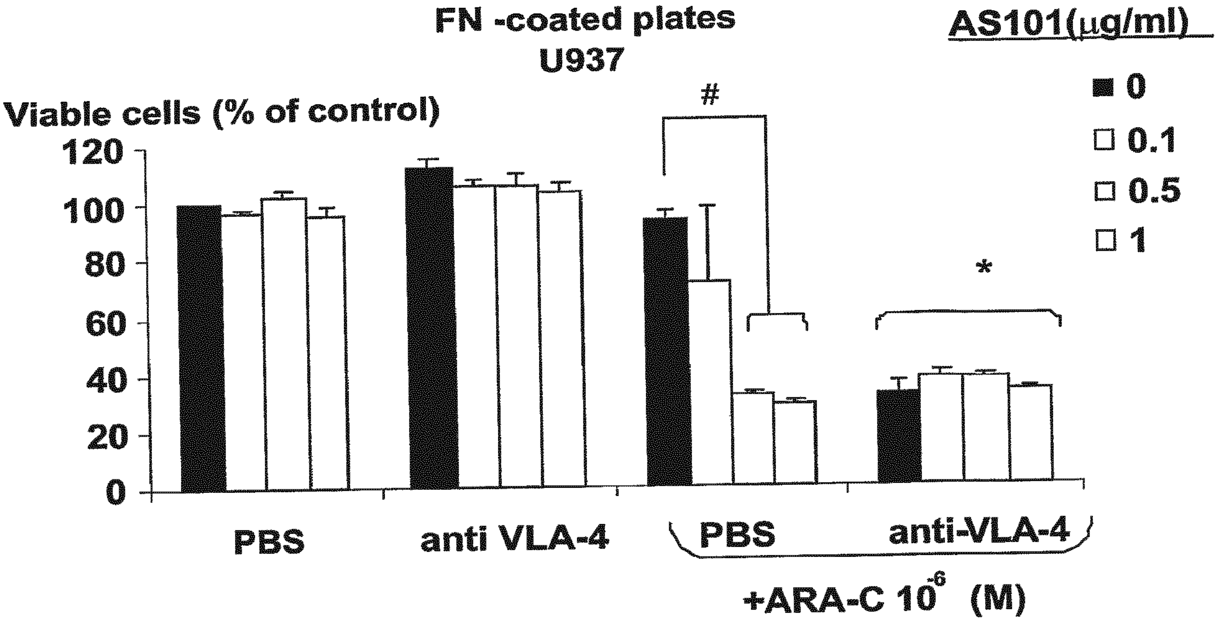


FIG. 33A

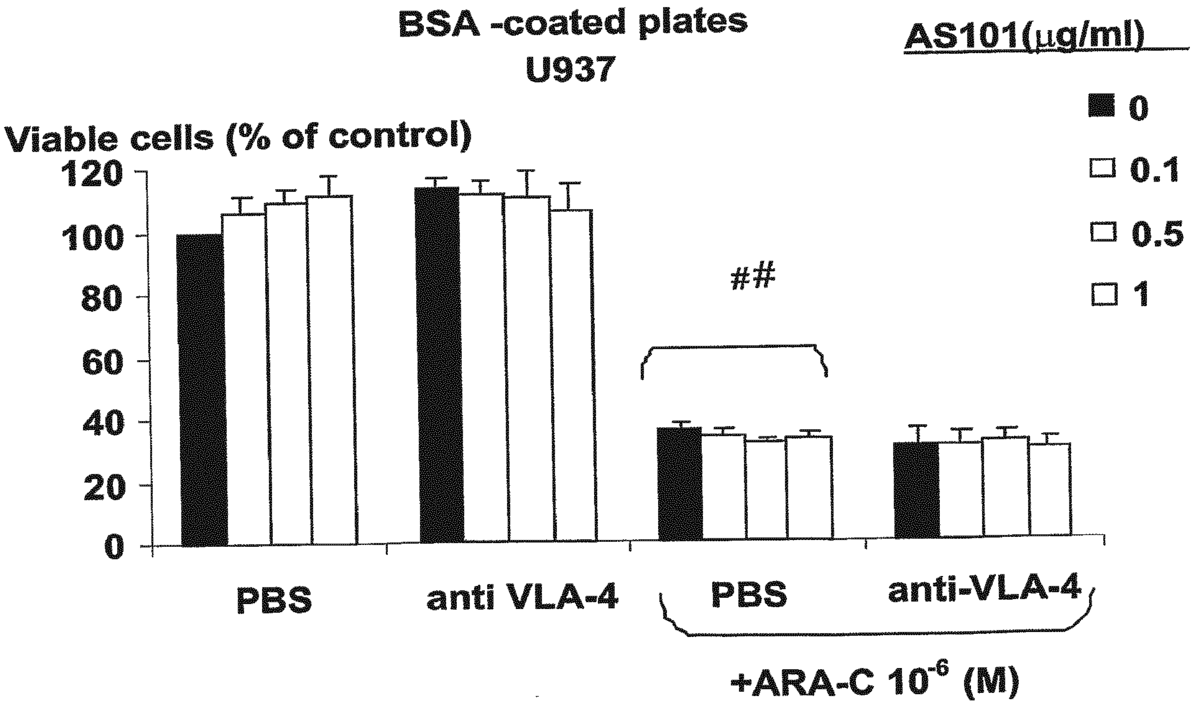


FIG. 33B

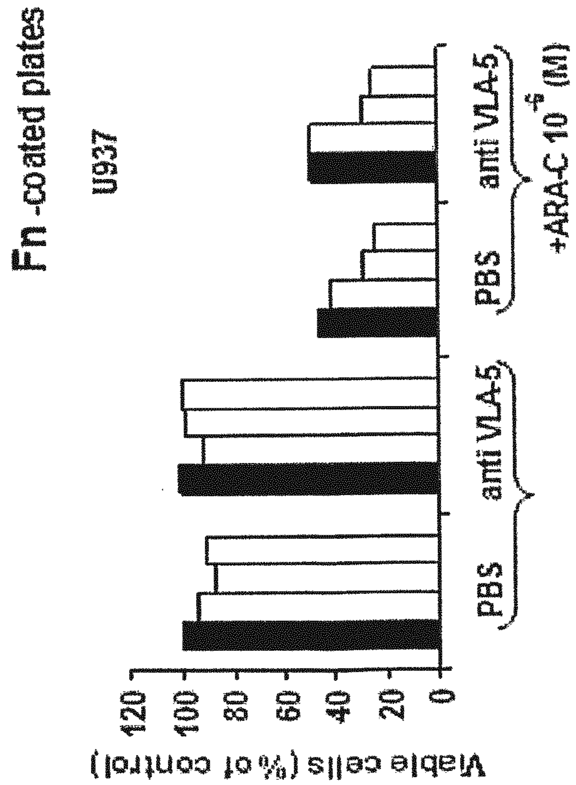


FIG. 34A

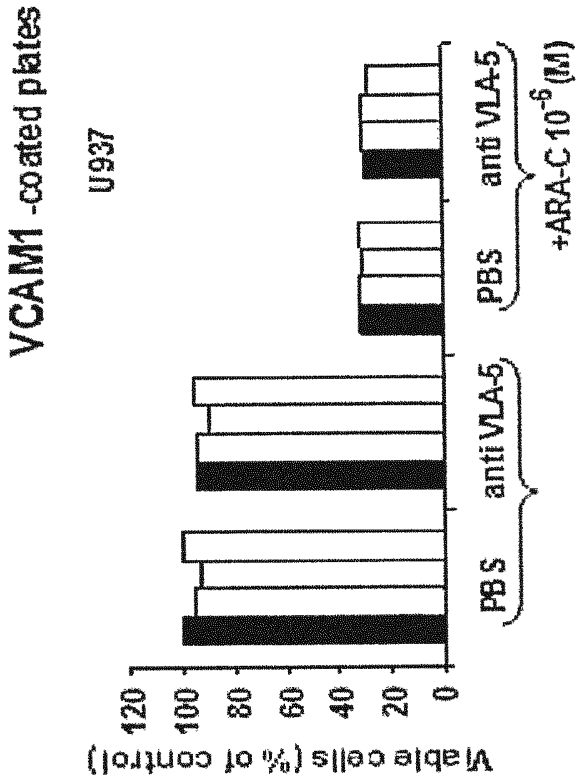


FIG. 34B

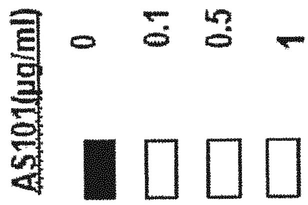


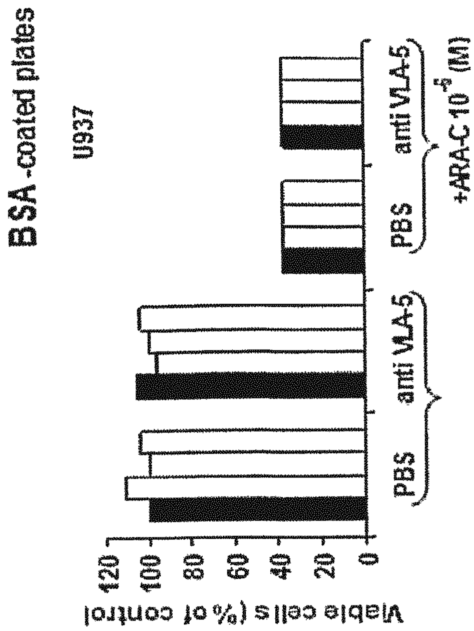


FIG. 34C

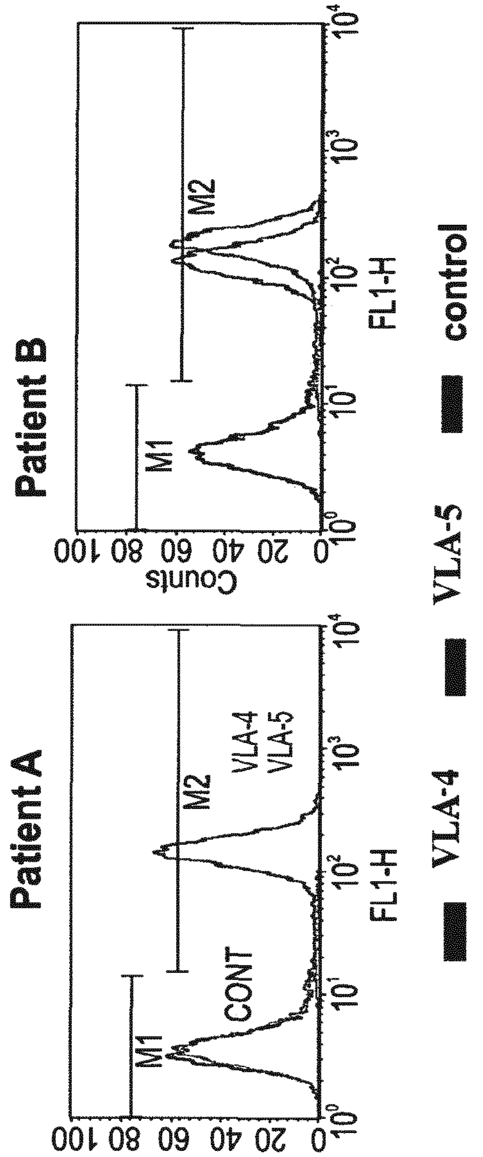


FIG. 35A

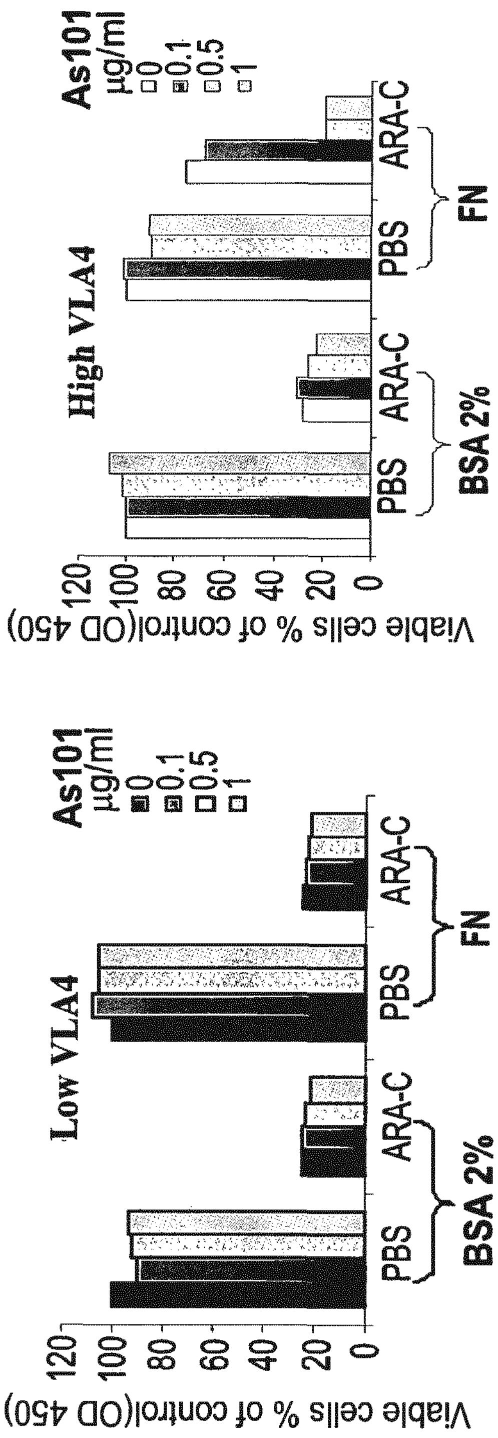


FIG. 35B

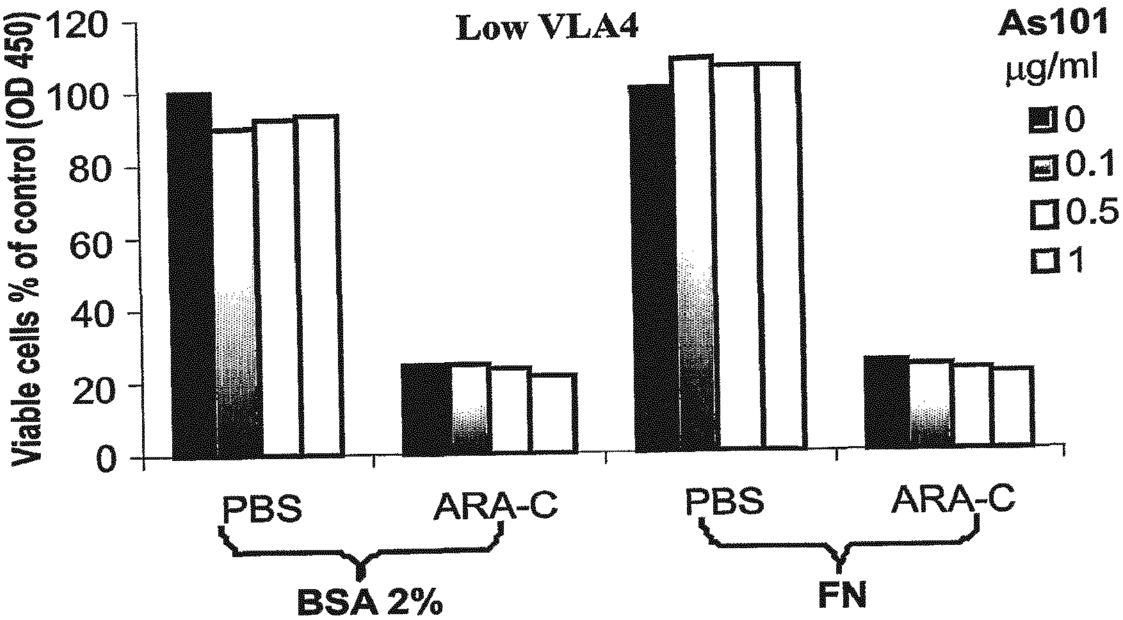


FIG. 36A

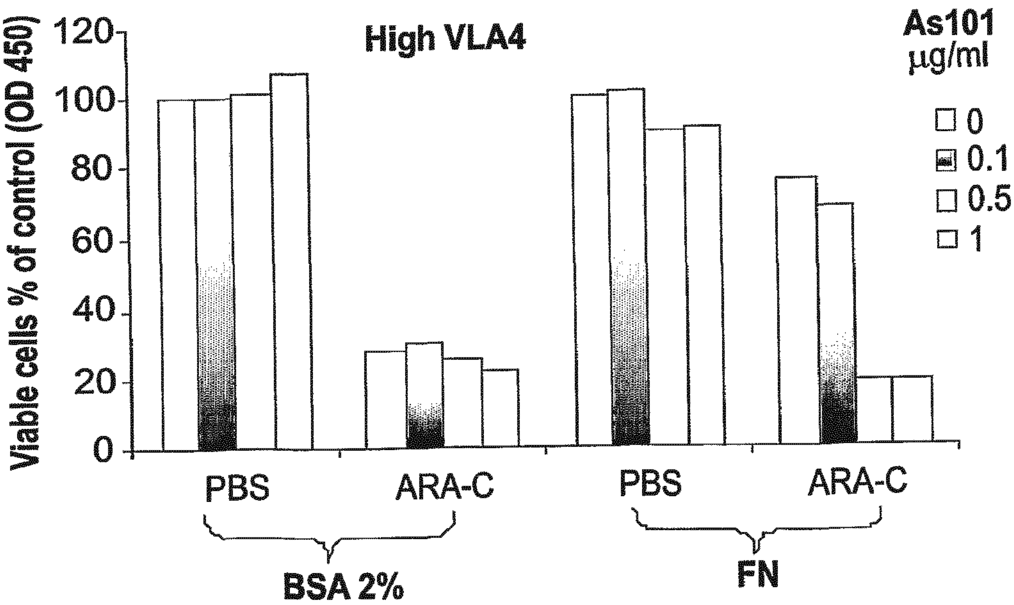


FIG. 36B

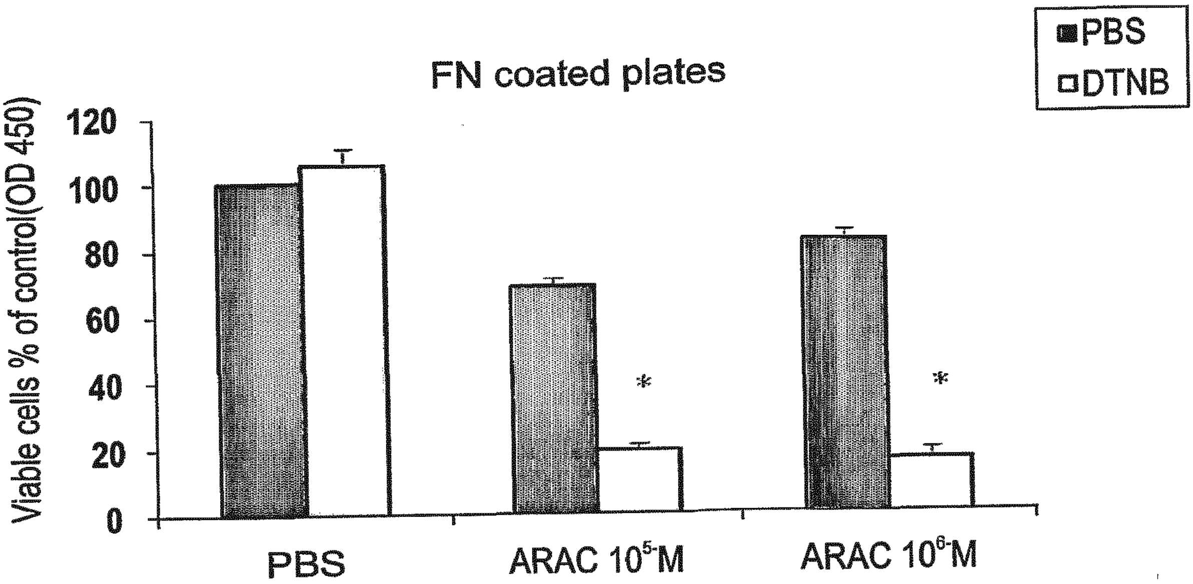


FIG. 37A

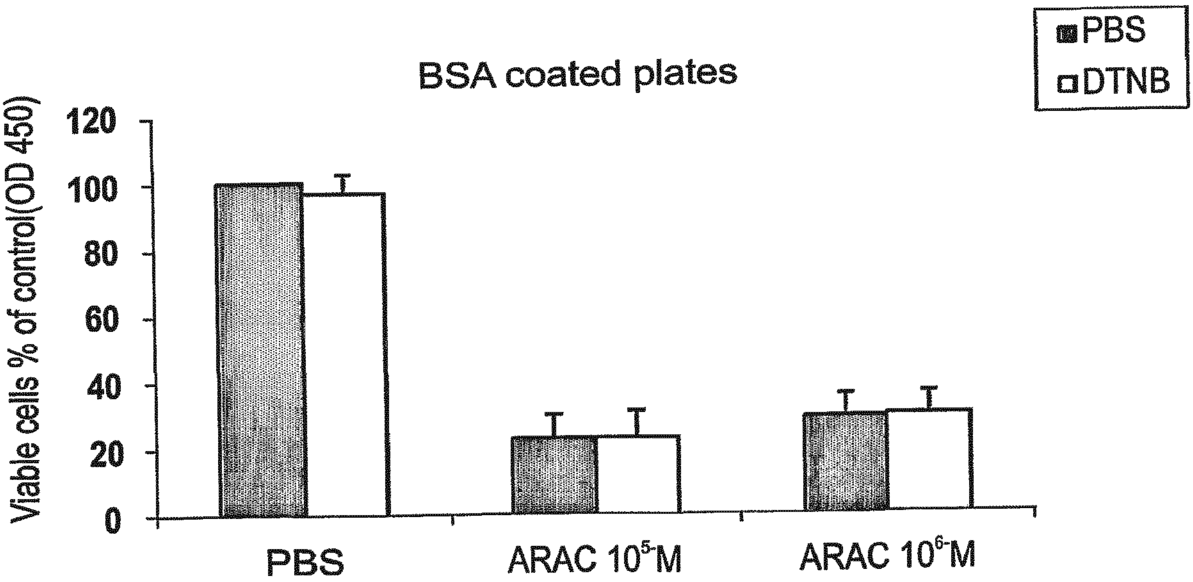


FIG.37B

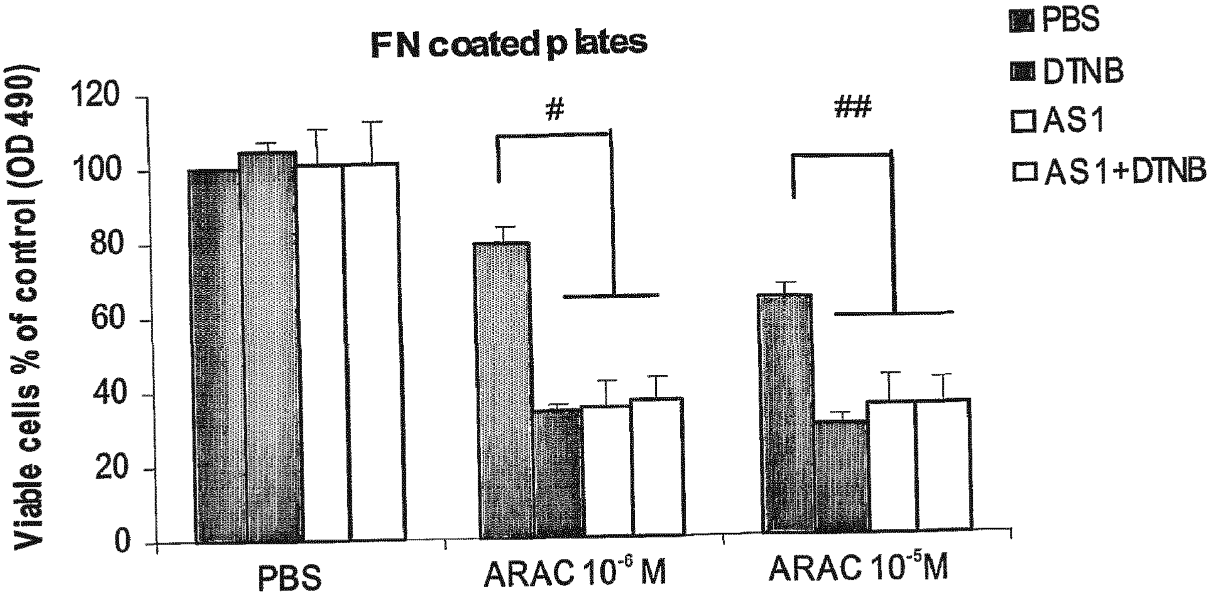


FIG.38A

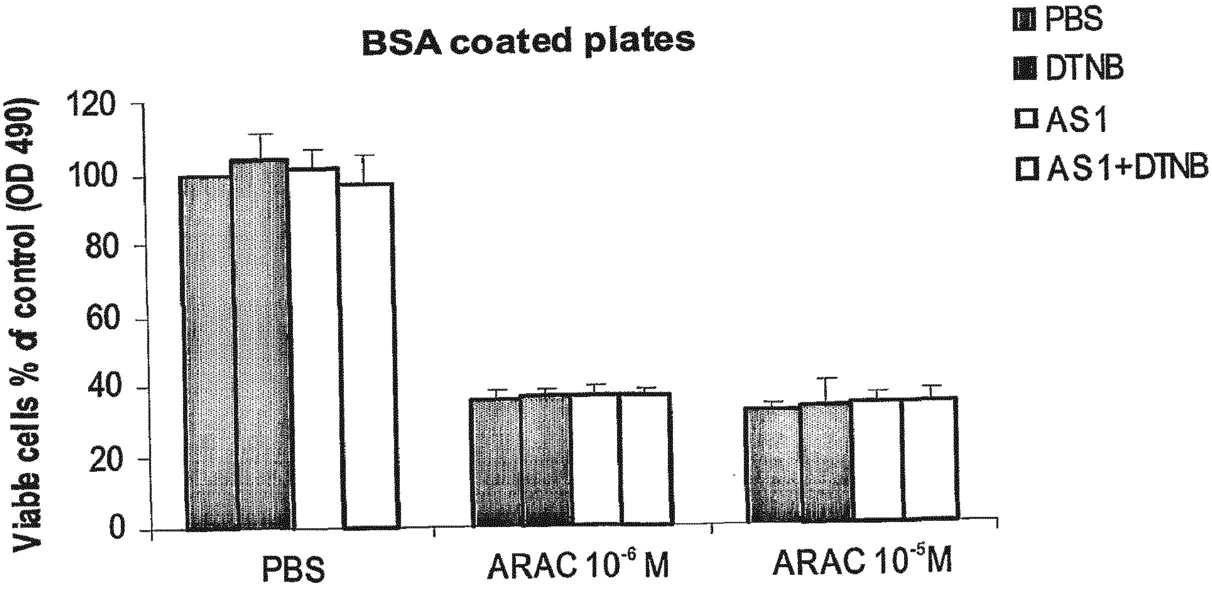


FIG. 38B

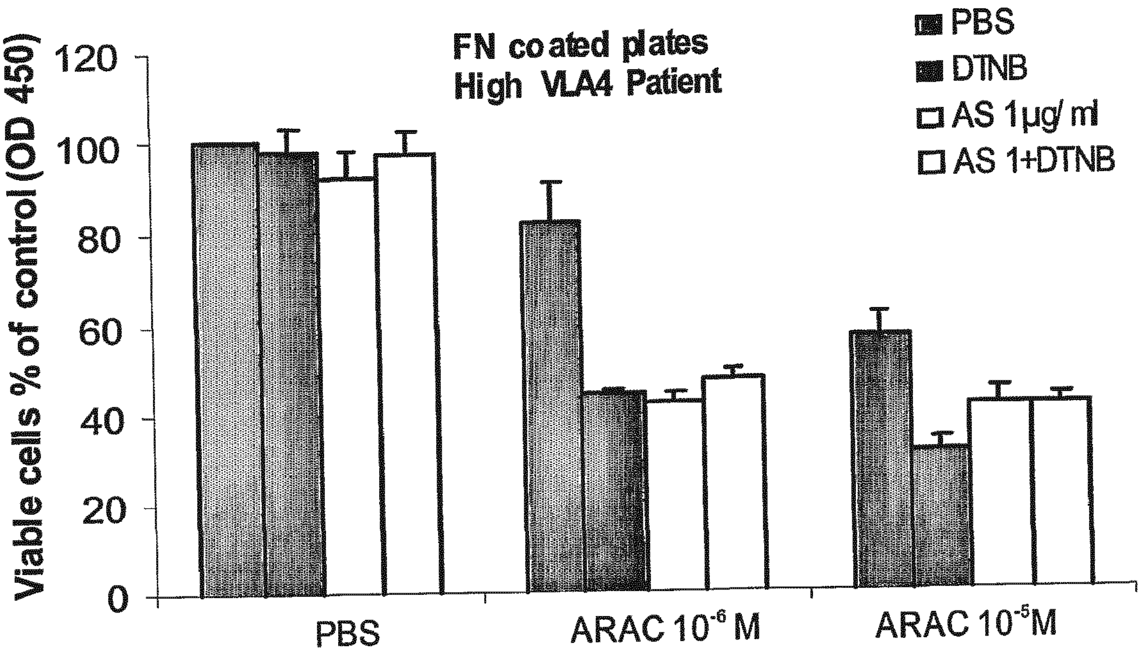


FIG.39A

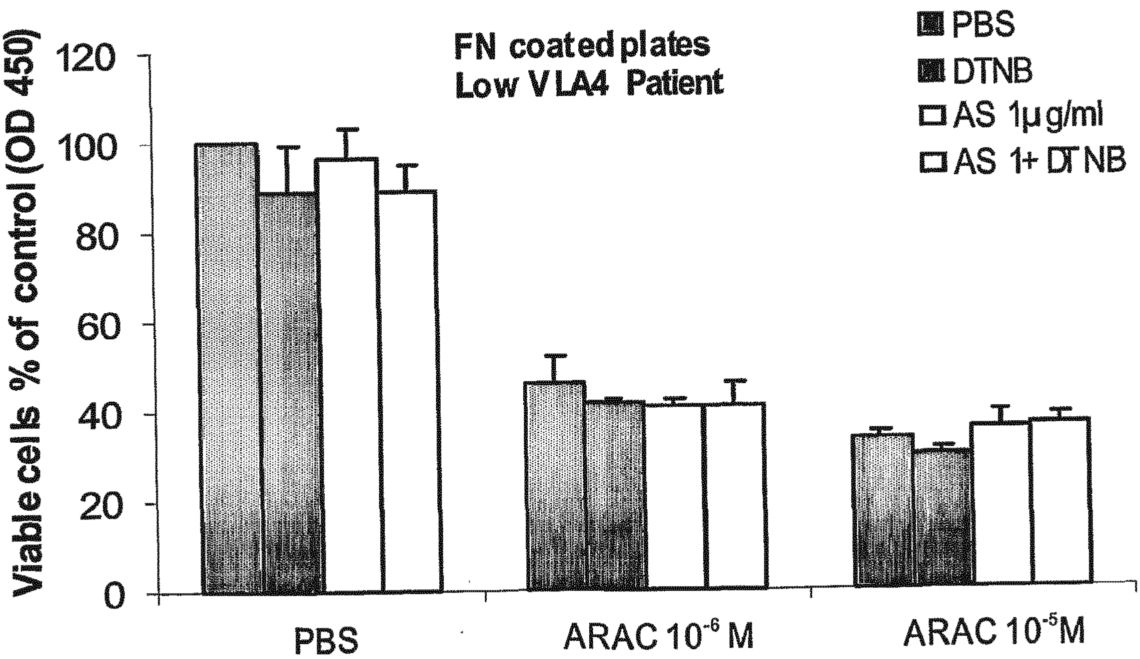


FIG.39B

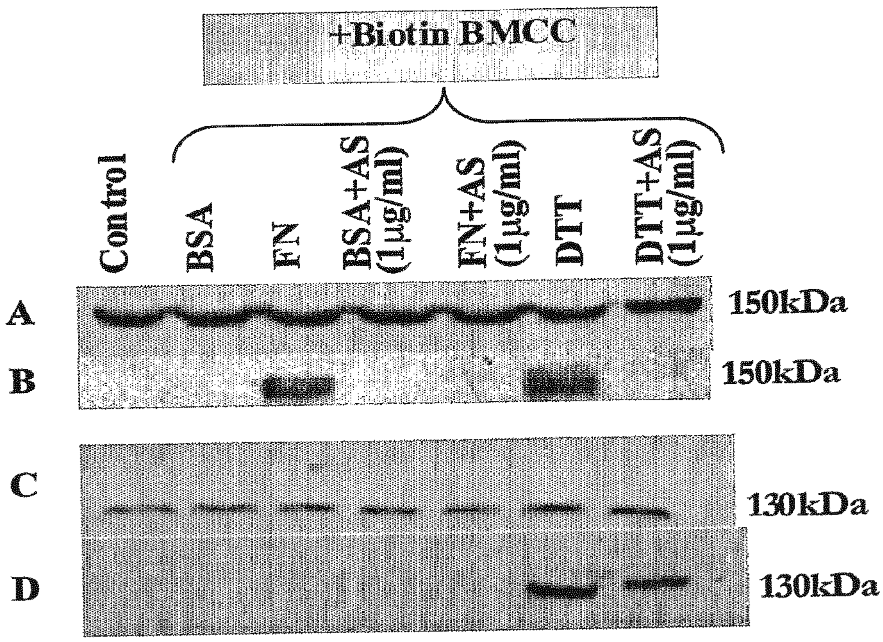
+Biotin BMCC
Control
BSA
FN
BSA+AS (1μg/ml)
FN+AS (1μg/ml)
DTT
DTT+AS (1μg/ml)
A
B
C
D
150kDa
150kDa
130kDa
130kDa

FIG. 40

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4752614 A
- US 4761490 A
- US 4764461 A
- US 4929739 A
- WO 2006030437 A
- US 4962207 A
- US 5093135 A
- US 5102908 A
- US 5213899 A


### Non-patent literature cited in the description


- **FOLKMAN et al.** *Nature,* 1989, vol. 339, 58
- **KIM et al.** *Nature,* 1993, vol. 362, 841
- **HORI et al.** *Cancer Res.,* 1991, vol. 51, 6180
- **ZETTER.** *Annu. Rev. Med.,* 1998, vol. 49, 407
- **BERGERS et al.** *Science,* 1999, vol. 284, 808
- **DAVIS ; SENGER.** *Circ. Res.,* 2005, vol. 97, 1093
- **MCNEEL et al.** *Clin. Cancer Res.,* 2005, vol. 11, 7851
- **LAINER ; BRAHN.** *Expert Opin. Investig. Drugs,* 2005, vol. 14, 1
- **STUPACK.** *Cell Death and Differen.,* 2005, vol. 12, 1021
- **CHUNG ; MERCURIO.** *Molecules and Cells,* 2004, vol. 17, 203
- **HOGG et al.** *J. Cell Sci.,* 2003, vol. 16, 4695
- **VAN ASSCHE ; RUTGEERTS.** *Am J Physiol Gastrointest Liver Physiol,* 2005, vol. 288, G169
- **RUDICK et al.** *N. Eng. J. Med.,* 2006, vol. 354, 911
- **NISHIBORI et al.** *J. Pharmacol Sci,* 2003, vol. 92, 7
- **KILSHAW ; HIGGINS.** *J. Exp. Med.,* 2002, vol. 196, 873
- **ORR et al.** *Mol. Biol. Cell,* 2006, vol. 17, 4686
- **STOUFFER ; SMYTH.** *Arterio. Thrombosis Vasc. Biol.,* 2003, vol. 23, 1971
- **FLICK et al.** *Exp. Boil. Med.,* 2004, vol. 229, 1105
- **BERGH et al.** *Endocrinology,* 2005, vol. 146, 2864
- **ENGLEMAN et al.** *J. Clin. Investig.,* 1997, vol. 99, 2284
- **IWANIEC et al.** *J. Appl. Physiol.,* 2005, vol. 98, 690
- **MIURA et al.** *Proc. Nat. Acad. Sci.,* 2005, vol. 102, 14022
- **ROSS et al.** *Circulation Res.,* 1998, vol. 82, 1160
- **JENKINS et al.** *J. Clin. Invest.,* 2006, vol. 116, 1606
- **SHEPPARD.** *Chest,* 2002, vol. 121, 21S
- **MA et al.** *Am. J. Pathol.,* 2003, vol. 163, 1261
- **RUIZ-TORRES et al.** *J. Nephrol.,* 2005, vol. 18, 334
- **STAUN-RAM ; SHALEV.** *Reprod. Biol. Endocrinol.,* 2005, vol. 3, 56
- **TATONE ; CARBONE.** *Reprod. Biol. Endocrinol.,* 2006, vol. 4, 48
- **STEWART ; NEMEROW.** *Trends in Microbiol.,* 2007, vol. 15, 500
- **BALLANA et al.** *Blood,* 07 October 2008
- **BENTZ ; YUROCHKO.** *Proc. Natl. Acad. Sci. USA,* 2008, vol. 105, 5531
- **MEADS et al.** *Clin. Cancer Res.,* 2008, vol. 14, 2519
- **MATSUNAGA et al.** *Nature Med.,* 2003, vol. 9, 1158
- **FINGL et al.** The Pharmacological Basis of Therapeutics. 1975, 1
- *Synthesis,* August 1989, 635
- **ALBECK et al.** *ChemMedChem,* vol. 2, 1601-1606
- **KALECHMAN et al.** *Int. J. Cancer,* 2000, vol. 86, 281
- **COLGAN et al.** Current Protocols in Immunology. John Wiley & Sons, 2003
- **MAHY ; KANGRO.** Virology Methods Manual. Academic Press, 1996
- **SHARMA et al.** *Virology,* 1997, vol. 239, 150
- **CHU ; NG.** *J. Virol.,* 2004, vol. 78, 10543
- **CHU ; NG.** *J. Biol. Chem.,* 2004, vol. 279, 54533